(19) **Europäisches Patentamt / European Patent Office / Office européen des brevets**

(11) **EP 4 455 158 A1**

(12) # EUROPEAN PATENT APPLICATION
published in accordance with Art. 153(4) EPC

(43) Date of publication:
**30.10.2024 Bulletin 2024/44**

(21) Application number: **22911345.1**

(22) Date of filing: **22.12.2022**

(51) International Patent Classification (IPC):
*C07K 14/52* *(2006.01)*    *C07K 19/00* *(2006.01)*
*C12N 1/15* *(2006.01)*    *C12N 1/19* *(2006.01)*
*C12N 1/21* *(2006.01)*    *C12N 5/10* *(2006.01)*
*C12N 15/19* *(2006.01)*    *C12N 15/62* *(2006.01)*
*C12N 15/63* *(2006.01)*

(52) Cooperative Patent Classification (CPC):
**C07K 14/52; C07K 19/00; C12N 5/10; C12N 15/62;
C12N 15/63; C12N 15/74; C12N 15/80; C12N 15/81**

(86) International application number:
**PCT/JP2022/047366**

(87) International publication number:
**WO 2023/120643 (29.06.2023 Gazette 2023/26)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **23.12.2021 JP 2021209975**

(71) Applicant: **CHUGAI SEIYAKU KABUSHIKI KAISHA
Tokyo 115-8543 (JP)**

(72) Inventors:
• **YAMAMOTO, Yohei
Gotemba-shi, Shizuoka 412-8513 (JP)**
• **KAMON, Hokuto
Kamakura-shi, Kanagawa 247-8530 (JP)**
• **WATANABE, Miho
Gotemba-shi, Shizuoka 412-8513 (JP)**
• **TSUTSUI, Haruka
Gotemba-shi, Shizuoka 412-8513 (JP)**

(74) Representative: **Vossius & Partner
Patentanwälte Rechtsanwälte mbB
Siebertstrasse 3
81675 München (DE)**

Remarks:
The complete document including Reference Table(s) and the Sequence Listing(s) can be downloaded from the EPO website

(54) **CXCR3 LIGAND HAVING ENHANCED CXCR3-EXPRESSING CELL MIGRATION ACTIVITY**

(57) The present disclosure relates to CXCR3 ligands having CXCR3-expressing-cell migration-inducing activity, and specifically to amino acid modifications and amino acid sequences that are important for CXCR3-expressing-cell migration-inducing activity.

EP 4 455 158 A1

**Description**

[Technical Field]

The present disclosure relates to CXCR3 ligands, methods of producing CXCR3 ligands, use of CXCR3 ligands, methods of enhancing the activity of CXCR3 ligands to induce migration of cells expressing CXCR3, and methods of improving the stability in blood of CXCR3 ligands.

[Background Art]

Chemokine receptor CXCR3 (also called G Protein-coupled Receptor 9 (GPR9) and CD183) belongs to the CXC chemokine receptor family and is a G protein-coupled receptor (GPCR) that binds to chemokines CXCL9, CXCL10, and CXCL11. CXCR3 is expressed primarily in activated T-helper type 1 (Th1) lymphocytes and cytotoxic T cells, but is also present in natural killer cells, macrophages, dendritic cells, and B lymphocyte subsets. The chemokines CXCL9, CXCL10, and CXCL11 are three naturally-occurring CXCR3 ligands. The interaction of CXCR3 and its ligands is involved in guiding receptor-bearing cells to specific parts of the body, especially sites of inflammation, immune impairment, and immune dysfunction (NPL 1: Tokunaga, Zhang et al. 2017).
**CXCL10 (C-X-C motif chemokine 10) is also called "IP10 (interferon gamma-induced protein 10)" or "small inducible cytokine B10" and is a chemokine belonging to the CXC subfamily.**
C-X-C motif chemokine 10 (CXCL10) is known to promote T cell migration activity via CXCR3. Furthermore, it has been reported that direct administration of CXCL10 into a mouse tumor increases T cells in the tumor (NPL 2: Rainczuk, Rao et al. 2014).
C-X-C motif chemokine 11 (C-X-C motif chemokine ligand 11, CXCL11) is a C-X-C chemokine which is also called I-TAC (Interferon-inducible T-cell alpha chemoattractant) or IP-9 (Interferon-gamma-inducible protein 9), and naturally-occurring CXCL11 is said to bind to CXCR3 more strongly than naturally-occurring CXCL10 and naturally-occurring CXCL9 (NPL 3: Aguilera-Duran and Romo-Mancillas 2020).
C-X-C motif chemokine 9 (C-X-C motif chemokine ligand 9, CXCL9) is a C-X-C chemokine which is also called Monokine induced by gamma-interferon (MIG).
Chemokines are known to bind to glycosaminoglycans (GAGs) as well as GPCR. GAGs are abundant, e.g., on the surface of epithelial cells and chemokines are thought to localize there by binding to GAGs. Chemokines localized by binding to GAGs are in a state of equilibrium between binding to and dissociation from GAGs and dissociated chemokines are thought to exhibit their function by binding to GPCR (NPL 4: Graham, Handel et al. 2019).
It is known that binding to GAGs is also critical for CXCL10 to exert its function and the modifications where amino acids at positions 12, 14, 20, and 25 are substituted with K or R are reported to enhance GAG binding activity of CXCL10, thereby enhancing the migration of T cells (PTL 1: ANTAGONIS BIOTHERAPEUTICS GMBH, WO2021/038033) (NPL 5: Gerlza, Nagele et al. 2019).

[Citation List]

[Patent Literature]

[PTL 1] WO2021/038033

[Non-Patent Literature]

[NPL 1] Tokunaga, R et al., (2017). Cancer Treat Rev 63: 40-47.
[NPL 2] Rainczuk, A. et al, (2014). Int J Cancer 134(3): 530-541.
[NPL 3] Aguilera-Duran, G et al. (2020). Molecules 25(19).
[NPL 4] Graham, G. J. et al, (2019). Trends Immunol 40(6): 472-481.
[NPL 5] Gerlza, T., Met al. (2019). Protein Eng Des Sel. 31;32(8):367-373

[Summary of Invention]

[Technical Problem]

The present disclosure provides CXCR3 ligands, methods of producing the CXCR3 ligands, and methods of using the CXCR3 ligands. The present disclosure also provides methods of enhancing the activity of CXCR3 ligands to induce migration of CXCR3-expressing-cells, and methods of improving the stability in blood of CXCR3 ligands.

[Solution to Problem]

The present inventors discovered amino acid modifications and amino acid sequences of CXCR3 ligands that are important for enhancement of CXCR3-expressing-cell migration-inducing activity. Moreover, present inventors discovered amino acid modifications that improve the stability in blood of CXCR3 ligands.
The present disclosure specifically includes the embodiments exemplified below:

[A]

(A1) A CXCR3 ligand having enhanced activity to induce migration of a cell expressing CXCR3 relative to a parent CXCR3 ligand, wherein at least either or both modifications of: (i) introduction of a disulfide bond and (ii) modification of an amino acid in an N-terminal region are added to the amino acid sequence of the parent CXCR3 ligand;
(A2) the CXCR3 ligand according to (A1), wherein the parent CXCR3 ligand has a C-X-C motif;
(A3) the CXCR3 ligand according to (A2), wherein the C-X-C motif is C-T-C (Cys-Thr-Cys), C-L-C (Cys-Leu-Cys), or C-S-C (Cys-Ser-Cys);
(A4) the CXCR3 ligand according to any one of (A1) to (A3), wherein the parent CXCR3 ligand comprises at least a portion of the amino acid sequence of a naturally-occurring human CXCR3 ligand;
(A5) the CXCR3 ligand according to (A4), wherein the naturally-occurring human CXCR3 ligand is a naturally-occurring human CXCL10;
(A6) the CXCR3 ligand according to (A4) or (A5), wherein the site of introduction of a disulfide bond is a site other than a β-sheet region of the parent CXCR3 ligand;
(A7) the CXCR3 ligand according to any one of (A2) to (A6), wherein the parent CXCR3 ligand has three β-strands, and wherein the disulfide bond is formed by substituting each of at least one amino acid contained in any two regions selected from:

(i) a first loop between the C-X-C motif and a first β-strand located closest to an N-terminal side;
(ii) a second loop between the first β-strand and a second β-strand located second closest to the N-terminal side; and
(iii) a third loop between the second β-strand and a third β-strand located third closest to the N-terminal side;

in the amino acid sequence of the parent CXCR3 ligand with Cys;
(A8) the CXCR3 ligand according to any one of (A5) to (A7), wherein the disulfide bond is introduced into the CXCR3 ligand by substituting amino acids corresponding to the amino acid positions of the following (i) and (ii) with Cys:

(i) at least one position selected from the group consisting of positions 18, 14, 21, 25, and 41; and
(ii) at least one position selected from the group consisting of positions 60, 55, 67, 46, and 56; in the amino acid sequence of the naturally-occurring human CXCL10;

(A9) the CXCR3 ligand according to any one of (A5) to (A8), wherein the disulfide bond is introduced into amino acid positions corresponding to at least one selected from the group consisting of the following combinations of amino acid positions:

(i) positions 18 and 60;
(ii) positions 14 and 55;
(iii) positions 21 and 67;
(iv) positions 25 and 46; and
(v) positions 41 and 56;

in the amino acid sequence of the naturally-occurring human CXCL 10;
(A10) the CXCR3 ligand according to any one of (A5) to (A9), wherein the disulfide bond is introduced into amino acid positions corresponding to positions 18 and 60 in the amino acid sequence of the naturally-occurring human CXCL 10;
(A11) the CXCR3 ligand according to any one of (A1) to (A10), wherein amino acids at positions 18 and 60 in the amino acid sequence of the parent CXCR3 ligand are substituted with Cys;

(A12) the CXCR3 ligand according to any one of (A1) to (A11), wherein the 18th amino acid from the N-terminus is substituted from P to C and the 60th amino acid is substituted from A to C in the amino acid sequence of the parent CXCR3 ligand;

(A13) the CXCR3 ligand according to any one of (A2) to (A12), wherein the modification of an amino acid in an N-terminal region is substitution of an amino acid closer to the N-terminus than the C-X-C motif in the parent CXCR3 ligand;

(A14) the CXCR3 ligand according to any one of (A5) to (A13), wherein the modification of an amino acid in an N-terminal region is an amino acid substitution at an amino acid position corresponding to between positions 1 and 8 in the amino acid sequence of the naturally-occurring human CXCL10;

(A15) the CXCR3 ligand according to any one of (A1) to (A14), wherein the modification of an amino acid in an N-terminal region is substitution of an amino acid in the N-terminal region with any one of Y, F, H, T, and M;

(A16) the CXCR3 ligand according to any one of (A1) to (A15), wherein the modification of an amino acid in an N-terminal region is substitution of the amino acid at position 1 in the amino acid sequence of the parent CXCR3 ligand with Tyr;

(A17) the CXCR3 ligand according to any one of (A1) to (A16), wherein the first amino acid from the N-terminus in the amino acid sequence of the parent CXCR3 ligand is V and the modification of an amino acid in an N-terminal region comprises the amino acid substitution of V1Y;

(A18) the CXCR3 ligand according to any one of (A1) to (A17), further wherein the second amino acid from the N-terminus in the amino acid sequence of the parent CXCR3 ligand is P and the modification of an amino acid in an N-terminal region comprises the amino acid substitution of P2V;

(A19) the CXCR3 ligand according to any one of (A1) to (A18), further wherein at least one modification selected from an amino acid substitution, deletion, and insertion is made;

(A20) a CXCR3 ligand having enhanced activity to induce migration of a cell expressing CXCR3 relative to a parent CXCR3 ligand comprising a portion of the amino acid sequence of a naturally-occurring human CXCL10, wherein amino acids at positions corresponding to the 18th amino acid and the 60th amino acid in the amino acid sequence of the naturally-occurring human CXCL10 are substituted with Cys;

(A21) the CXCR3 ligand according to (A20), further wherein an amino acid at a position corresponding to the first in the amino acid sequence of the naturally-occurring human CXCL10 is substituted with Tyr;

(A22) a CXCR3 ligand having enhanced activity to induce migration of a cell expressing CXCR3 relative to a parent CXCR3 ligand comprising a portion of the amino acid sequence of a naturally-occurring human CXCL10, wherein an amino acid at a position corresponding to the first in the amino acid sequence of the naturally-occurring human CXCL10 is substituted with Tyr;

(A23) the CXCR3 ligand according to any one of (A20) to (A22), further wherein an amino acid at a position corresponding to the second amino acid in the amino acid sequence of the naturally-occurring human CXCL10 is substituted with Val in the CXCR3 ligand;

(A24) the CXCR3 ligand according to any one of (A1) to (A23), wherein the parent CXCR3 ligand is resistant to furin protease;

(A25) the CXCR3 ligand according to any one of (A1) to (A24), wherein the parent CXCR3 ligand is a CXCL10 variant produced by adding the R75A modification to the amino acid sequence of the naturally-occurring human CXCL 10;

(A26) the CXCR3 ligand according to any one of (A1) to (A17), wherein the 7th amino acid from the N-terminus in the amino acid sequence of the parent CXCR3 ligand is V, and the amino acid modification in the N-terminal region comprises a V7P amino acid substitution; and (A27) the CXCR3 ligand according to any one of (A26), which has increased stability in blood relative to a naturally-occurring human CXCL10 or a parent CXCR3 ligand comprising a portion of the amino acid sequence of a naturally-occurring human CXCL10, due to the V7P amino acid substitution.

[B]

(B1) A polypeptide, wherein amino acids corresponding to amino acid positions 18 and 60 when taking the N-terminal amino acid of a naturally-occurring human CXCL10 as position 1 are substituted with Cys in the amino acid sequence of the naturally-occurring human CXCL10 or a CXCL 10 variant;

(B2) the polypeptide according to (B 1), further wherein an amino acid corresponding to position 1 of the naturally-occurring human CXCL10 is Tyr;

(B3) the polypeptide according to (B 1) or (B2), further wherein an amino acid corresponding to position 2 of the naturally-occurring human CXCL10 is Val;

(B4) a polypeptide, wherein the N-terminal amino acid (position 1) is substituted with Tyr in the amino acid sequence of a naturally-occurring human CXCL10 or a CXCL10 variant;

(B5) the polypeptide according to (B4), further wherein an amino acid corresponding to position 2 of the naturally-occurring human CXCL10 is Val;

(B6) the polypeptide according to (B 1) to (B5), further wherein an amino acid corresponding to position 7 of the naturally-occurring human CXCL10 is Pro;

(B7) a polypeptide, which is a CXCR3 ligand and has the sequence of Y-P-L or Y-V-L at the N-terminus;

(B8) a polypeptide, which is a CXCR3 ligand and has the sequence of Y-P-L-S-R-T (SEQ ID NO: 19) or Y-V-L-S-R-T (SEQ ID NO: 20) at the N-terminus;

(B9) a polypeptide having the amino acid sequence of any one of (a1) to (a3):

(a1) the sequence set forth in any one of SEQ ID NOs: 4-12;

(a2) a sequence showing 90% or more sequence identity to the sequence set forth in any one of SEQ ID NOs: 4-12, and which is a CXCR3 ligand; and

(a3) a sequence comprising 10 or less modifications selected from an amino acid substitution, insertion, deletion, and addition in the sequence set forth in any one of SEQ ID NOs: 4-12, and which is a CXCR3 ligand;

(B10) the polypeptide according to (B9), wherein the amino acid at position 1 is Tyr;

(B11) the polypeptide according to (B9) or (B10), wherein amino acids at positions 18 and 60 are Cys;

(B12) the polypeptide according to any one of (B9) to (B 1 1), wherein the amino acid at position 2 is Val;

(B13) the polypeptide according to any one of (B9) to (B12), wherein the amino acid at position 7 is Pro;

(B14) the polypeptide according to any one of (B 1) to (B 13), further wherein an amino acid corresponding to position 75 of the naturally-occurring human CXCL10 is substituted with Ala;

(B15) the polypeptide according to any one of (B 1) to (B14), wherein the polypeptide has a C-X-C motif and further has any one of (b 1) to (b6) at the C-terminus of the C-X-C motif:

(b 1) the sequence from the 12th amino acid to the 77th amino acid of any one of SEQ ID NOs: 4-12;

(b2) the sequence from the 12th amino acid to the 77th amino acid of SEQ ID NO: 25;

(b3) the sequence from the 12th amino acid to the 73rd amino acid of SEQ ID NO: 26;

(b4) the sequence from the 12th amino acid to the 103rd amino acid of SEQ ID NO: 27;

(b5) the sequence from the 12th amino acid to the 77th amino acid of SEQ ID NO: 1; and

(b6) the sequence from the 12th amino acid to the 77th amino acid of SEQ ID NO: 28;

(B16) a polypeptide having the following amino acid sequence:
(X1)(X2)LSRTVRCT CISISNQ(X3)VN PRSLEKLEII PASQFCPRVE IIATMKKKG EKRCLNPESK(X4) IKNLLKAVSK ERSK(X5)SP (SEQ ID NO: 53),
wherein (X1) is V or Y, (X2) is P or V, (X3) and (X4) are C, and (X5) is R or A;

(B17) a polypeptide having the following amino acid sequence:
(X1)(X2)LSRTVRCT CISISNQ(X3)VN PRSLEKLEII PASQFCPRVE IIATMKKKG EKRCLNPESK(X4) IKNLLKAVSK ERSK(X5)SP (SEQ ID NO: 54),
wherein (X1) is Y, (X2) is P or V, (X3) is P or C, (X4) is A or C, and (X5) is R or A;

(B18) the polypeptide according to any one of (B 1) to (B 17), further wherein at least one modification selected from an amino acid substitution, deletion, and insertion is made;

(B19) the polypeptide according to any one of (B 1) to (B 18), which has CXCR3-expressing-cell migration-inducing activity;

(B20) the polypeptide according to any one of (B 1) to (B 19), wherein CXCR3-expressing-cell migration-inducing activity of the polypeptide is equal to or greater than CXCR3-expressing-cell migration-inducing activity of a naturally-occurring human CXCL10;

(B21) the polypeptide according to any one of (B 1) to (B20), wherein CXCR3-expressing-cell migration-inducing activity of the polypeptide is greater than CXCR3-expressing-cell migration-inducing activity of a variant produced by adding the R75A modification to the naturally-occurring human CXCL10;

(B22) the polypeptide according to any one of (B 1) to (B21), wherein the concentration of the polypeptide when CXCR3-expressing-cell migration-inducing activity of the polypeptide is at maximum is equal to or lower than the concentration of a naturally-occurring human CXCL10 when CXCR3-expressing-cell migration-inducing activity of the naturally-occurring human CXCL10 is at maximum;

(B23) the polypeptide according to any one of (B 1) to (B22), wherein the concentration of the polypeptide when CXCR3-expressing-cell migration-inducing activity of the polypeptide is at maximum is lower than the concentration of a naturally-occurring human CXCL10 when CXCR3-expressing-cell migration-inducing activity of a variant produced by adding the R75A modification to the naturally-occurring human CXCL10 is at maximum;

(B24) the polypeptide according to any one of (B20) to (B23), wherein CXCR3-expressing-cell migration-inducing activity of the polypeptide is determined by a cell migration rate;

(B25) the polypeptide according to any one of (B19) to (B24), wherein the CXCR3-expressing-cell is a transfectant or a cell isolated from a living body that expresses CXCR3; and (B26) the polypeptide according to any one of (B19) to (B25), wherein the CXCR3-expressing-cell is a cell expressing human CXCR3.

[C]

(C1) An isolated nucleic acid encoding the CXCR3 ligand according to [A] or the polypeptide according to [B];

(C2) a vector having the nucleic acid according to (C1);

(C3) a host cell comprising the nucleic acid according to (C1) or the vector according to (C2);

(C4) a method of producing the CXCR3 ligand according to [A] or the polypeptide according to [B], comprising culturing the host cell according to (C3) such that the CXCR3 ligand or the polypeptide is produced;

(C5) the method according to (C4), further comprising recovering the polypeptide or the CXCR3 ligand from the host cell;

(C6) a fusion protein comprising the CXCR3 ligand according to [A] or the polypeptide according to [B];

(C7) the fusion protein according to (C6), wherein the CXCR3 ligand according to [A] or the polypeptide according to [B] is fused with an antibody Fc region;

(C8) the fusion protein according to (C7), wherein the CXCR3 ligand according to [A] or the polypeptide according to [B] is fused with an intact antibody or an antibody fragment;

(C9) the fusion protein according to (C7) or (C8), wherein the CXCR3 ligand or the polypeptide and the antibody Fc region, or the CXCR3 ligand or the polypeptide and the intact antibody or the antibody fragment are fused via a linker;

(C10) a pharmaceutical composition comprising the CXCR3 ligand according to [A], the polypeptide according to [B], or the fusion protein according to any one of (C6) to (C9); and (C11) a method of treating a disease, comprising administering the CXCR3 ligand according to [A], the polypeptide according to [B], or the fusion protein according to any one of (C6) to (C9).

[D]

(D1) A method of enhancing CXCR3-expressing-cell migration-inducing activity of a CXCR3 ligand, comprising adding at least either or both modifications of: (i) introduction of a disulfide bond and (ii) modification of an amino acid in an N-terminal region to the amino acid sequence of a parent CXCR3 ligand;

(D2) the method according to (D1), wherein the parent CXCR3 ligand has a C-X-C motif;

(D3) the method according to (D2), wherein the C-X-C motif is C-T-C (Cys-Thr-Cys), C-L-C (Cys-Leu-Cys), or C-S-C (Cys-Ser-Cys);

(D4) the method according to (D3), wherein the parent CXCR3 ligand comprises a portion of the amino acid sequence of a naturally-occurring human CXCR3 ligand;

(D5) the method according to (D4), wherein the naturally-occurring human CXCR3 ligand is a naturally-occurring human CXCL10;

(D6) the method according to either of (D4) or (D5), wherein the modification comprises introduction of a disulfide bond and the disulfide bond is introduced at a site other than a β-sheet region of the parent CXCR3 ligand;

(D7) the method according to any one of (D2) to (D6), wherein the parent CXCR3 ligand has three β-strands, and wherein the disulfide bond is formed by substituting each of at least one amino acid contained in any two regions selected from:

(i) a first loop between the C-X-C motif and a first β-strand located closest to an N-terminal side;
(ii) a second loop between the first β-strand and a second β-strand located second closest to the N-terminal side; and
(iii) a third loop between the second β-strand and a third β-strand located third closest to the N-terminal side;

in the amino acid sequence of the parent CXCR3 ligand with Cys;

(D8) the method according to any one of (D5) to (D7), wherein the disulfide bond is introduced by substituting amino acids corresponding to the amino acid positions of the following (i) and (ii) with Cys:

(i) at least one position selected from the group consisting of positions 18, 14, 21, 25, and 41; and
(ii) at least one position selected from the group consisting of positions 60, 55, 67, 46, and 56; in the amino acid sequence of the naturally-occurring human CXCL10;

(D9) the method according to any one of (D5) to (D8), wherein the disulfide bond is introduced into amino acid positions corresponding to at least one selected from the group consisting of the following combinations of amino acid positions:

(i) positions 18 and 60;
(ii) positions 14 and 55;
(iii) positions 21 and 67;
(iv) positions 25 and 46; and
(v) positions 41 and 56;

in the amino acid sequence of the naturally-occurring human CXCL10;

(D10) the method according to any one of (D2) to (D9), wherein the modification comprises modification of an amino acid in an N-terminal region and the modification of an amino acid in an N-terminal region is substitution of an amino acid closer to the N-terminus than the C-X-C motif of the parent CXCR3 ligand;

(D11) the method according to any one of (D5) to (D10), wherein the modification of an amino acid in an N-terminal region is an amino acid substitution at an amino acid position corresponding to between positions 1 and 8 in the amino acid sequence of the naturally-occurring human CXCL10;

(D12) the method according to any one of (D1) to (D10), wherein the modification of an amino acid in an N-terminal region is substitution of an amino acid in the N-terminal region with any one of Y, F, H, T, and M;

(D13) the method according to any one of (D1) to (D12), wherein the first amino acid from the N-terminus in the amino acid sequence of the parent CXCR3 ligand is V and the method comprises adding the amino acid substitution of V1Y;

(D14) the method according to any one of (D1) to (D13), wherein the 18th amino acid from the N-terminus is P and the 60th amino acid is A in the amino acid sequence of the parent CXCR3 ligand, and the method comprises adding the amino acid substitutions of P18C and A60C;

(D15) the method according to any one of (D1) to (D14), further wherein the second amino acid from the N-terminus in the amino acid sequence of the parent CXCR3 ligand is P and the method comprises adding the amino acid substitution of P2V;

(D16) the method according to (D1) to (D15), further comprising adding at least one modification selected from an amino acid substitution, deletion, and insertion;

(D17) a method of enhancing the activity of a parent CXCR3 ligand, which comprises a portion of the amino acid sequence of a naturally-occurring human CXCL10, to induce migration of a cell expressing CXCR3, the method comprising substituting amino acids at positions corresponding to the 18th amino acid and the 60th amino acid in the amino acid sequence of the naturally-occurring human CXCL10 with Cys;

(D18) the method according to (D17), further comprising substituting an amino acid at a position corresponding to the first in the amino acid sequence of the naturally-occurring human CXCL10 with Tyr;

(D19) a method of enhancing the activity of a parent CXCR3 ligand, which comprises a portion of the amino acid sequence of a naturally-occurring human CXCL10, to induce migration of a cell expressing CXCR3, the method comprising substituting an amino acid at a position corresponding to the first in the amino acid sequence of the naturally-occurring human CXCL10 with Tyr;

(D20) the method according to any one of (D17) to (D19), further comprising substituting an amino acid at a position corresponding to the second amino acid in the amino acid sequence of the naturally-occurring human CXCL10 with Val;

(D21) the method according to any one of (D1) to (D20), wherein the parent CXCR3 ligand is resistant to furin protease;

(D22) the method according to any one of (D1) to (D21), wherein the parent CXCR3 ligand is a CXCL10 variant produced by adding the R75A modification to the amino acid sequence of the naturally-occurring human CXCL10; and

(D23) the method according to any one of (D1) to (D22), further wherein the 7th amino acid from the N-terminus in the amino acid sequence of the parent CXCR3 ligand is V and the method comprises adding the amino acid substitution of V7P.

[E]

(E1) A method of producing a CXCR3 ligand having enhanced CXCR3-expressing-cell migration-inducing activity relative to a parent ligand, wherein the method comprises adding at least either or both modifications of: (i) introduction of a disulfide bond and (ii) modification of an amino acid in an N-terminal region to the amino acid sequence of the parent CXCR3 ligand;

(E2) the method according to (E1), wherein the parent CXCR3 ligand has a C-X-C motif;

(E3) the method according to (E2), wherein the C-X-C motif is C-T-C (Cys-Thr-Cys), C-L-C (Cys-Leu-Cys), or C-S-C (Cys-Ser-Cys);

(E4) the method according to (E2), wherein the parent CXCR3 ligand comprises a portion of the amino acid sequence of the sequence of a naturally-occurring human CXCR3 ligand;

(E5) the method according to (E3), wherein the naturally-occurring human CXCR3 ligand is a naturally-occurring human CXCL10;

(E6) the method according to (E4) or (E5), wherein the disulfide bond is introduced at a site other than a β-sheet region of the parent CXCR3 ligand;

(E7) the method according to any one of (E2) to (E6), wherein the parent CXCR3 ligand has three β-strands, and wherein the disulfide bond is formed by substituting each of at least one amino acid contained in any two regions selected from:

(i) a first loop between the C-X-C motif and a first β-strand located closest to an N-terminal side;
(ii) a second loop between the first β-strand and a second β-strand located second closest to the N-terminal side; and
(iii) a third loop between the second β-strand and a third β-strand located third closest to the N-terminal side;

in the amino acid sequence of the parent CXCR3 ligand with Cys;

(E8) the method according to any one of (E5) to (E7), wherein the introduction of a disulfide bond is by substituting amino acids corresponding to the amino acid positions of the following (i) and (ii) with Cys:

(i) at least one position selected from the group consisting of positions 18, 14, 21, 25, and 41; and
(ii) at least one position selected from the group consisting of positions 60, 55, 67, 46, and 56; in the amino acid sequence of the naturally-occurring human CXCL 10;

(E9) the method according to any one of (E5) to (E8), wherein the disulfide bond is introduced into amino acid positions corresponding to at least one selected from the group consisting of the following combinations of amino acid positions:

(i) positions 18 and 60;
(ii) positions 14 and 55;
(iii) positions 21 and 67;
(iv) positions 25 and 46; and
(v) positions 41 and 56;

in the amino acid sequence of the naturally-occurring human CXCL 10;

(E10) the method according to any one of (E1) to (E9), wherein the amino acid at position 18 is P and the amino acid at position 60 is A in the amino acid sequence of the parent CXCR3 ligand, and the method comprises the amino acid substitutions of P18C and A60C;

(E11) the method according to any one of (E3) to (E10), wherein the modification of an amino acid in an N-terminal region is substitution of an amino acid closer to the N-terminus than the C-X-C motif of the parent CXCR3 ligand;

(E12) the method according to any one of (E5) to (E11), wherein the modification of an amino acid in an N-terminal region is made between positions 1 and 8 in the amino acid sequence of the naturally-occurring human CXCL10;

(E13) the method according to any one of (E1) to (E12), wherein the modification of an amino acid in an N-terminal region is substitution of an amino acid in the N-terminal region with any one of Y, F, H, T, and M;

(E14) the method according to any one of (E1) to (E13), wherein the amino acid at position 1 in the amino acid sequence of the parent CXCR3 ligand is V and the method comprises the amino acid substitution of V1Y;

(E15) the method according to any one of (E1) to (E14), further wherein the amino acid at position 2 in the amino acid sequence of the parent CXCR3 ligand is P and the method comprises the amino acid substitution of P2V;

(E16) the method according to any one of (E1) to (E15), wherein CXCR3-expressing-cell migration-inducing activity of the CXCR3 ligand is equal to or greater than a naturally-occurring human CXCR3 ligand;

(E17) a method of producing a CXCR3 ligand having enhanced CXCR3-expressing-cell migration-inducing activity relative to a parent CXCR3 ligand comprising a portion of the amino acid sequence of a naturally-occurring human CXCL10, the method comprising substituting amino acids at positions corresponding to the 18th amino acid and the 60th amino acid in the amino acid sequence of the naturally-occurring human CXCL10 with Cys;

(E18) the method according to (E15), further comprising substituting an amino acid at a position corresponding to the first in the amino acid sequence of the naturally-occurring human CXCL10 with Tyr;

(E19) a method of producing a CXCR3 ligand having enhanced CXCR3-expressing-cell migration-inducing activity relative to a parent CXCR3 ligand comprising a portion of the amino acid sequence of a naturally-occurring human CXCL10, the method comprising substituting an amino acid at a position corresponding to the first in the amino acid sequence of the naturally-occurring human CXCL10 with Tyr;

(E20) the method according to any one of (E15) to (E17), further comprising substituting an amino acid at a position corresponding to the second amino acid in the amino acid sequence of the naturally-occurring human CXCL10 with Val;

(E21) the method according to any one of (E1) to (E20), wherein the parent CXCR3 ligand is resistant to furin protease;

(E22) the method according to any one of (E1) to (E21), wherein the parent CXCR3 ligand is a CXCL10 variant produced by adding the R75A modification to the amino acid sequence of the naturally-occurring human CXCL10;

(E23) the method according to any one of (E1) to (E22), further wherein the 7th amino acid from the N-terminus in the amino acid sequence of the parent CXCR3 ligand is V and the method comprises the amino acid substitution of V7P; and

(E24) a CXCR3 ligand produced by the method according to any one of (E1) to (E23).

[F]

(F 1) A CXCR3 ligand having improved stability in blood relative to a parent CXCR3 ligand, wherein the amino acid corresponding to amino acid position 7 when taking the N-terminal amino acid in the amino acid sequence of the parent CXCR3 ligand as position 1 is substituted with Pro;

(F2) the CXCR3 ligand according to (F1), wherein the parent CXCR3 ligand has a C-X-C motif; (F3) the CXCR3 ligand according to (F2), wherein the C-X-C motif is C-T-C (Cys-Thr-Cys), C-L-C (Cys-Leu-Cys), or C-S-C (Cys-Ser-Cys);

(F4) the CXCR3 ligand according to any one of (F 1) to (F3), wherein the parent CXCR3 ligand comprises at least a portion of the amino acid sequence of a naturally-occurring human CXCR3 ligand;

(F5) the CXCR3 ligand according to (F4), wherein the naturally-occurring human CXCR3 ligand is a naturally-occurring human CXCL10;

(F6) the CXCR3 ligand according to any one of (F1) to (F5), wherein the 7th amino acid from the N-terminus is substituted from V to P in the amino acid sequence of the parent CXCR3 ligand;

(F7) the CXCR3 ligand according to any one of (F1) to (F6) having enhanced activity to induce migration of a cell expressing CXCR3 relative to a parent CXC3 ligand, wherein either or both modifications of:

(i) introduction of a disulfide bond; and
(ii) modification of an amino acid in an N-terminal region;

are further added to the amino acid sequence of the parent CXCR3 ligand;

(F8) the CXCR3 ligand according to (F7), wherein the disulfide bond is introduced into amino acid positions corresponding to positions 18 and 60 in the amino acid sequence of the naturally-occurring human CXCL10;

(F9) the CXCR3 ligand according to (F7) or (F8), wherein amino acids at positions 18 and 60 in the amino acid sequence of the parent CXCR3 ligand are substituted with Cys;

(F10) the CXCR3 ligand according to any one of (F7) to (F9), wherein the 18th amino acid from the N-terminus is substituted from P to C and the 60th amino acid is substituted from A to C in the amino acid sequence of the parent CXCR3 ligand;

(F11) the CXCR3 ligand according to any one of (F7) to (F10), wherein the modification of an amino acid in an N-terminal region is substitution of the amino acid at position 1 in the amino acid sequence of the parent CXCR3 ligand with Tyr;

(F12) the CXCR3 ligand according to any one of (F7) to (F11), wherein the first amino acid from the N-terminus in the amino acid sequence of the parent CXCR3 ligand is V and the modification of an amino acid in an N-terminal region comprises the amino acid substitution of V1Y;

(F13) the CXCR3 ligand according to any one of (F1) to (F13), wherein the second amino acid from the N-terminus in the amino acid sequence of the parent CXCR3 ligand is P and the modification of an amino acid in an N-terminal region comprises the amino acid substitution of P2V;

(F14) the CXCR3 ligand according to any one of (F1) to (F13), further wherein at least one modification selected from an amino acid substitution, deletion, and insertion is made;

(F15) the CXCR3 ligand according to any one of (F1) to (F14), wherein the parent CXCR3 ligand is resistant to furin protease;

(F16) the CXCR3 ligand according to any one of (F1) to (F15), wherein the parent CXCR3 ligand is a CXCL10 variant produced by adding the R75A modification to the amino acid sequence of the naturally-occurring human CXCL10;

(F17) a method of improving the stability in blood of a CXCR3 ligand, wherein the method comprises substituting the amino acid corresponding to amino acid position 7 when taking the N-terminal amino acid in the amino acid sequence of the parent CXCR3 ligand as position 1 with Pro;

(F18) the method according to (F17), wherein the parent CXCR3 ligand comprises at least a portion of the amino acid sequence of a naturally-occurring human CXCL10;

(F19) a method according to (F17) or (F18), wherein the method further comprises adding at least one modification selected from substitution, deletion, and insertion of an amino acid;

(F20) a method of producing a CXCR3 ligand having enhanced stability in blood relative to a parent ligand, wherein the method comprises substituting the amino acid corresponding to amino acid position 7 when taking the N-terminal amino acid in the amino acid sequence of the parent CXCR3 ligand as position 1 with Pro;

(F21) the method according to (F20), wherein the parent CXCR3 ligand comprises at least a portion of the amino acid sequence of a naturally-occurring human CXCL10;

(F22) the method according to (F20) or (F21), wherein the method further comprises adding at least one modification selected from substitution, deletion, and insertion of an amino acid; and (F23) a CXCR3 ligand produced by the method according to any one of (F20) to (F22).

[G]

(G1) A polypeptide, wherein an amino acid corresponding to amino acid position 7 when taking the N-terminal amino acid of a naturally-occurring human CXCL10 as position 1 is substituted with Pro in the amino acid sequence of the naturally-occurring human CXCL10 or a CXCL10 variant;

(G2) the polypeptide according to (G1), further wherein an amino acid corresponding to position 18 and an amino acid corresponding to position 60 of the naturally-occurring human CXCL10 are substituted with Cys;

(G3) the polypeptide according to (G1) or (G2), further wherein an amino acid corresponding to position 1 of the naturally-occurring human CXCL10 is Tyr;

(G4) the polypeptide according to (G1) to (G3), further wherein an amino acid corresponding to position 2 of the naturally-occurring human CXCL10 is Val;

(G5) a polypeptide, which is a CXCR3 ligand and has the sequence of Y-P-L-S-R-T-P (SEQ ID NO: 67), Y-V-L-S-R-T-P (SEQ ID NO: 68), V-P-L-S-R-T-P (SEQ ID NO: 70), or V-V-L-S-R-T-P (SEQ ID NO: 71) at the N-terminus; and

(G6) a polypeptide having the following amino acid sequence:

(X1)(X2)LSRT(X6)RCT CISISNQ(X3)VN PRSLEKLEII PASQFCPRVE IIATMKKKG EKRCLNPESK(X4) IKNLLKAVSK ERSK(X5)SP (SEQ ID NO: 69);

wherein (X1) is V or Y, (X2) is P or V, (X3) is P or C, (X4) is A or C, (X5) is R or A, and (X6) is P.

[H]

(H1) An isolated nucleic acid encoding the CXCR3 ligand according to [F] or the polypeptide according to [G];

(H2) a vector having the nucleic acid according to (H1);

(H3) a host cell comprising the nucleic acid according to (H1) or the vector according to (H2);

(H4) a method of producing the CXCR3 ligand according to [F] or the polypeptide according to [G], comprising culturing the host cell according to (H3) such that the CXCR3 ligand or the polypeptide is produced;

(H5) the method according to (H4), further comprising recovering the polypeptide or the CXCR3 ligand from the host cell;

(H6) a fusion protein comprising the CXCR3 ligand according to [F] or the polypeptide according to [G];

(H7) the fusion protein according to (H6), wherein the CXCR3 ligand according to [F] or the polypeptide according to [G] is fused with an antibody Fc region;

(H8) the fusion protein according to (H6), wherein the CXCR3 ligand according to [F] or the polypeptide according to [G] is fused with an intact antibody or an antibody fragment;

(H9) the fusion protein according to (H8), wherein the intact antibody or antibody fragment has at least one cleavage site, and binding with the CXCR3 ligand or the polypeptide is reduced when in a state where the intact antibody or the antibody fragment is cleaved at at least one cleavage site;

(H10) the fusion protein according to (H9), wherein in a state where the cleavage site is cleaved, the ligand or the polypeptide dissociates from the intact antibody or the antibody fragment;

(H1 1) the fusion protein according to (H9) or (H10), wherein the cleavage site comprises a protease cleavage sequence;

(H12) the fusion protein according to (H1 1), wherein the protease is a target tissue-specific protease;

(H13) the fusion protein of (H12), wherein the target tissue is a cancer tissue or an inflamed tissue, and the target tissue-specific protease is a cancer tissue-specific protease or an inflamed tissue-specific protease;

(H14) the fusion protein according to any one of (H1 1) to (H13), wherein the protease is at least one protease selected from metalloprotease, serine protease, aspartic protease, cysteine protease, threonine protease, matriptase, and urokinase (uPA);

(H15) the fusion protein according to any one of (H9) to (H14), wherein a flexible linker is added to one end or both ends of the cleavage site or the protease cleavage sequence;

(H16) the fusion protein according to any one of (H9) to (H15), wherein the intact antibody or the antibody fragment has an antibody constant region, antibody VH, and antibody VL, and the cleavage site or the protease cleavage sequence is located at at least one of the following positions:

near the boundary between the antibody constant region and the antibody VH; and
near the boundary between the antibody constant region and the antibody VL;

(H17) the fusion protein according to (H16), wherein the cleavage site or the protease cleavage sequence is inserted into at least one of the following:

(i) an arbitrary position in the sequence from the 109th amino acid (Kabat numbering) of the antibody VH to the 122nd amino acid (EU numbering) of the antibody heavy chain constant region; and
(ii) an arbitrary position in the sequence from the 104th amino acid (Kabat numbering) of the antibody VL to the 113rd amino acid (EU numbering) of the antibody light chain constant region;

(H18) the fusion protein according to (H16) or (H17), wherein the antibody VL and the antibody VH of the intact antibody or the antibody fragment are associated, and the association is cancelled by cleavage of the cleavage site or is cancelled by cleavage of the protease cleavage sequence by a protease;

(H19) the fusion protein according to (H7) to (H18), wherein the CXCR3 ligand or the polypeptide and the antibody Fc region, or the CXCR3 ligand or the polypeptide and the intact antibody or the antibody fragment are fused via a linker;

(H20) a pharmaceutical composition comprising the CXCR3 ligand according to [A], the polypeptide according to [B], or the fusion protein according to any one of (H6) to (H19); and (H21) a method of treating a disease, wherein the method comprises administering the CXCR3 ligand according to [A], the polypeptide according to [B], or the fusion protein according to any one of (H6) to (H19).

[Brief Description of Drawings]

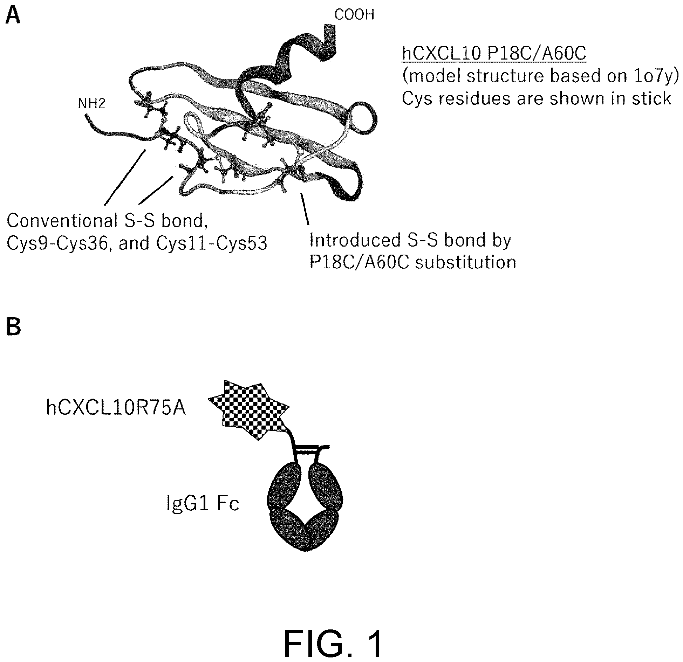

Fig. 1 shows the molecular structure of a CXCL10 variant. Fig. 1A shows the model structure (ribbon model) of the two pre-existing disulfide bonds (Conventional S-S bond, Cys9-Cys36 and Cys11-Cys53) and of a disulfide bond formed by the two cysteine substitutions (Introduced S-S bond by P18C/A60C substitutions). Fig. 1B shows a schematic diagram displaying the structure of the hCXCL10 mutant Fc fusion prepared in Example 1.

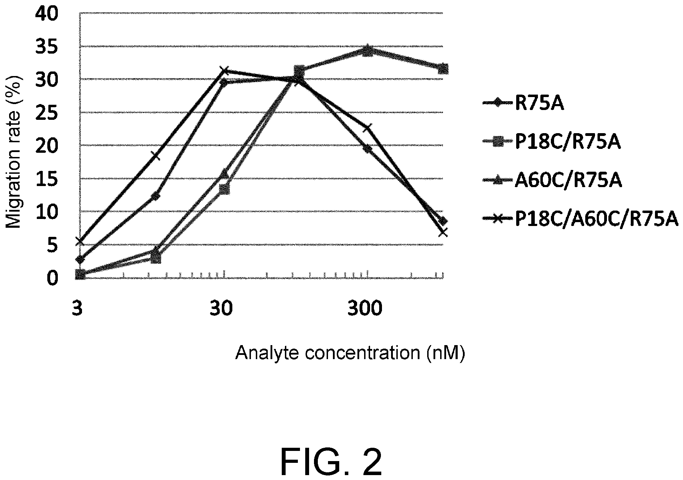

Fig. 2 is a graph showing the results of comparing the ability of CXCL10 variants (#1, #5, #6, and #7) in inducing BaF3/mCXCR3 cells. Table 3 shows the amino acid substitution(s) introduced into each mutant. In the figure, the ordinate shows the migration rate while the abscissa shows the concentration (nM) of the chemokines (CXCL10 variants). The migration rate was calculated as a relative value of luminescence from the lower chamber to luminescence obtained from the total amount of cells loaded in the upper chamber, *i.e.,* **"luminescence value/luminescence value from all cells".**

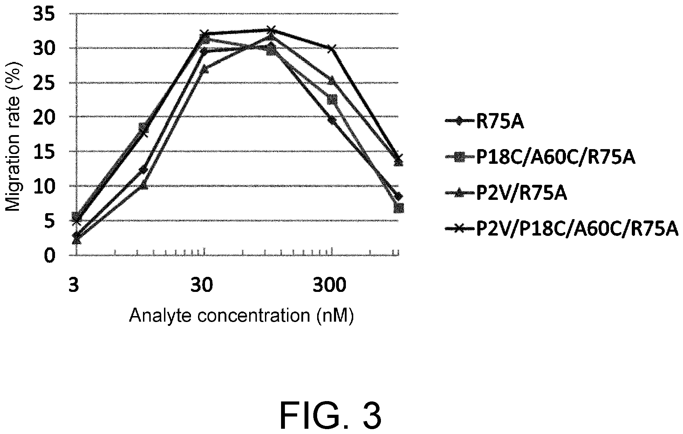

Fig. 3 is a graph showing the results of comparing the ability of CXCL10 variants (#1, #7, #8, and #10) in inducing BaF3/mCXCR3 cells. Table 4 shows the amino acid substitution(s) introduced into each mutant. In the figure, the ordinate shows the migration rate while the abscissa shows the concentration (nM) of the chemokines (CXCL10 variants). The migration rate was calculated as a relative value of luminescence from the lower chamber to luminescence obtained from the total amount of cells loaded in the upper chamber, *i.e.,* **"luminescence value/luminescence value from all cells".**

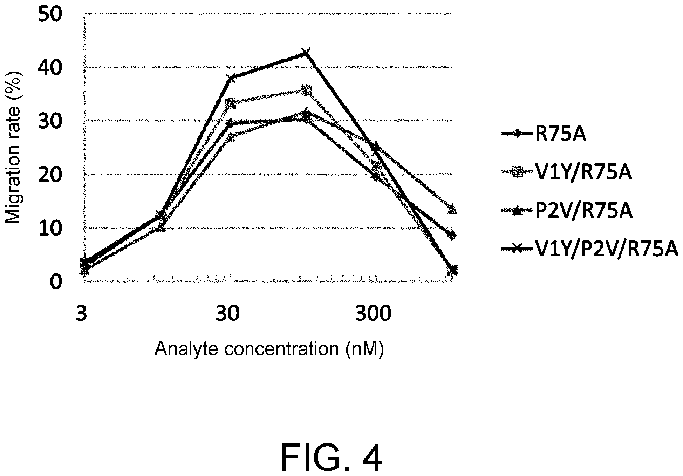

Fig. 4 is a graph showing the results of comparing the ability of CXCL10 variants (#1, #4, #8, and #9) in inducing BaF3/mCXCR3 cells. Table 5 shows the amino acid substitution(s) introduced into each mutant. In the figure, the ordinate shows the migration rate while the abscissa shows the concentration (nM) of the chemokines (CXCL10 variants). The migration rate was calculated as a relative value of luminescence from the lower chamber to luminescence obtained from the total amount of cells loaded in the upper chamber, *i.e.,* **"luminescence value/luminescence value from all cells".**

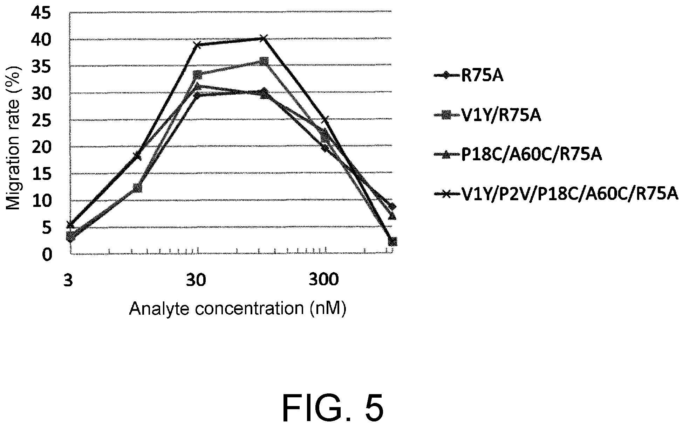

Fig. 5 is a graph showing the results of comparing the ability of CXCL10 variants (#1, #4, #7, and #12) in inducing BaF3/mCXCR3 cells. Table 6 shows the amino acid substitution(s) introduced into each mutant. In the figure, the ordinate shows the migration rate while the abscissa shows the concentration (nM) of the chemokines (CXCL10 variants). The migration rate was calculated as a relative value of luminescence from the lower chamber to luminescence obtained from the total amount of cells loaded in the upper chamber, *i.e.,* **"luminescence value/luminescence value from all cells".**

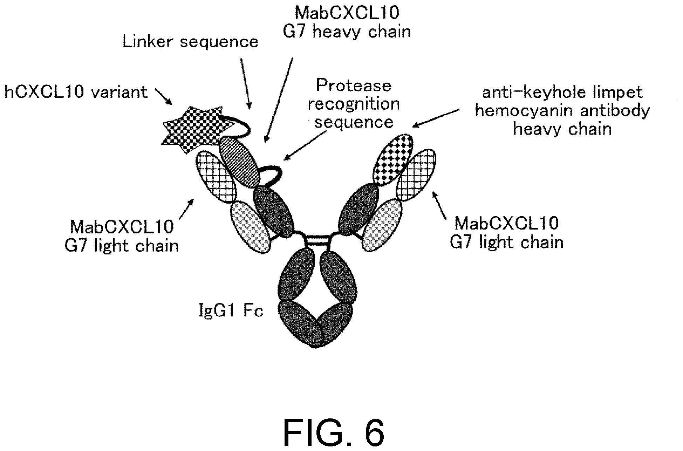

Fig. 6 is a schematic diagram showing the molecular structure of the hCXCL10 fusion-type anti-hCXCL10 antibody produced in Example 3.

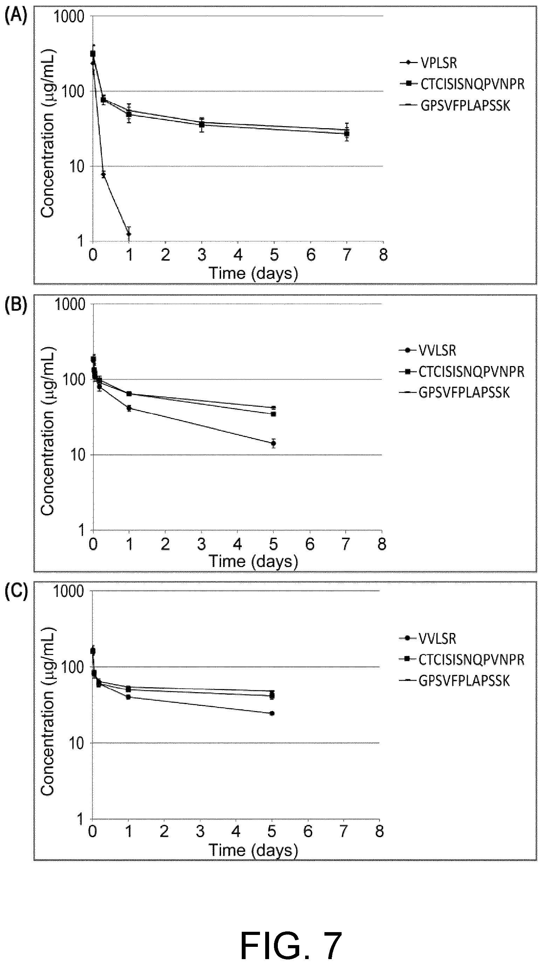

Fig. 7 is a set of graphs showing the changes in concentrations in the plasma after administration of the antibody (A) hCXCL10R75A.G4SGGGG.G7HFR0039H.12aa0054-G1T4h/G7L-LT0//IC17HdK-G1T4k.H435R/G7L-LT0, (B) hCXCL10R75A.0016.G4SGGGG.G7HFR0039H.12aa0054-G1T4h/G7L-LT0//IC17HdK-G1T4k.H435R/G7L-LT0, or (C) hCXCL10R75A.0303.G4SGGGG.G7HFR0039H.12aa0054-G1T4h/G7L-LT0//IC17HdK-G1T4k.H435R/G7L-LT0, which were quantified by each of the monitor peptides.

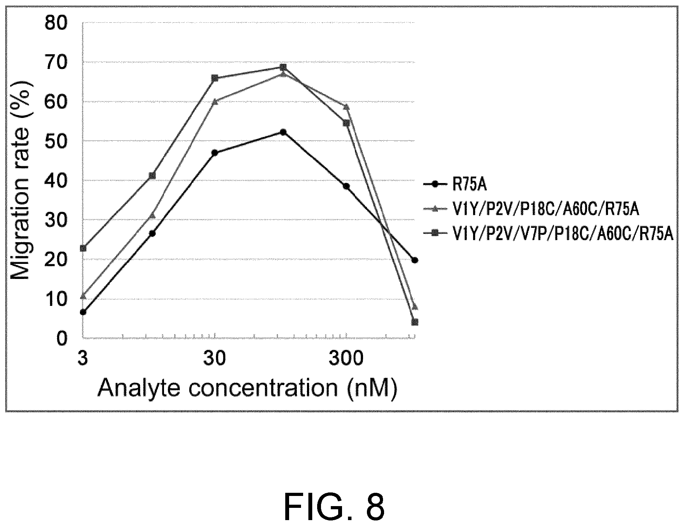

Fig. 8 is a graph showing the cell migration-inducing activities of Fc fusions of hCXCL10 variants: hCXCL10R75A (having R75A), hCXCL10R75A.0670 (having V1Y/P2V/P18C/A60A), and hCXCL10R75A.0613 (having V1Y/P2V/V7P/P18C/A60A).

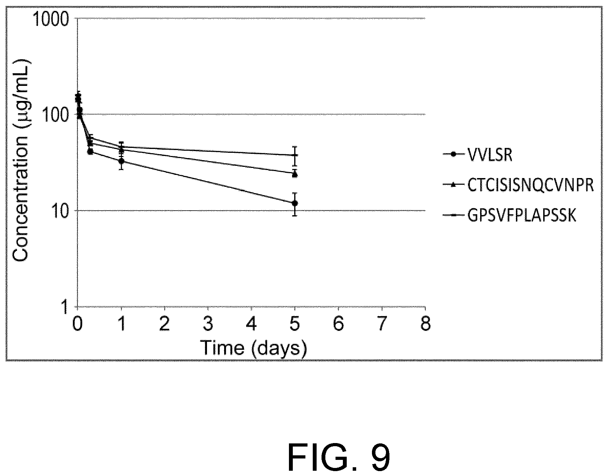

Fig. 9 is a graph showing the changes in concentrations in the plasma after administration of the hCXCL10 fusion-type anti-hCXCL10 antibody hCXCL10R75A.0659.G4SGGGG.G7HFR0039H.0004.N0222-G1T4h/G7L.R38E-LT0//IC17HdK-G1T4k.H435R/G7L.R38E-LT0, which were quantified by each of the monitor peptides.

[Description of Embodiments]

The following definitions and detailed descriptions are provided to facilitate the understanding of the present disclosure explained herein.

Amino acids

In the present specification, each amino acid is indicated by one-letter code or three-letter code, or both, as represented by, for example, Ala/A, Leu/L, Arg/R, Lys/K, Asn/N, Met/M, Asp/D, Phe/F, Cys/C, Pro/P, Gln/Q, Ser/S, Glu/E, Thr/T, Gly/G, Trp/W, His/H, Tyr/Y, Ile/I, or Val/V.

Amino acid modifications

For the modification of an amino acid in the amino acid sequence of a CXCR3 ligand, a method known in the art such as site-directed mutagenesis (Kunkel et al. (Proc. Natl. Acad. Sci. USA (1985) 82, 488-492)) or overlap extension PCR can be appropriately used. Several methods known in the art can also be used as amino acid modification methods for substituting an amino acid with an amino acid other than a naturally-occurring amino acid (Annu. Rev. Biophys. Biomol. Struct. (2006) 35, 225-249; and Proc. Natl. Acad. Sci. U.S.A. (2003) 100 (11), 6353-6357). For example, a tRNA-containing cell-free translation system (Clover Direct (Protein Express)) in which a non-naturally-occurring amino acid is bound to an amber suppressor tRNA complementary to the UAG codon (amber codon), which is a stop codon, is also preferably used.

In the present specification, the term "and/or" used to represent amino acid modification sites is meant to include every combination in which "and" and "or" are appropriately combined. Specifically, for example, the phrase "amino acids at positions 33, 55, and/or 96 are substituted" includes the following variations of amino acid modification:

(a) position 33, (b) position 55, (c) position 96, (d) positions 33 and 55, (e) positions 33 and 96, (f) positions 55 and 96, and (g) positions 33, 55, and 96. Alternatively, the phrase "amino acids at positions 33, 55, and/or 96 are substituted" is synonymous to the phrase "an amino acid at least one (one, two, or three) position selected from the group consisting of positions 33, 55, and **96 is substituted".**

Further, in the present specification, "an amino acid corresponding to position X" which is used to express the site of an amino acid modification means a homologous amino acid residue after an amino acid sequence is aligned when an amino acid position is shifted by modification such as insertion or deletion. Specifically, in a variant of a wild-type ligand for example, an amino acid corresponding to position X of the amino acid position in the amino acid sequence of the wild-type ligand is an amino acid residue homologous thereto after the amino acid sequence of the wild-type ligand and the amino acid sequence of the variant are aligned. The position may be the same as position X in the amino acid sequence of the variant and it may be different when the amino acid position is changed due to insertion or deletion.

In the present specification, amino acid modifications include not only changing the structure of amino acids but also changing into different amino acid sequences by mutating part or all of an amino acid sequence. Modification of amino acid sequences can be prepared by introducing an appropriate codon substitution into nucleotide sequences encoding the amino acid sequences, or by peptide synthesis. An amino acid modification includes, for example, deletion from amino acid sequences and/or insertion into amino acid sequences and/or substitution of a residue in amino acid sequences. When an appropriate codon substitution is introduced into nucleotide sequences encoding amino acid sequences, peptides consisting of amino acid sequences after the substitution can be obtained by translating nucleotide sequences after the substitution into peptides encoded by them. Alternatively, when substitution is achieved by peptide synthesis, peptides consisting of amino acid sequences after the substitution can be directly obtained. The function and properties of final constructs can vary depending on amino acid modifications. Any combination of deletion, insertion, and substitution can be made to arrive at the final constructs, provided that the final constructs possess a desired characteristic. That is, in the present invention, amino acid modifications can include any selected from the group consisting of an amino acid deletion, insertion into an amino acid sequence, and substitution of a residue in an amino acid sequence.

In the present specification, an expression in which the one-letter codes or three-letter-codes of amino acids before and after modification are written prior to and subsequent to a number representing a particular position can be appropriately used for representing an amino acid substitution. For example, the modification P2A or Pro2Ala used for substituting an amino acid contained in a CXCR3 ligand represents the substitution of Pro at position 2 from the N-terminus (which may also be expressed herein as the second amino acid from the N-terminus, position 2 in the amino acid sequence, the second amino acid in the amino acid sequence, and the like) of the CXCR3 ligand with Ala. Specifically, the number represents an amino acid position as counted from the N-terminus of the CXCR3 ligand; the one-letter code or three-letter code of the amino acid written prior to the number represents the amino acid before the substitution; and the one-letter code or three-letter code of the amino acid subsequent to the number represents the amino acid after the substitution.

Polypeptides

Polypeptides according to the present invention usually refer to peptides having a length of about 4 amino acids or longer, and proteins. That is, polypeptides are a series of peptides connected through amide bonds or proteins comprising a plurality of a series of polypeptides connected through amide bonds. Furthermore, polypeptides according to the present invention are usually polypeptides consisting of an artificially designed sequence but are not particularly limited thereto, and may be, for example, polypeptides derived from an organism. Polypeptides according to the present invention may be any of naturally-occurring polypeptides, synthetic polypeptides, recombinant polypeptides, and so on. Moreover, fragments of the polypeptides are also included in polypeptides according to the present invention.

CXCR3

Chemokine receptor CXCR3 (also called G Protein-coupled Receptor 9 (GPR9) and CD 183) belongs to the CXC chemokine family and is a G Protein-coupled receptor that binds to the chemokines CXCL9, CXCL10, and CXCL11. CXCR3 is expressed primarily in activated T-helper type 1 (Th1) lymphocytes, but is also present in natural killer cells, macrophages, dendritic cells, and B lymphocyte subsets. The chemokines CXCL9, CXCL10, and CXCL11 are three naturally-occurring CXCR3 ligands. The interaction of CXCR3 and its ligands (hereinafter referred to as the CXCR3 axis) is involved in guiding receptor-bearing cells to specific parts of the body, especially sites of inflammation, immune impairment, and immune dysfunction. Unless otherwise indicated, the term "CXCR3" as used herein indicates any natural CXCR3 from any vertebrate source, including mammals such as primates (e.g., humans) and rodents (e.g., mice and rats). The entire amino acid sequence of human CXCR3 is shown by Refseq: NP_001495.

In a particular embodiment, CXCR3 is a naturally-occurring human CXCR3 (SEQ ID NO: 51) and cells expressing CXCR3 are cells expressing naturally-occurring human CXCR3.

Chemokines and CXC chemokines

Chemokines are a family of homogeneous serum proteins of 7 to 16 kDa originally characterized by their ability to induce leukocyte migration. Most of chemokines have four characteristic cysteines (Cys) and are classified into C-X-C (or alpha, CXC), C-C (or beta), C (or gamma), and CX3C (or delta) chemokine classes, according to motifs displayed by the first two cysteines. Two disulfide bonds are formed between the first and third cysteines and between the second and fourth cysteines. In general, the disulfide bridges are considered necessary. Clark-Lewis and collaborators have reported that the disulfide bonds are crucial for the chemokine activity of at least CXCL10 (Clark-Lewis et al., J. Biol. Chem. 269: 16075-16081, 1994).

Subfamilies of C-X-C (or alpha, CXC) are further classified, according to the presence of an ELR motif (Glu-Leu-Arg) preceding the first cysteine, into two groups: ELR-CXC chemokines and non-ELR-CXC chemokines (see e.g., Clark-Lewis, supra; and Belperio et al., J. Leukoc. Biol. 68: 1-8, 2000). CXCL10, CXCL11, and CXCL9 are all non-ELR-CXC chemokines.

CXCL 10

CXC motif chemokine 10 (C-X-C motif chemokine ligand 10, CXCL10) is a C-X-C chemokine, which is also called interferon-induced protein-10 (IP-10). It is induced by interferon-$\gamma$ **and TNF-$\alpha$ and produced by keratinocytes, endothelial cells, fibroblasts, and** monocytes. CXCL10 is thought to play a role in the recruitment of activated T cells to sites of tissue inflammation (Dufour, et al., J Immunol., 168: 3195-204, 2002). In addition, CXCL10 may play a role in hypersensitivity reactions. It may also play a role in the development of inflammatory demyelinating neuropathy (Kieseier, et al., Brain 125: 823-34, 2002).

Researches indicate that CXCL10 may be useful in the engraftment of stem cells following transplantation (Nagasawa, T., Int. J. Hematol. 72: 408-11, 2000), in the mobilization of stem cells (Gazitt, Y., J. Hematother Stem Cell Res 10: 229-36, 2001; and Hattori et al., Blood 97: 3354-59, 2001), and in an enhancement of antitumor immunity (Nomura et al., Int. J. Cancer 91: 597-606, 2001; and Mach and Dranoff, Curr. Opin. Immunol. 12: 571-75, 2000). For example, previous reports known to those skilled in the art discuss the biological activity of chemokine (Bruce, L. et al., Methods in Molecular Biology (2000) vol. 138, pp. 129-134; Raphaele, B. et al., Methods in Molecular Biology (2000) vol. 138, pp. 143-148; and Paul D. Ponath et al., Methods in Molecular Biology (2000) vol. 138, pp. 113-120). The physiological activity of CXCL10 is exerted by binding to the chemokine receptor CXCR3 expressed on the cell surface (Booth V. et al, Biochemistry. 41 (33): 10418-25).

**Unless otherwise indicated, the term "CXCL10" as used herein refers to any natural** CXCL10 from any vertebrate source, including mammals such as primates (e.g., humans) and **rodents (e.g., mice and rats). The term refers not to the "full-length", unprocessed CXCL10, but** to mature CXCL10 secreted extracellularly as a result of intracellular processing. As used herein, those that have not undergone processing are referred to as CXCL10 precursors. The term also encompasses naturally-occurring mutants of CXCL10 such as splice mutants and allelic mutants. Naturally-occurring human CXCL10 is expressed as a CXCL10 precursor (Refseq Accession number: NP_001556) and then secreted extracellularly as a protein with the sequence set forth in SEQ ID NO: 25. The complete amino acid sequence of the rhesus monkey CXCL10 precursor is indicated by Refseq Accession number: AKK95955, and the complete amino acid sequence of the mouse CXCL10 precursor is indicated by Refseq Accession number: NP_067249. The **expression "animal source" not only implies that a molecule of interest was isolated from** biological materials obtained from animals but also that artificially synthesized protein molecules have the same amino acid sequence as a natural CXCL10.

Natural CXCL10 or naturally-occurring CXCL10 mutants to which artificial amino acid **modifications have been added are called "CXCL10 variants". CXCL10 variants may also be** referred to as recombinant CXCL10 polypeptides.

CXCL 11

C-X-C motif chemokine 11 (CXC motif chemokine ligand 11, CXCL11) is a C-X-C chemokine, which is also called I-TAC (Interferon-inducible T-cell alpha chemoattractant) or IP-9 (Interferon-gamma-inducible Protein 9). CXCL11 gene expression is strongly induced by **IFN-$\gamma$ and IFN-$\beta$, and also by IFN-$\alpha$ (**Rani MR, The Journal of Biological Chemistry. 271 (37): 22878-84).

CXCL11 has a biological activity to activate T cells. CXCL11 is known to exert its biological activity by binding to the chemokine receptor CXCR3 expressed on the cell surface, and naturally-occurring CXCL11 is said to bind to CXCR3 more strongly than naturally-occurring CXCL10 and naturally-occurring CXCL9 (Cole KE., The Journal of Experimental Medicine. 187 (12): 2009-21; Tensen CP, The Journal of Investigative Dermatology. 112 (5): 716-22).

**The term "CXCL11" as used herein refers to any natural CXCL11 from any vertebrate** source, including mammals such as primates (e.g., humans) and rodents (e.g., mice and rats), **unless otherwise indicated. The term**

**refers not to a "full-length", unprocessed CXCL11, but to a** mature CXCL11 that is secreted extracellularly as a result of intracellular processing. As used herein, those that have not undergone processing are referred to as CXCL 11 precursors. The term also includes naturally-occurring mutants of CXCL11, such as splice mutants and allelic mutants. Natural human CXCL11 is expressed as a CXCL11 precursor (Refseq Accession number: NP_005400) and then secreted extracellularly as a protein with the sequence set forth in SEQ ID NO: 26.

Natural CXCL11 or naturally-occurring CXCL11 mutants to which artificial amino acid **modifications have been added are called "CXCL11 variants". CXCL11 variants may also be** referred to as recombinant CXCL 11 polypeptides.

CXCL9

C-X-C motif chemokine 9 (C-X-C motif chemokine ligand 9, CXCL9) is a C-X-C chemokine, which is also called Monokine induced by gamma interferon (MIG). CXCL9 is an IFN-$\gamma$-induced T-cell chemoattractant and is known to exert its biological activity by binding to the chemokine receptor CXCR3 expressed on the cell surface.

**The term "CXCL9" as used herein refers to any natural CXCL9 from any vertebrate** source, including mammals such as primates (e.g., humans) and rodents (e.g., mice and rats), **unless otherwise indicated. The term refers not to "full-length", unprocessed CXCL9, but to** mature CXCL9 that is secreted extracellularly as a result of intracellular processing. As used herein, those that have not undergone processing are referred to as CXCL9 precursors. The term also includes naturally-occurring mutants of CXCL9, such as splice mutants and allelic mutants. Natural human CXCL9 is expressed as a CXCL9 precursor (Refseq Accession number: NP_002407) and then secreted extracellularly as a protein with the sequence set forth in SEQ ID NO: 27.

Natural CXCL9 or naturally-occurring CXCL9 mutants to which artificial amino acid **modifications have been added are called "CXCL9 variants". CXCL**9 variants may also be referred to as recombinant CXCL9 polypeptides.

Chimeric proteins can be prepared by fusing parts of CXCL10, CXCL11, CXCL9 and such with each other. For example, a human CXCL10-human CXCL11 chimeric protein (hITIP) (SEQ ID NO: 28) in which the 1st to 24th amino acid residues of human CXCL11 (SEQ ID NO: 26) and the 25th to 77th amino acid residues of a human CXCL10 variant (SEQ ID NO: 1) are bound can be prepared. Such chimeric proteins can also be called CXCR3 ligands as long as they can bind to CXCR3.

The human CXCL10-human CXCL11 chimeric protein (hITIP) (SEQ ID NO: 28), in which the 1st to 24th amino acid residues of human CXCL11 (SEQ ID NO: 26) and the 25th to 77th amino acid residues of a human CXCL10 variant (SEQ ID NO: 1) are bound, and to which **further amino acid modifications have been added is called an "hITIP variant".**

Interaction

In the context of compounds (*e.g.*, peptide compounds) that interact with specific amino **acid residues on target proteins, "interaction" means non-**covalent interaction exemplified by hydrogen bonds, ionic bonds, hydrophobic interaction, and van der Waals interaction, or bonds through other molecules such as water molecules. In the present specification, a specific amino acid residue in a protein and a compound can be determined to have interaction when the interatomic distance between a non-hydrogen atom included in the amino acid residue and a non-hydrogen atom included in the compound (when the bond is via other molecules such as water molecules, the interatomic distance to the other molecules) is four angstrom (**Å) or less. The** interatomic distance can be determined using, for example, an X-ray analyzer and structure analysis software.

**A "change in interaction" means that the mode of non-**covalent interaction or bonds through other molecules such as water molecules is different. Examples include a change in the distance between interacting residues, a change to different residues, a change in the type of bonds involved (*e.g.*, from a hydrogen bond to an ionic bond), and such.

Activation of receptors by chemokines

Activation of receptors by chemokines is explained by the two-step model. The first step is a receptor binding step where ligands bind to an N-terminal region of receptors. The following second step is an activation step and the structure of the receptors is induced to an active form by interaction between an N-terminal region of the ligands and the receptors. Activation of receptors is also thought to follow the two-step model in C-X-C chemokines.

**The ligands' region critical for binding in the first step is called a docking domain and is** formed by the N-loop (the first loop) from the cysteine pair (C-X-C motif) conserved in C-X-C chemokines to $\beta$-strand 1 (the first $\beta$-strand), the 30s loop (the second loop) between $\beta$-strand 1 and $\beta$-strand 2 (the second $\beta$-strand), and the 40s loop (the third loop) between $\beta$-strand 2 and $\beta$-strand 3 (the third $\beta$-strand). As described above, C-X-C chemokines such as CXCL10 have three $\beta$-strands: the first $\beta$-strand is located closest to an N-terminal side, $\beta$-strand 2 is located second closest to the N-terminal side, and β-strand 3 is located third closest to the N-terminal side.

In the second step, the N-terminal region of the ligands called a triggering domain interacts with the transmembrane regions of CXCR3 and induces the receptors to an activated structure. Since the N-terminal region of the ligands directly contributes to the structural change of the receptors, substitution or deletion of amino acid residues in this part is known to decrease activation efficiency of the receptors (Booth, Keizer et al. 2002, Clark-Lewis, Mattioli et al. 2003, Booth, Clark-Lewis et al. 2004, Allen, Crown et al. 2007, Aguilera-Duran and Romo-Mancillas 2020).

In the present specification, the N-terminal region of CXCR chemokines is a region at a side closer to the N-terminus than the C-X-C motif. That is, amino acids in the N-terminal region are amino acids located at a side closer to the N-terminus than the C at the N-terminal side of the cysteine pair conserved in CXC chemokines. For example, amino acids in the N-terminal region are amino acids at amino acid positions 1 to 8 when a CXCR chemokine is naturally-occurring CXCL10 or a variant thereof.

The steric structure of ligands is known to be critical for binding to receptors in the first step.

X-C motif chemokine ligand 1 (X-C motif chemokine 1, X-C motif chemokine ligand 1, or XCL1) is a chemokine, the receptor of which is XCR1, and contributes to activation of dendritic cells and T cells. XCL1 lacks a cysteine residue in an N-terminal region that is conserved in other chemokine families and forms only one disulfide bond in a molecule. Chemokines generally have the steric structure of the chemokine fold (α+β fold) formed by three β-strands and one α-helix. Meanwhile, XCL1 has less stable steric structure than other chemokine families due to the lack of the disulfide bond and is known to be present in a state of equilibrium between the chemokine form, which can bind to and activate the receptor, and the alternate form, which cannot bind to and activate the receptor (Dishman, Tyler et al. 2021). In view of this, the technique to modify XCL1 by introducing an additional disulfide bond into XCL1 so that the chemokine fold is preferentially formed like in other chemokines is reported. The modified XCL1 into which a disulfide bond is additionally introduced is shown to be able to activate XCR1-expressing cells at lower concentrations relative to wild-type XCL1 and accumulate many XCR1-expressing dendritic cells at an administration site when subcutaneously administered to mice (Tuinstra, Peterson et al. 2007, Matsuo, Kitahata et al. 2018).

Though not mentioned directly, enhancement of activity by structural fixation of XCL1 can be explained by the conformational selection model in protein-protein interaction. In this model, proteins are considered to have a certain level of flexibility in solution and be able to bind to their partners only after the structure of each protein reaches a state suitable for binding. Accordingly, if the structure of proteins can be fixed to a state close to a bonded structure, binding of the proteins is promoted as a result (Boehr, Nussinov et al. 2009).

In contrast, CXCR3 ligands such as CXCL10 have a cysteine residue in an N-terminal region and have the steric structure of the chemokine fold (α+β fold) formed by three β-strands and one α-helix. The alternate fold like XCL1 is not observed. Thus, introduction of a disulfide bond, which was useful in enhancing XCL1 activity, appears not so useful in enhancing CXCL10 activity.

The inventors of the present invention considered the possibility that an N-terminal region, the α-helix region at the C-terminus, and the docking domain centering around the N-loop are locally in a state of equilibrium between a structure binding to CXCR3 and a structure not binding to CXCR3, and examined modifications that stabilize these regions in CXCL10 to a state binding to CXCR3, based on conformational selection.

In activation in the second step, the N-terminal region of ligands interacts with the transmembrane region of receptors, and the interaction induces the structural change and activation of the receptors. Thus, the N-terminal region of the ligands is a region that greatly affects activation of the receptors.

It is known that ligands with this region cleaved lose activity in many cases. Meanwhile, it is also reported that the original sequence of some chemokines such as CXCL8 and CCL3L1 is in an inactive state and becomes an active state when cleaved by proteases. From such backgrounds, cleavage of an N-terminal region of chemokines by proteases is thought to be responsible for regulation of chemokine activity (Allen, Crown et al. 2007, Mortier, Van Damme et al. 2008).

Cleavage of an N-terminal region of chemokines has little effect on binding to receptors. Therefore, this region can be considered to be a region that regulates chemokine activity independently from binding to receptors.

There are many reports including the above literature about techniques to make chemokines in an inactive state to an active state. Furthermore, inactivation of CXCL10 by cleavage of the N-terminus of CXCL10 is known (Proost, Schutyser et al. 2001, Repnik, Starr et **al. 2015). However, to the best of the author's knowledge, there has not been a report showing** enhancement of activity by modifications.

It is clear from the activation pattern that modifications of residues critical for interaction between an N-terminal region of ligands and receptors in the activation step result in reduced activity due to insufficient induction of structural change. Meanwhile, it is difficult to achieve modifications that more strongly induce the structure in an activated state by substituting residues already involved in the interaction.

The inventors of the present invention thought that penetration of an N-terminal region contributing to the activation step into the transmembrane regions and subsequent interaction can be stabilized by regulating the hydrophobicity and the size of side chains of the N-terminal region and attempted a search about substitution of amino acid residues in the N-terminal region.

(References Cited)

Allen, S. J., S. E. Crown and T. M. Handel (2007). "Chemokine: receptor structure, interactions, and antagonism." Annu Rev Immunol 25: 787-820.

Boehr, D. D., R. Nussinov and P. E. Wright (2009). "The role of dynamic conformational ensembles in biomolecular recognition." Nat Chem Biol 5(11): 789-796.

Booth, V., I. Clark-Lewis and B. D. Sykes (2004). "NMR structure of CXCR3 binding chemokine CXCL11 (ITAC)." Protein Sci 13(8): 2022-2028.

Booth, V., D. W. Keizer, M. B. Kamphuis, I. Clark-Lewis and B. D. Sykes (2002). "The CXCR3 Binding Chemokine IP-10/CXCL10: Structure and Receptor Interactions." Biochemistry 41(33): 10418-10425.

Clark-Lewis, I., I. Mattioli, J. H. Gong and P. Loetscher (2003). "Structure-function relationship between the human chemokine receptor CXCR3 and its ligands." J Biol Chem 278(1): 289-295.

Dishman, A. F., R. C. Tyler, J. C. Fox, A. B. Kleist, K. E. Prehoda, M. M. Babu, F. C. Peterson and B. F. Volkman (2021). "Evolution of fold switching in a metamorphic protein." Science 371(6524): 86-90.

Matsuo, K., K. Kitahata, F. Kawabata, M. Kamei, Y. Hara, S. Takamura, N. Oiso, A. Kawada, O. Yoshie and T. Nakayama (2018). "A Highly Active Form of XCL1/Lymphotactin Functions as an Effective Adjuvant to Recruit Cross-Presenting Dendritic Cells for Induction of Effector and Memory CD8(+) T Cells." Front Immunol 9: 2775.

Mortier, A., J. Van Damme and P. Proost (2008). "Regulation of chemokine activity by posttranslational modification." Pharmacol Ther 120(2): 197-217.

Proost, P., E. Schutyser, P. Menten, S. Struyf, A. Wuyts, G. Opdenakker, M. Detheux, M. Parmentier, C. Durinx and A.-M. Lambeir (2001). "Amino-terminal truncation of CXCR3 agonists impairs receptor signaling and lymphocyte chemotaxis, while preserving antiangiogenic properties." Blood, The Journal of the American Society of Hematology 98(13): 3554-3561.

Repnik, U., A. E. Starr, C. M. Overall and B. Turk (2015). "Cysteine Cathepsins Activate ELR Chemokines and Inactivate Non-ELR Chemokines." J Biol Chem 290(22): 13800-13811.

Tuinstra, R. L., F. C. Peterson, E. S. Elgin, A. J. Pelzek and B. F. Volkman (2007). "An Engineered Second Disulfide Bond Restricts Lymphotactin/XCL1 to a Chemokine-like Conformation with XCR1 Agonist Activity." Biochemistry 46(10): 2564-2573.

Method for measuring the binding between a CXCR3 ligand and CXCR3

The binding of a CXCR3 ligand to CXCR3 can be assessed by a well-known method such as FACS, an ELISA format, a BIACORE method using amplified luminescent proximity homogeneous assay (ALPHA) screening or surface plasmon resonance (SPR) phenomena, or bio-layer interferometry (BLI) (Octet) (Proc. Natl. Acad. Sci. USA (2006) 103 (11), 4005-4010).

ALPHA screening is carried out based on the following principle according to ALPHA technology that uses two beads, a donor and an acceptor. Luminescence signals are detected only when molecules bound with the donor beads interact with molecules bound with the acceptor beads and when the two beads are close to one another. Laser-excited photosensitizers in the donor beads convert ambient oxygen into singlet oxygen in an excited state. The singlet oxygen molecules spread around the donor beads and when they reach the nearby acceptor beads, they induce chemiluminescent reaction in the beads to result in light emission. When the molecule bound with the donor bead and the molecule bound with the acceptor bead do not interact, chemiluminescent reaction does not occur because singlet oxygen produced by the donor bead does not reach the acceptor bead.

For example, a biotin-labeled CXCR3 ligand is bound to the donor bead, and a glutathione S transferase (GST)-tagged CXCR3 peptide is bound to the acceptor bead. The N-terminal extracellular domain region (1st to 53rd amino acids) or a partial fragment thereof (22nd to 42nd amino acids, Biochemistry (2002) 41, 10418-10425) can be used as the CXCR3 peptide. In addition, it is more desirable that the Y residue at position 27 and/or the Y residue at position 29 of CXCR3 have/has undergone sulfation modification (MOLECULAR AND CELLULAR BIOLOGY, Aug. 2006, p. 5838-5849). In the absence of competing untagged CXCR3 ligands, the CXCR3 ligand and CXCR3 peptide interact to produce a signal at 520-620 nm. The untagged CXCR3 ligand competes with the tagged CXCR3 ligand for interaction with the CXCR3 peptide. Decrease in fluorescence resulting from the competition can be quantified to determine relative binding affinity. Biotinylation of a CXCR3 ligand, such as CXCL10, using sulfo-NHS-biotin or the like is known in the art. A method which involves, for example, fusing a polynucleotide encoding the CXCR3 peptide in-frame with a polynucleotide encoding GST to form a fused gene, expressing the GST-fused CXCR3 peptide in cells or the like carrying a vector that permits expression of the fused gene, and purifying the GST-fused CXCR3 peptide using a glutathione column, can be appropriately adopted as a method for tagging a CXCR3 peptide with GST. The obtained signals are preferably analyzed using, for example, the software GRAPHPAD PRISM (GraphPad Software, Inc., San Diego) adapted to a one-site competition model based on non-linear regression analysis.

One (CXCR3 ligand) of the substances between which the interaction is to be observed is immobilized onto a thin gold film of a sensor chip. The sensor chip is irradiated with light from the back such that total reflection occurs at the interface between the thin gold film and glass. As a result, a site having a drop in reflection intensity (SPR signal) is formed in a portion of reflected light. The other one (analyte, the full-length CXCR3 or the aforementioned CXCR3 peptides can be used as the analyte when fixing a CXCR3 ligand) of the substances between which the interaction is to be observed is poured onto the surface of the sensor chip, and when the analyte binds with the CXCR3 ligand, the mass of the immobilized CXCR3 ligand molecule increases and results in the change of refractive index of the solvent on the sensor chip surface. This change in refractive index shifts the position of the SPR signal (in contrast, the position of the signal returns when dissociation occurs). The Biacore system plots on the ordinate the amount of the above-mentioned shift, i.e., change in mass on the sensor chip surface, and displays time-dependent change in mass as assay data (sensorgram). Kinetics (association rate constant (ka) and dissociation rate constant (kd)) is determined from the curve of the sensorgram, and dissociation constant (KD) is determined from the ratio between the two constants. Inhibition assay or equilibrium analysis is also preferably used in the BIACORE method. Examples of inhibition assay are described in Proc. Natl. Acad. Sci. USA (2006) 103 (11), 4005-4010, and examples of equilibrium analysis are described in Methods Enzymol. 2000; 323: 325-40. Using a similar method, the binding of a CXCR3 ligand to CXCR3 can be measured by immobilizing the full-length CXCR3 or the aforementioned CXCR3 peptides on a thin gold film of a sensor chip and pouring the CXCR3 ligand as the analyte. The full-length CXCR3 or the aforementioned CXCR3 peptides immobilized on the thin gold film of the sensor chip may be a purified protein or peptide. Alternatively, a cell expressing them or a cell membrane fraction thereof may also be used.

The CXCR3 ligand of the present disclosure can specifically bind to the full-length CXCR3 or to the aforementioned CXCR3 peptides with a dissociation constant (KD) of 100 $\mu$M, 10 $\mu$M, 1 $\mu$M, 100 nM, 50 nM, 10 nM, 5 nM, 1 nM, 500 pM, 400 pM, 350 pM, 300 pM, 250 pM, 200 pM, 150 pM, 100 pM, 50 pM, 25 pM, 10 pM, 5 pM, 1 pM, 0.5 pM, or 0.1 pM or less.

Alternatively, the binding activity of a CXCR3 ligand to the immobilized full-length CXCR3 or an aforementioned CXCR3 peptide can be evaluated based on the ELISA principle. For example, the full-length CXCR3 or an aforementioned CXCR3 peptide is immobilized in the wells of an ELISA plate. A CXCR3 ligand solution is brought into contact with the immobilized full-length CXCR3 or aforementioned CXCR3 peptide in the wells, and a CXCR3 ligand that binds to the immobilized full-length CXCR3 or aforementioned CXCR3 peptide is detected by an antibody that binds to the CXCR3 ligand. Alternatively, the CXCR3 ligand is immobilized in the wells of an ELISA plate, full-length CXCR3 or aforementioned CXCR3 peptide solution is brought into contact with the immobilized CXCR3 ligand in the wells, and the full-length CXCR3 or an aforementioned CXCR3 peptide that binds to the immobilized CXCR3 ligand is detected by an antibody that binds to the full-length CXCR3 or the aforementioned CXCR3 peptide.

As a method of measuring the binding between a CXCR3 ligand and CXCR3, there is also the method of labelling the CXCR3 ligand using a radioisotope. Specifically, a radioisotope-labeled CXCR3 ligand is prepared, added to CXCR3-expressing-cells, and incubated. The incubated sample is passed through a filter to adsorb the CXCR3 ligand bound to CXCR3 onto the filter together with the cells. The amount of CXCR3 ligand adsorbed on the filter can be measured by drying the filter and measuring the radiation amount. There is a report of applying this method to CXCL10 (Molecular and Cellular Biology, Aug. 2006, p. 5838-5849, Vol. 26, No. 15, The Journal of Biological Chemistry Vol. 278, No. 19, Issue of May 9, pp. 17066-17074, 2003).

In addition, by measuring the 15N-1H HSQC NMR spectrum and 2D NOESY NMR spectrum of a 15N-labeled CXCR3 ligand and comparing the NMR spectra obtained before and after the addition of a CXCR3 peptide, residues involved in CXCR3 binding within the CXCR3 ligand and the strength of the bond can also be analyzed (Biochemistry, 2002, 41, 10418-10425).

CXCR3 ligands

**The term "CXCR3 ligand" as used herein refers to a molecule capable of binding to** CXCR3. For example, proteins comprising sites that interact with the ligand-binding site of CXCR3 are included in the CXCR3 ligands of the present disclosure. The term refers to mature CXCR3 ligands that are secreted extracellularly as a result of intracellular processing, and not to **"full-length" proteins that have not undergone processing. As used herein, those that have not** undergone processing are referred to as CXCR3 ligand precursors. Moreover, the CXCR3 ligands of the present disclosure may exist in a form fused with an antibody, an antibody Fc region, another type of protein such as albumin, or a polypeptide. In the present specification, CXCR3 ligands include not only naturally-occurring CXCR3 ligands but also recombinant polypeptides and recombinant proteins produced by adding modifications thereto. In a specific embodiment, CXCR3 ligands are polypeptides that can bind to CXCR3.

In a specific embodiment, the CXCR3 ligands provided in the present specification have the activity to induce migration of cells expressing CXCR3. In a specific embodiment, the CXCR3 ligands provided in the present specification have the activity to induce migration of CXCR3-expressing-cells such as activated T-helper type 1 (Th1) lymphocytes, cytotoxic T cells, natural killer cells, macrophages, dendritic cells, B lymphocyte subsets, and some epithelial cells, endothelial cells, and Ba/F3 cells.

The activity of a target protein to cause migration of cells expressing CXCR3 can be measured using a transfectant or cells isolated from a living body which express CXCR3. As an example of a specific method, Ba/F3 transfectant cells expressing mouse CXCR3 (mCXCR3) (hereinafter BaF3/mCXCR3) and HTS Transwell™-96 Permeable **Supports with 5.0 μm Pore** Polycarbonate Membrane (Cat. 3387, Corning) are used, with the target protein as analyte. After adjusting the final concentration of each analyte to be analyzed in solution to a concentration **selected from 3 nM, 10 nM, 30 nM, 100 nM, 300 nM, and 1000 nM, 236 μL of each solution is** transferred to the lower chamber. Then, BaF3/mCXCR3 cells are seeded into the upper chamber **at 75 μL/well so as to be 2.0 × 10⁵** cells/well, and the reaction is carried out for 5 hours, under the **conditions of 5% carbon dioxide and 37 °C. After reacting for 5 hours, 100 μL of the solution in** the lower chamber is transferred to Assay plate, 96well, with Lid White, Flat Bottom (Cat, 3917, **Corning) and 100 μL of CellTiter-**Glo2.0™ Luminescent Cell Viability Assay solution (Cat. G9242, Promega) is added. After reacting at room temperature for 10 minutes, the luminescence value is measured with Envision (PerkinElmer) to evaluate the level of migration of cells into the lower chamber. The amount of cells that migrated into the lower chamber is reflected by the luminescence intensity.

The activity of target proteins to induce migration of cells expressing CXCR3 is measured by migration and invasion of cells to Boyden chamber (transwell) or tissue, namely cell migration rates.

In an embodiment, the CXCR3 ligands provided in the present specification are CXCR3 ligands having enhanced activity to induce migration of cells expressing CXCR3 relative to parent CXCR3 ligands, where either or both modifications of: (i) introduction of a disulfide bond; and (ii) amino acid modification in an N-terminal region; are at least added to the amino acid sequence of the parent CXCR3 ligands.

In a specific embodiment, the parent CXCR3 ligands have a C-X-C motif. In a specific embodiment, the parent CXCR3 ligands have C-T-C (Cys-Thr-Cys), C-L-C (Cys-Leu-Cys), or C-S-C (Cys-Ser-Cys) as the C-X-C motif.

In a specific embodiment, the amino acid sequence of the parent CXCR3 ligands comprises at least part of the amino acid sequence of naturally-occurring human CXCR3 ligands. In a specific embodiment, the amino acid sequence of the parent CXCR3 ligands comprises at least part of the amino acid sequence of naturally-occurring human CXCL10.

In a specific embodiment, the CXCR3 ligands provided in the present specification are variants of parent CXCR3 ligands comprising part of the amino acid sequence of naturally-occurring human CXCL10. The CXCR3 ligands provided in the present specification comprise part of the amino acid sequence of naturally-occurring human CXCL10.

In a specific embodiment, the CXCR3 ligands provided in the present specification are variants of naturally-occurring human CXCL10. The CXCR3 ligands provided in the present specification are CXCR3 ligands where modification is further added to parent CXCR3 ligands which are variants of naturally-occurring human CXCL10. For example, the CXCR3 ligands provided in the present specification are CXCR3 ligands where modification is further added to parent CXCR3 ligands which are variants produced by conferring resistance to furin protease to naturally-occurring human CXCL10. For example, the CXCR3 ligands provided in the present specification are CXCR3 ligands where an amino acid modification to enhance the activity to induce migration of cells expressing CXCR3 relative to parent CXCR3 ligands is further added to the amino acid sequence of the parent CXCR3 ligands which are variants produced by adding the R75A modification to naturally-occurring human CXCL10 in order to confer resistance to furin protease. In this case, the part of the amino acid sequence of naturally-occurring human CXCL10 is an amino acid sequence comprising the 75th amino acid from the N-terminus of the amino acid sequence of naturally-occurring human CXCL10.

In a specific embodiment, the CXCR3 ligands provided in the present specification are CXCR3 ligands having enhanced activity to induce migration of cells expressing CXCR3 relative to parent CXCR3 ligands comprising part of the amino acid sequence of naturally-occurring human CXCL10, where amino acids at positions corresponding to the 18th amino acid and the 60th amino acid in the amino acid sequence of the naturally-occurring human CXCL10 are substituted with Cys. In a specific embodiment, the CXCR3 ligands are CXCR3 ligands, further where an amino acid at a position corresponding to the first in the amino acid sequence of the naturally-occurring human CXCL10 is substituted with Tyr. Moreover, an amino acid at a position corresponding to the second amino acid in the amino acid sequence of the naturally-occurring human CXCL10 may be substituted with Val in the CXCR3 ligands.

In a specific embodiment, the CXCR3 ligands provided in the present specification are CXCR3 ligands having enhanced activity to induce migration of cells expressing CXCR3 relative to parent CXCR3 ligands comprising part of the amino acid sequence of naturally-occurring human CXCL10, where an amino acid at a position corresponding to the first in the amino acid sequence of the naturally-occurring human CXCL10 is substituted with Tyr. Moreover, an amino acid at a position corresponding to the second amino acid in the amino acid sequence of the naturally-occurring human CXCL10 may be substituted with Val in the CXCR3 ligands.

The CXCR3-expressing-cell migration-inducing activity of the CXCL10 variants provided in the present specification is higher than parent CXCR3 ligands. That is, the CXCR3-expressing-cell migration-inducing activity of the CXCL10 variants provided in the present specification is enhanced relative to parent CXCR3 ligands. For example, the CXCR3-expressing-cell migration-inducing activity of the CXCL10 variants provided in the present specification is 110% or more of the CXCR3-expressing-cell migration-inducing activity of parent CXCR3 ligands.

When verifying that the CXCR3 ligands of the present invention have enhanced activity to induce migration of cells expressing CXCR3 relative to parent CXCR3 ligands, it is specifically preferable to include the parent CXCR3 ligands as a control in the system for analyzing CXCR3-expressing-cell migration-inducing activity and compare the fluorescence intensities of CXCR3 ligands and the control, of which the activities are to be compared, that were obtained from the same experiment round, so that CXCR3 cells used for measurement are in the same condition. The CXCR3-expressing-cell migration-inducing activity of CXCR3 ligands can show 110% or more of the CXCR3-expressing-cell migration-inducing activity of the parent CXCR3 ligands in at least one of the concentrations from 3 nM, 10 nM, 30 nM, 100 nM, 300 nM, and 1000 nM, although the analyte concentration in the system for analyzing CXCR3-expressing-cell migration-inducing activity is not limited.

When fusion proteins comprising CXCR3 ligands are used as an analyte for analyzing CXCR3-expressing-cell migration-inducing activity, it is preferable that parent CXCR3 ligands used as a control are in a molecular format similar to the fusion proteins comprising CXCR3 ligands (*i.e.*, it is preferable to prepare a control fusion protein using the parent CXCR3 ligands instead of the CXCR3 ligands provided in the present specification).

In a specific embodiment, the CXCR3-expressing-cell migration-inducing activity of the CXCR3 ligands provided in the present specification is equal to or higher than naturally-occurring human CXCR3 ligands. In a specific embodiment, the CXCR3-expressing-cell migration-inducing activity of the CXCR3 ligands provided in the present specification is equal to or higher than naturally-occurring human CXCL10. Comparison with the naturally-occurring human CXCR3 ligands and the naturally-occurring human CXCL10 is performed as described above.

The enhancement of CXCR3-expressing-cell migration-inducing activity is verified, for example, by the increase of the number of cells migrating in the assay system or the decrease of the EC50 value. The enhancement of the CXCR3-expressing-cell migration-inducing activity of the CXCR3 ligands of the present invention is verified, for example, by a higher number of cells migrating in the assay system or a lower EC50 value compared to parent CXCR3 ligands.

A higher CXCR3-expressing-cell migration-inducing activity of the CXCR3 ligands of the present invention relative to the CXCR3-expressing-cell migration-inducing activity of parent CXCR3 ligands is verified, for example, by a higher number of cells migrating in the assay system in the CXCR3 ligands compared to the parent CXCR3 ligands or a lower EC50 value in the CXCR3 ligands compared to the parent CXCR3 ligands.

In a specific embodiment, the CXCR3 ligand concentration when the CXCR3-expressing-cell migration-inducing activity of the CXCR3 ligands provided in the present specification is at maximum is lower than the concentration of parent CXCR3 ligands when the CXCR3-expressing-cell migration-inducing activity of the proteins is at maximum.

An equal or a higher CXCR3-expressing-cell migration-inducing activity of the CXCR3 ligands of the present invention compared to the CXCR3-expressing-cell migration-inducing activity of naturally-occurring human CXCL10 is verified, for example, by an equal or a higher number of cells migrating in the assay system in the CXCR3 ligands relative to the naturally-occurring human CXCL10, or an equal or a lower EC50 value in the CXCR3 ligands relative to the naturally-occurring human CXCL10.

In a specific embodiment, the CXCR3 ligand concentration when the CXCR3-expressing-cell migration-inducing activity of the CXCR3 ligands provided in the present specification is at maximum is lower than the concentration of naturally-occurring human CXCL10 when the CXCR3-expressing-cell migration-inducing activity of the protein is at maximum.

The amino acid modification in an N-terminal region that enhances the activity to induce migration of cells expressing CXCR3 includes, for example, substitution of an amino acid residue in the N-terminal region with an amino acid residue that further stabilizes penetration of the cells expressing CXCR3 into the transmembrane regions and subsequent interaction. Candidates for the amino acid modification in an N-terminal region that increases the activity of CXCR3 ligands to induce migration of cells expressing CXCR3 relative to parent CXCR3 ligands include, for example, substitution of the first amino acid residue from the N-terminus of naturally-occurring human CXCL10 with Y, F, H, T, or M when the parent CXCR3 ligands are naturally-occurring human CXCL10 or CXCL10 variants in which modifications have been added to the amino acid sequence of naturally-occurring human CXCL10.

In a specific embodiment, the first amino acid from the N-terminus of the CXCR3 ligands and the polypeptides provided in the present specification is not V. In a specific embodiment, the second amino acid from the N-terminus of the CXCR3 ligands and the polypeptides provided in the present specification is not P.

In a specific embodiment, the first amino acid from the N-terminus of the CXCR3 ligands and the polypeptides provided in the present specification is Y. In a specific embodiment, the second amino acid from the N-terminus of the CXCR3 ligands and the polypeptides provided in the present specification is V. In a specific embodiment, the first V from the N-terminus of the sequence of parent CXCR3 ligands is substituted with Y in the CXCR3 ligands and the polypeptides provided in the present specification. In a specific embodiment, parent CXCR3 ligands comprise at least part of the sequence of naturally-occurring human CXCL10 (SEQ ID NO: 25) and at least the first V from the N-terminus of the sequence of the naturally-occurring human CXCL10 (SEQ ID NO: 25) or a human CXCL10 variant (SEQ ID NO: 1) is substituted with Y in the CXCR3 ligands and the polypeptides provided in the present specification. In this case, the at least part of the sequence of naturally-occurring human CXCL10 (SEQ ID NO: 25) is a sequence comprising the first and the second amino acids from the N-terminus of naturally-occurring human CXCL 10. In a preferred embodiment, CXCR3 ligands and polypeptides consisting of these amino acid sequences have an equal or a higher CXCR3-expressing-cell migration-inducing activity relative to the CXCR3-expressing-cell migration-inducing activity of naturally-occurring human CXCL10.

In a specific embodiment, at least the second P from the N-terminus of the sequence of naturally-occurring human CXCL10 (SEQ ID NO: 25) or a human CXCL10 variant (SEQ ID NO: 1) is substituted with V in the CXCR3 ligands and the polypeptides provided in the present specification.

In a specific embodiment, the CXCR3 ligands provided in the present specification comprise an amino acid substitution at an N-terminal side of the C-X-C motif of parent CXCR3 ligands. In a specific embodiment where the parent CXCR3 ligands comprise at least part of the amino acid sequence of naturally-occurring human CXCL10, the CXCR3 ligands provided in the present specification comprise an amino acid substitution at an amino acid position corresponding to between positions 1 and 8 in the amino acid sequence of the naturally-occurring human CXCL10. In a specific embodiment, the CXCR3 ligands provided in the present specification comprise substitution of an amino acid at position 1 in the amino acid sequence of the parent CXCR3 ligands.

In a specific embodiment, the CXCR3 ligands and the polypeptides provided in the present specification have the sequence of Y-P-L or Y-V-L at the N-terminus.

In a specific embodiment, the CXCR3 ligands and the polypeptides provided in the present specification have the sequence of Y-P-L-S-R-T (SEQ ID NO: 13) or Y-V-L-S-R-T (SEQ ID NO: 14) at the N-terminus.

The insertion site of a disulfide bond that enhances the activity to induce migration of cells expressing CXCR3 is extracted by predicting physical property values of variants using MD simulation, protein structure modeling, or such. The combinations of positions that are modified to cysteine residues are extracted as candidates of sites that increase the stability of the variants compared to parent CXCR3 ligands.

The positions modified to cysteine residues which are candidates of sites that increase the stability of the variants compared to the parent CXCR3 ligands are sites other than the b-sheet regions of the parent CXCR3 ligands. In a specific embodiment, a disulfide bond is introduced by substituting amino acids in the docking domain of the CXCR3 ligands with cysteines. In a specific embodiment, parent CXCR3 ligands have three b-strands and a disulfide bond is formed by substituting each of at least one amino acid contained in any two regions selected from: (i) a first loop between a C-X-C motif and a first b-strand located closest to an N-terminal side; (ii) a second loop between the first b-strand and a second b-strand located second closest to the N-terminal side; and (iii) a third loop between the second b-strand and a third b-strand located third closest to the N-terminal side; in the amino acid sequence of the parent CXCR3 ligands with Cys. For example, at least one amino acid contained in the first loop and at least one amino acid contained in the second loop are substituted with cysteines to introduce a disulfide bond between the first loop and the second loop. Alternatively, for example, at least one amino acid contained in the first loop and at least one amino acid contained in the third loop are substituted with cysteines to introduce a disulfide bond between the first loop and the third loop. Further alternatively, for example, at least one amino acid contained in the second loop and at least one amino acid contained in the third loop are substituted with cysteines to introduce a disulfide bond between the second loop and the third loop.

When the parent CXCR3 ligands are naturally-occurring human CXCL10 or CXCL10 variants in which modifications have been added to the amino acid sequence of naturally-occurring human CXCL10, for example, the positions modified to cysteine residues which are candidates of sites that increase the stability of the variants compared to the parent CXCR3 ligands include the 14th, the 18th, the 21st, the 25th, the 41st, the 46th, the 55th, the 56th, the 60th, and the 67th from the N-terminus of naturally-occurring human CXCL10. When the parent CXCR3 ligands are naturally-occurring human CXCL10 or CXCL10 variants in which modifications have been added to the amino acid sequence of naturally-occurring human CXCL10, for example, the combination of the positions modified to cysteine residues is any amino acid combination selected from the 14th and the 55th, the 18th and the 60th, the 21st and the 67th, the 25th and the 46th, and the 41st and the 56th from the N-terminus of naturally-occurring human CXCL10.

In a specific embodiment, at least one amino acid selected from the 14th, the 18th, the 21st, the 25th, the 41st, the 46th, the 55th, the 56th, the 60th, and the 67th from the N-terminus is C in the CXCR3 ligands provided in the present specification.

In a specific embodiment, any amino acid combination selected from the 14th and the 55th, the 18th and the 60th, the 21st and the 67th, the 25th and the 46th, and the 41st and the 56th from the N-terminus is C in the CXCR3 ligands provided in the present specification. That is, the amino acid sequences where a combination selected from the above combinations is substituted with C are preferable as the CXCR3 ligands provided in the present specification. In a certain embodiment, substitution to be introduced is at least one pair and can be arbitrarily selected from one to five pairs. In a preferred embodiment, CXCR3 ligands and polypeptides consisting of these amino acid sequences have an equal or a higher CXCR3-expressing-cell migration-inducing activity relative to the CXCR3-expressing-cell migration-inducing activity of naturally-occurring human CXCL10.

In a specific embodiment, amino acids in any amino acid combination selected from the 14th and the 55th, the 18th and the 60th, the 21st and the 67th, the 25th and the 46th, and the 41st and the 56th from the N-terminus of the sequence of naturally-occurring human CXCL10 (SEQ ID NO: 25) or a human CXCL10 variant (SEQ ID NO: 1) are substituted with C in the CXCR3 ligands provided in the present specification.

In a specific embodiment, the 18th amino acid from the N-terminus of the CXCR3 ligands and the polypeptides provided in the present specification is C. In a specific embodiment, the 60th amino acid from the N-terminus of the CXCR3 ligands provided in the present specification is C. In these cases, the at least part of the sequence of naturally-occurring human CXCL10 (SEQ ID NO: 25) is a sequence comprising the 18th and the 60th amino acids from the N-terminus of naturally-occurring human CXCL10. In a preferred embodiment, CXCR3 ligands and polypeptides consisting of these amino acid sequences have an equal or a higher CXCR3-expressing-cell migration-inducing activity relative to the CXCR3-expressing-cell migration-inducing activity of the naturally-occurring human CXCL10.

In a specific embodiment, the 18th amino acid from the N-terminus of the CXCR3 ligands and the polypeptides provided in the present specification is not P. In a specific embodiment, the 60th amino acid from the N-terminus of the CXCR3 ligands provided in the present specification is not A.

In a specific embodiment, at least the 18th P from the N-terminus is substituted with C and the 60th A from the N-terminus is substituted with C in the sequence of naturally-occurring human CXCL10 (SEQ ID NO: 25) or a human CXCL10 variant (SEQ ID NO: 1) in the CXCR3 ligands and the polypeptides provided in the present specification.

In a specific embodiment, the 18th P from the N-terminus of the sequence of parent CXCR3 ligands is substituted with V in the CXCR3 ligands and the polypeptides provided in the present specification. In a specific embodiment, parent CXCR3 ligands comprise at least part of the sequence of naturally-occurring human CXCL10 (SEQ ID NO: 25) and at least each of the 18th P and the 60th A from the N-terminus of the sequence of naturally-occurring human CXCL10 (SEQ ID NO: 25) or a human CXCL10 variant (SEQ ID NO: 1) is substituted with C in the CXCR3 ligands and the polypeptides provided in the present specification. In this case, the amino acid sequence comprising at least part of the sequence of naturally-occurring human CXCL10 (SEQ ID NO: 25) can be an amino acid sequence comprising the 18th and the 60th amino acids from the N-terminus. In a preferred embodiment, CXCR3 ligands and polypeptides consisting of these amino acid sequences have an equal or a higher CXCR3-expressing-cell migration-inducing activity compared to the CXCR3-expressing-cell migration-inducing activity of the naturally-occurring human CXCL10.

In a specific embodiment, the CXCR3 ligands provided in the present specification are polypeptides having the following amino acid sequence: (X1)(X2)LSRTVRCT CISISNQ(X3)VN PRSLEKLEII PASQFCPRVE IIATMKKKG EKRCLNPESK(X4) IKNLLKAVSK ERSK(X5)SP (SEQ ID NO: 53), wherein (X1) is V or Y, (X2) is P or V, (X3) and (X4) are C, and (X5) is R or A.

In a specific embodiment, the CXCR3 ligands provided in the present specification are polypeptides having the following amino acid sequence: (X1)(X2)LSRTVRCT CISISNQ(X3)VN PRSLEKLEII PASQFCPRVE IIATMKKKG EKRCLNPESK(X4) IKNLLKAVSK ERSK(X5)SP (SEQ ID NO: 54), wherein (X1) is Y, (X2) is P or V, (X3) is P or C, (X4) is A or C, and (X5) is R or A.

In a particular embodiment, the CXCR3 ligand provided herein has a C-X-C motif. The two cysteines contained in the C-X-C motif can each form disulfide bonds with cysteines other than those contained in the C-X-C motif comprised in the CXCR3 ligand. The C-X-C motif in the CXCR3 ligand can be selected from C-T-C (Cys-Thr-Cys), C-L-C (Cys-Leu-Cys), and C-S-C (Cys-Ser-Cys).

In a particular embodiment, the CXCR3 ligand provided herein is any of a CXCL10 variant, a CXCL11 variant, a CXCL9 variant, an hITIP variant, and a chimeric protein prepared from those variants.

In a particular embodiment, the CXCR3 ligand and polypeptide provided herein has any of the following sequences (b1) to (b7):

(b1) a sequence shown by any one of SEQ ID NOs: 4 to 12;
(b2) a sequence showing 90% or more sequence identity to SEQ ID NO: 1;
(b3) a sequence showing 90% or more sequence identity to SEQ ID NO: 25;
(b4) a sequence showing 90% or more sequence identity to SEQ ID NO: 26;
(b5) a sequence showing 90% or more sequence identity to SEQ ID NO: 27;
(b6) a sequence showing 90% or more sequence identity to SEQ ID NO: 28; and
(b7) a sequence comprising 10 or less amino acid substitutions, insertions, or deletions to a sequence selected from SEQ ID NOs: 1, 4 to 12, and 25 to 28.

In a preferred embodiment, these CXCR3 ligands and polypeptides have an equal or a higher CXCR3-expressing-cell migration-inducing activity compared to the CXCR3-expressing-cell migration-inducing activity of the naturally-occurring human CXCL10.

In a particular embodiment, the CXCR3 ligand and polypeptide provided herein have a sequence showing 90% or more sequence identity, 95% or more sequence identity, 96% or more sequence identity, 97% or more sequence identity, 98% or more sequence identity, or 99% or more sequence identity to any one of the sequences of SEQ ID NOs: 1, 4 to 12, and 25 to 28.

In one embodiment, the CXCR3 ligands provided in the present specification are CXCR3 ligands in which the amino acid corresponding to amino acid position 7, when taking the N-terminal amino acid in the amino acid sequence of the parent CXCR3 ligands as position 1, is substituted with Pro and having improved stability in blood relative to the parent CXCR3 ligands. In a specific embodiment, the amino acid sequence of the parent CXCR3 ligands comprises at least part of the amino acid sequence of naturally-occurring human CXCR3 ligands. In a specific embodiment, the amino acid sequence of the parent CXCR3 ligands comprises at least part of the amino acid sequence of naturally-occurring human CXCL10. In a specific embodiment, the CXCR3 ligands provided in the present specification are variants of parent CXCR3 ligands comprising part of the amino acid sequence of naturally-occurring human CXCL10. The CXCR3 ligands provided in the present specification comprise part of the amino acid sequence of naturally-occurring human CXCL10. In a specific embodiment, the CXCR3 ligands provided in the present specification are variants of naturally-occurring human CXCL10. The CXCR3 ligands provided in the present specification are CXCR3 ligands where modification is further added to parent CXCR3 ligands which are variants of naturally-occurring human CXCL10.

For example, the CXCR3 ligands having improved stability in blood relative to a parent CXCR3 ligand, provided in the present specification, are variants to which further modifications have been added to parent CXCR3 ligands which are variants produced by adding amino acid modifications for enhancing the CXCR3-expressing-cell migration-inducing activity to naturally-occurring human CXCL10. For example, the CXCR3 ligands having improved stability in blood relative to parent CXCR3 ligands, provided in the present specification, are variants to which further amino acid modifications for improving stability in blood relative to the parent CXCR3 ligands have been added to the amino acid sequences of the parent CXCR3 ligands which are variants to which either or both modifications of: (i) introduction of a disulfide bond; and (ii) amino acid modification in an N-terminal region; which are amino acid modifications for enhancing CXCR3-expressing-cell migration-inducing activity, are at least added to naturally-occurring human CXCR10.

For example, the CXCR3 ligands having improved stability in blood relative to parent CXCR3 ligands, provided in the present specification, are CXCR3 ligands to which further modifications have been added to parent CXCR3 ligands which are variants produced by conferring resistance to DPPIV cleavage to naturally-occurring human CXCL10. For example, the CXCR3 ligands having improved stability in blood relative to parent CXCR3 ligands, provided in the present specification, are CXCR3 ligands to which further amino acid modifications for improving stability in blood relative to the parent CXCR3 ligands have been added to the amino acid sequence of the parent CXCR3 ligands which are variants produced by adding the P2V modification to naturally-occurring human CXCL10 to confer resistance to DPPIV cleavage.

For example, the CXCR3 ligands having improved stability in blood relative to parent CXCR3 ligands, provided in the present specification, are CXCR3 ligands to which further modifications have been added to parent CXCR3 ligands which are variants produced by conferring resistance to furin protease to naturally-occurring human CXCL10. For example, the CXCR3 ligands having improved stability in blood relative to parent CXCR3 ligands, provided in the present specification, are CXCR3 ligands to which further amino acid modifications for improving stability in blood relative to the parent ligands have been added to the amino acid sequences of the parent CXCR3 ligands which are variants produced by adding the R75A modification to naturally-occurring human CXCL10 to confer resistance to furin protease.

In a specific embodiment, the CXCR3 ligands of the present disclosure are CXC3 ligands which contain one or more of the following modifications: amino acid modifications for improving stability in blood relative to parent CXCR3 ligands; amino acid modifications for enhancing CXCR3-expressing cell migration-inducing activity; amino acid modifications for conferring resistance to DPPIV cleavage; and amino acid modifications for conferring furin protease resistance.

In a specific embodiment, the CXCR3 ligands provided in the present specification are CXCR3 ligands and are polypeptides having the sequence of Y-P-L-S-R-T-P (SEQ ID NO: 67), Y-V-L-S-R-T-P (SEQ ID NO: 68), V-P-L-S-R-T-P (SEQ ID NO: 70), or V-V-L-S-R-T-P (SEQ ID NO: 71) at the N-terminus.

In a specific embodiment, the CXCR3 ligands provided in the present specification are polypeptides having the following amino acid sequence: (X1)(X2)LSRT(X6)RCT CISISNQ(X3)VN PRSLEKLEII PASQFCPRVE IIATMKKKG EKRCLNPESK(X4) IKNLLKAVSK ERSK(X5)SP (SEQ ID NO: 69),

wherein (X1) is V or Y, (X2) is P or V, (X3) is P or C, (X4) is A or C, (X5) is R or A, and (X6) is P.

CXCR3 ligand mutants

In certain embodiments, amino acid sequence mutants of the CXCR3 ligands provided herein are contemplated. Amino acid sequence mutants of a CXCR3 ligand may be prepared by introducing appropriate modifications into the nucleotide sequence encoding the CXCR3 ligand, or by peptide synthesis. Such modifications include, for example, deletions from, and/or insertions into and/or substitutions of residues within the amino acid sequences of the CXCR3 ligand. Any combination of deletion, insertion, and substitution can be made to arrive at the final construct, provided that the final construct possesses the desired characteristics (e.g., activity to cause migration of CXCR3-expressing-cells).

Substitution, insertion, and deletion mutants

In certain embodiments, CXCR3 ligand mutants having one or more amino acid substitutions are provided. Conservative substitutions are shown in Table 1 under the heading of "preferred substitutions." More substantial changes are provided in Table 1 under the heading of "exemplary substitutions," and as further described below in reference to amino acid side chain classes. Amino acid substitutions may be introduced into a CXCR3 ligand of interest and the products may be screened for a desired activity, e.g., retained/improved activity to cause migration of CXCR3-expressing-cells.

[Table 1]

| Original residue | Exemplary substitutions | Preferred substitutions |
|---|---|---|
| Ala (A) | Val; Leu; Ile | Val |
| Arg (R) | Lys; Gln; Asn | Lys |
| Asn (N) | Gln; His; Asp, Lys; Arg | Gln |
| Asp (D) | Glu; Asn | Glu |
| Cys (C) | Ser; Ala | Ser |
| Gln (Q) | Asn; Glu | Asn |
| Glu (E) | Asp: Gln | Asp |
| Gly (G) | Ala | Ala |
| His (H) | Asn; Gln; Lys; Arg | Arg |
| Ile (I) | Leu; Val; Met; Ala; Phe; Norleucine | Leu |
| Leu (L) | Norleucine ; Ile; Val; Met: Ala; Phe | Ile |
| Lys (K) | Arg; Gln; Asn | Arg |
| Met (M) | Leu; Phe; Ile | Leu |
| Phe (F) | Trp; Leu; Val; Ile; Ala; Tyr | Tyr |
| Pro (P) | Ala | Ala |
| Ser (S) | Thr | Thr |
| Thr (T) | Val; Ser | Ser |
| Trp (W) | Tyr; Phe | Tyr |
| Tyr (Y) | Trp; Phe; Thr; Ser | Phe |
| Val (V) | Ile; Leu; Met; Phe; Ala; Norleucine | Leu |

Amino acids may be grouped according to common side-chain properties:

(1) hydrophobic: Norleucine, Met, Ala, Val, Leu, Ile;
(2) neutral hydrophilic: Cys, Ser, Thr, Asn, Gln;
(3) acidic: Asp, Glu;
(4) basic: His, Lys, Arg;
(5) residues that influence chain orientation: Gly, Pro;
(6) aromatic: Trp, Tyr, Phe.

Non-conservative substitutions will entail exchanging a member of one of these classes for another class.
In a particular embodiment, one or more modifications may be carried out regarding the substitutions, insertions, or deletions as long as such modifications do not substantially reduce the abilities of the CXCR3 ligand. In a particular embodiment, the CXCR ligand mutant described above comprises one, two, three, four, five, six, seven, eight, nine, or ten amino acid modifications.
When modification is added to CXCR3 ligands, an amino acid position identified in the present disclosure is preferably adjusted by aligning an amino acid sequence before the modification and an amino acid sequence after the modification to identify a homologous amino acid residue. For example, positions corresponding to the 18th and the 60th from the N-terminus of naturally-occurring human CXCL 10 mean positions of amino acid positions of variants assuming that there has been no change or shift of an amino acid position by modification other than substitution of the 18th and the 60th amino acids. When an amino acid position value is changed into an amino acid position larger than the 18th by insertion of an amino acid(s), for example, the amino acid position is counted after decreasing the number of inserted amino acid(s). When an amino acid position value is changed into an amino acid position smaller than the 18th or the 60th by deletion of an amino acid(s), for example, the amino acid position is counted after increasing the number of deleted amino acid(s).

When modification is added to CXCR3 ligands, an amino acid position identified in the present disclosure can be identified in the amino acid sequence of variants.

Amino acid sequence identity

"Percent (%) amino acid sequence identity" with respect to a reference polypeptide sequence is defined as the percentage of amino acid residues in a candidate sequence that are identical with the amino acid residues in the reference polypeptide sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN, Megalign (DNASTAR) software, or GENETYX (registered trademark) (Genetyx Co., Ltd.). Those skilled in the art can determine appropriate parameters for aligning sequences, including any algorithms needed to achieve maximal alignment over the full length of the sequences being compared.

The ALIGN-2 sequence comparison computer program was authored by Genentech, Inc., and the source code has been filed with user documentation in the U.S. Copyright Office, Washington D.C., 20559, where it is registered under U.S. Copyright Registration No. TXU510087. The ALIGN-2 program is publicly available from Genentech, Inc., South San Francisco, California, or may be compiled from the source code. The ALIGN-2 program should be compiled for use on a UNIX operating system, including digital UNIX V4.0D. All sequence comparison parameters are set by the ALIGN-2 program and do not vary.

In situations where ALIGN-2 is employed for amino acid sequence comparisons, the % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) is calculated as follows:

$$100 \text{ times the fraction } X/Y$$

where X is the number of amino acid residues scored as identical matches by the sequence alignment program ALIGN-**2 in that program's alignment of A and B, and where Y is the total** number of amino acid residues in B. It will be appreciated that where the length of amino acid sequence A is not equal to the length of amino acid sequence B, the % amino acid sequence identity of A to B will not equal the % amino acid sequence identity of B to A. Unless specifically stated otherwise, all % amino acid sequence identity values used herein are obtained as described in the immediately preceding paragraph using the ALIGN-2 computer program.

Methods of enhancing the activity of CXCR3 ligands to induce migration of cells expressing CXCR3

The present disclosure is also related to methods of enhancing the activity of CXCR3 ligands to induce migration of cells expressing CXCR3. The methods of enhancing the activity of CXCR3 ligands to induce migration of cells expressing CXCR3 provided in the present specification comprise modifying the sequence of parent CXCR3 ligands. In a specific embodiment, the methods of enhancing the activity of CXCR3 ligands to induce migration of cells expressing CXCR3 provided in the present specification comprise modifying an amino acid in an N-terminal region of the sequence of parent CXCR3 ligands. In a specific embodiment, the methods of enhancing the activity of CXCR3 ligands to induce migration of cells expressing CXCR3 provided in the present specification comprise introducing a disulfide bond into the sequence of parent CXCR3 ligands.

In a specific embodiment, parent CXCR3 ligands used in the methods of enhancing the activity of CXCR3 ligands to induce migration of cells expressing CXCR3 provided in the present specification have a C-X-C motif. In a specific embodiment, the C-X-C motif is C-T-C (Cys-Thr-Cys), C-L-C (Cys-Leu-Cys), or C-S-C (Cys-Ser-Cys).

In a specific embodiment, parent CXCR3 ligands used in the methods of enhancing the activity of CXCR3 ligands to induce migration of cells expressing CXCR3 provided in the present specification comprise part of the amino acid sequence of naturally-occurring human CXCR3 ligands. In a specific embodiment, the naturally-occurring human CXCR3 ligands are naturally-occurring human CXCL10.

In a specific embodiment, the first amino acid from the N-terminus in parent CXCR3 ligands used in the methods of enhancing the activity of CXCR3 ligands to induce migration of cells expressing CXCR3 provided in the present specification is V. In a specific embodiment, the second amino acid from the N-terminus in parent CXCR3 ligands used in the methods of enhancing the activity of CXCR3 ligands to induce migration of cells expressing CXCR3 provided in the present specification is P. In a more specific embodiment, the N-terminal sequence of parent CXCR3 ligands used in the methods of enhancing the activity of CXCR3 ligands to induce migration of cells expressing CXCR3 provided in the present specification is V-P-L or V-V-L. In a further specific embodiment, the N-terminal sequence of parent CXCR3 ligands used in the methods of enhancing CXCR3-expressing-cell migration-inducing activity of CXCR3 ligands provided in the present specification is V-P-L-S-R-T (SEQ ID NO: 19) or V-V-L-S-R-T (SEQ ID NO: 20).

In a specific embodiment, parent CXCR3 ligands used in the methods of enhancing the activity of CXCR3 ligands to induce migration of cells expressing CXCR3 provided in the present specification have a C-X-C motif. The two cysteines contained in the C-X-C motif can independently form a disulfide bond with a cysteine other than those in the C-X-C motif comprised in the parent CXCR3 ligands. The C-X-C motif in the parent CXCR3 ligands can be selected from C-T-C (Cys-Thr-Cys), C-L-C (Cys-Leu-Cys), and C-S-C (Cys-Ser-Cys). In a specific embodiment, the C-X-C motif in the parent CXCR3 ligands can be located adjacent to the C-terminus of N-terminal sequence V-P-L-S-R-T-V-R (SEQ ID NO: 21) or V-V-L-S-R-T-V-R (SEQ ID NO: 22).

In a specific embodiment, parent CXCR3 ligands used in the methods of enhancing the activity of CXCR3 ligands to induce migration of cells expressing CXCR3 provided in the present specification can also have any one of (b 1) to (b6) at the C-terminus of the C-X-C motif:

(b 1) the sequence from the 12th amino acid to the 77th amino acid of any one of SEQ ID NOs: 4-12;
(b2) the sequence from the 12th amino acid to the 77th amino acid of SEQ ID NO: 25;
(b3) the sequence from the 12th amino acid to the 73rd amino acid of SEQ ID NO: 26;
(b4) the sequence from the 12th amino acid to the 103rd amino acid of SEQ ID NO: 27;
(b5) the sequence from the 12th amino acid to the 77th amino acid of SEQ ID NO: 1; and
(b6) the sequence from the 12th amino acid to the 77th amino acid of SEQ ID NO: 28.

In a specific embodiment, parent CXCR3 ligands used in the methods of enhancing the activity of CXCR3 ligands to induce migration of cells expressing CXCR3 provided in the present specification are selected from naturally-occurring CXCL10, naturally-occurring CXCL11, naturally-occurring CXCL9, CXCL10 variants, CXCL11 variants, CXCL9 variants, and chimeric proteins produced therefrom.

In a specific embodiment, parent CXCR3 ligands used in the methods of enhancing the activity of CXCR3 ligands to induce migration of cells expressing CXCR3- provided in the present specification are resistant to furin protease. In a specific embodiment, parent CXCR3 ligands used in the methods of enhancing the activity of CXCR3 ligands to induce migration of cells expressing CXCR3 provided in the present specification are CXCL10 variants where the modification of R75A is added to the amino acid sequence of naturally-occurring human CXCL10.

In a specific embodiment, the methods of enhancing the activity of CXCR3 ligands to induce migration of cells expressing CXCR3 provided in the present specification comprise adding at least either or both modifications of: (i) introduction of a disulfide bond; and (ii) amino acid modification in an N-terminal region; to the amino acid sequence of parent CXCR3 ligands. In a specific embodiment, the methods of enhancing the activity of CXCR3 ligands to induce migration of cells expressing CXCR3 provided in the present specification comprise introducing at least one amino acid substitution of V1Y, or P18C and A60C to the amino acid sequence of the parent CXCR3 ligands.

In other words, the present specification provides use of either:

(1) substitution of the first amino acid in the parent CXCR3 ligands from V to Y; or
(2) substitution of the 18th amino acid from the N-terminus in the parent CXCR3 ligands from P to C and substitution of the 60th amino acid from A to C;

to enhance the activity of CXCR3 ligands to induce migration of cells expressing CXCR3.

In addition to the above, the methods of enhancing the activity of CXCR3 ligands to induce migration of cells expressing CXCR3 provided in the present specification can further comprise substituting the second amino acid from the N-terminus in the parent CXCR3 ligands from P to V.

Methods of improving stability in blood of CXCR3 ligands

The present disclosure is also related to methods of improving the stability in blood of CXCR3 ligands. The methods of improving the stability in blood of CXCR3 ligands provided in the present specification comprise modifying the sequence of parent CXCR3 ligands. In a specific embodiment, the methods of improving the stability in blood of CXCR3 ligands provided in the present specification comprise substituting the amino acid corresponding to amino acid position 7, when taking the N-terminal amino acid in the amino acid sequence of parent CXCR3 ligands as position 1, with Pro. In a specific embodiment, the 7th amino acid from the N-terminus of parent CXCR3 ligands used in the methods of improving the stability in blood of CXCR3 ligands provided in the present specification is V. In a specific embodiment, parent CXCR3 ligands used in the methods of improving the stability in blood of CXCR3 ligands provided in the present specification comprise part of the amino acid sequence of naturally-occurring human CXCR3 ligands. In a specific embodiment, the naturally-occurring human CXCR3 ligands are naturally-occurring human CXCL10.

In a specific embodiment, parent CXCR3 ligands used in the methods of improving the stability in blood of CXCR3 ligands provided in the present specification are selected from naturally-occurring CXCL10, naturally-occurring CXCL11, naturally-occurring CXCL9, CXCL10 variants, CXCL 11 variants, CXCL9 variants, and chimeric proteins produced therefrom.

In a specific embodiment, parent CXCR3 ligands used in the methods of improving the stability in blood of CXCR3 ligands provided in the present specification are resistant to furin protease. In a specific embodiment, parent CXCR3 ligands used in the methods of improving the stability in blood of CXCR3 ligands provided in the present specification are CXCL10 variants where the modification of R75A is added to the amino acid sequence of naturally-occurring human CXCL10.

In a specific embodiment, the methods of improving the stability in blood of CXCR3 ligands provided in the present specification further comprise adding at least either or both modifications of: (i) introduction of a disulfide bond; and (ii) amino acid modification in an N-terminal region; to the amino acid sequence of parent CXCR3 ligands. In a specific embodiment, the methods of improving the stability in blood of CXCR3 ligands provided in the present specification comprise introducing at least one amino acid substitution of V1Y, or P18C and A60C to the amino acid sequence of parent CXCR3 ligands.

In addition to the above, the methods of improving the stability in blood of CXCR3 ligands provided in the present specification can further comprise substituting the second amino acid from the N-terminus in the parent CXCR3 ligands from P to V.

Methods of producing CXCR3 ligands having enhanced stability in blood

The present disclosure is also related to methods of producing CXCR3 ligands having enhanced stability in blood relative to parent ligands. Methods of producing CXCR3 ligands having enhanced stability in blood relative to parent ligands provided herein in the present specification comprise modifying the sequence of parent CXCR3 ligands. In a specific embodiment, the methods of producing CXCR3 ligands having enhanced stability in blood relative to parent ligands provided in the present specification comprise substituting the amino acid corresponding to amino acid position 7, when taking the N-terminal amino acid in the amino acid sequence of the parent CXCR3 ligands as position 1, with Pro. In a specific embodiment, the 7th amino acid from the N-terminus of the parent CXCR3 ligands used in the methods of producing CXCR3 ligands having enhanced stability in blood relative to parent ligands provided in the present specification is V. In a specific embodiment, the parent CXCR3 ligands used in the methods of producing CXCR3 ligands having enhanced stability in blood relative to parent ligands provided in the present specification, comprise part of the amino acid sequence of naturally-occurring human CXCR3 ligands. In a specific embodiment, the naturally-occurring human CXCR3 ligands are naturally-occurring human CXCL10.

In a specific embodiment, the parent CXCR3 ligands used in the methods of producing CXCR3 ligands having enhanced stability in blood relative to parent ligands provided in the present specification are selected from naturally-occurring CXCL10, naturally-occurring CXCL11, naturally-occurring CXCL9, CXCL10 variants, CXCL11 variants, CXCL9 variants, and chimeric proteins produced therefrom.

In a specific embodiment, the parent CXCR3 ligands used in the methods of producing CXCR3 ligands having enhanced stability in blood relative to parent ligands provided in the present specification are resistant to furin protease. In a specific embodiment, the parent CXCR3 ligands used in the methods of producing CXCR3 ligands having enhanced stability in blood relative to parent ligands provided in the present specification are CXCL10 variants where the modification of R75A is added to the amino acid sequence of naturally-occurring human CXCL10.

In a specific embodiment, the methods of producing CXCR3 ligands having enhanced stability in blood relative to parent ligands provided in the present specification further comprise adding at least either or both modifications of: (i) introduction of a disulfide bond; and (ii) amino acid modification in an N-terminal region; to the amino acid sequence of the parent CXCR3 ligands. In a specific embodiment, the methods of producing CXCR3 ligands having enhanced stability in blood relative to parent ligands provided in the present specification comprise introducing at least one amino acid substitution of V1Y, or P18C and A60C to the amino acid sequence of the parent CXCR3 ligands.

In addition to the above, the methods of producing CXCR3 ligands having enhanced stability in blood relative to parent ligands provided in the present specification can further comprise substituting the second amino acid from the N-terminus in the parent CXCR3 ligands from P to V.

Fusion proteins comprising a CXCR3 ligand

One aspect of the disclosure relates to fusion proteins comprising a CXCR3 ligand. In a specific embodiment, the fusion proteins of the present disclosure relate to fusion proteins comprising a CXCR3 ligand at the N-terminus. The fusion proteins of the present disclosure may be fusion proteins in which a CXCR3 ligand and an antibody are fused, or may be fusion proteins in which a CXCR3 ligand and an antibody Fc region, another type of protein such as albumin, or a polypeptide are fused. Herein, a molecule that binds to a CXCR3 ligand may be called a ligand-binding molecule. A specific example includes a fusion protein in which an antibody (including an intact antibody, a full-length antibody, and antibody fragments), an antibody Fc region, another type of protein such as albumin, or a polypeptide is fused to the C-terminus of a CXCR3 ligand. A fusion protein comprising a CXCR3 ligand can be purified using a substance that binds to the fusion protein. For example, when fused with an antibody Fc region, adsorption onto immobilized protein A can be used to recover the fusion proteins comprising the CXCR3 ligand.
The ligand-binding molecules included in the fusion proteins of the present disclosure may be polypeptides having at least one cleavage site. Specifically, the fusion proteins of the present disclosure may be a fusion protein in which an antibody (including an intact antibody, a full-length antibody, and antibody fragments), an antibody Fc region, another type of protein such as albumin, or a polypeptide having at least one cleavage site is fused to the C-terminus of a CXCR3 ligand.
Ligand-binding molecules containing cleavage sites are described, for example, in WO 2018/097308 and WO 2019/107384. The cleavage sites may be located at any position in the polypeptides as long as they can reduce the binding of the ligand-binding molecules to the ligands following cleavage. Furthermore, one or a plurality of cleavage sites may be comprised in the polypeptides.
In one example, the ligand-binding molecule is an intact antibody or an antibody fragment having at least one cleavage site, and in a state where the intact antibody or antibody fragment is cleaved at at least one cleavage site, the binding with the CXCR3 ligand or the polypeptide having a sequence disclosed herein is reduced. For example, when in a state where the cleavage site is cleaved, the CXCR3 ligand or the polypeptide having a sequence disclosed herein separates from the intact antibody or the antibody fragment.
Cleavage sites include, for example, protease cleavage sequences. Proteases are, for example, target tissue-specific proteases, and examples are cancer tissue-specific proteases or inflamed tissue-specific proteases. Examples of proteases include metalloproteases, serine proteases, aspartic proteases, cysteine proteases, and threonine proteases, and an example may be matriptase or urokinase (uPA), but the proteases are not limited thereto.
Furthermore, flexible linkers may be added to one end or both ends of a cleavage site or protease cleavage sequence.
As an example, an intact antibody or antibody fragment has an antibody constant region, antibody VH, and antibody VL, and the cleavage site or the protease cleavage sequence is located at at least one of the following positions: near the boundary between the antibody constant region and the antibody VH; and near the boundary between the antibody constant region and the antibody VL. The cleavage site or the protease cleavage sequence may be inserted into at least one of the following: (i) an arbitrary position in the sequence from the 109th amino acid (Kabat numbering) of the antibody VH to the 122nd amino acid (EU numbering) of the antibody heavy chain constant region; and (ii) an arbitrary position in the sequence from the 104th amino acid (Kabat numbering) of the antibody VL to the 113rd amino acid (EU numbering) of the antibody light chain constant region. For example, the antibody VL and antibody VH of the intact antibodies or antibody fragments are associated, and the association is cancelled by cleavage of the cleavage site or cancelled by cleavage of the protease cleavage sequence by a protease.
Furthermore, the CXCR3 ligand or the polypeptide having a sequence disclosed herein may be fused with an antibody Fc region via a linker, or the CXCR3 ligand or the polypeptide having a sequence disclosed herein may be fused with an intact antibody or an antibody fragment via a linker.
In the fusion proteins of the present disclosure, the CXCR3 ligand and the fusion partner can be fused via a linker. For example, an arbitrary peptide linker that can be introduced by genetic engineering, or a synthetic compound linker (e.g., a linker disclosed in Protein Engineering, 9 (3), 299-305, 1996) can be used as the linker used in the fusion of the CXCR3 ligand with the fusion partner.
The length of the peptide linker is not particularly limited and may be appropriately selected by those skilled in the art according to the purpose. Examples of the peptide linker can include, but are not limited to:

Ser
Gly·Ser (GS)
Ser·Gly (SG)

Gly·Gly·Ser (GGS)
Gly·Ser·Gly (GSG)
Ser·Gly·Gly (SGG)
Gly·Ser·Ser (GSS)
Ser·Ser·Gly (SSG)
Ser·Gly·Ser (SGS)
Gly·Gly·Gly·Ser (GGGS, SEQ ID NO: 29)
Gly·Gly·Ser·Gly (GGSG, SEQ ID NO: 30)
Gly·Ser·Gly·Gly (GSGG, SEQ ID NO: 31)
Ser·Gly·Gly·Gly (SGGG, SEQ ID NO: 32)
Gly·Ser·Ser·Gly (GSSG, SEQ ID NO: 33)
Gly·Gly·Gly·Gly·Ser (GGGGS, SEQ ID NO: 34)
Gly·Gly·Gly·Ser·Gly (GGGSG, SEQ ID NO: 35)
Gly·Gly·Ser·Gly·Gly (GGSGG, SEQ ID NO: 36)
Gly·Ser·Gly·Gly·Gly (GSGGG, SEQ ID NO: 37)
Gly·Ser·Gly·Gly·Ser (GSGGS, SEQ ID NO: 38)
Ser·Gly·Gly·Gly·Gly (SGGGG, SEQ ID NO: 39)
Gly·Ser·Ser·Gly·Gly (GSSGG, SEQ ID NO: 40)
Gly·Ser·Gly·Ser·Gly (GSGSG, SEQ ID NO: 41)
Ser·Gly·Gly·Ser·Gly (SGGSG, SEQ ID NO: 42)
Gly·Ser·Ser·Ser·Gly (GSSSG, SEQ ID NO: 43)
Gly·Gly·Gly·Gly·Gly·Ser (GGGGGS, SEQ ID NO: 44)
Ser·Gly·Gly·Gly·Gly·Gly (SGGGGG, SEQ ID NO: 45)
Gly·Gly·Gly·Gly·Gly·Gly·Ser (GGGGGGS, SEQ ID NO: 46)
Ser·Gly·Gly·Gly·Gly·Gly·Gly (SGGGGGG, SEQ ID NO: 47)
(Gly·Gly·Gly·Gly·Ser (GGGGS, SEQ ID NO: 34))n
(Ser·Gly·Gly·Gly·Gly (SGGGG, SEQ ID NO: 39))n

wherein n is an integer of 1 or larger. However, the length and sequence of the peptide linker can be appropriately selected by those skilled in the art according to the purpose.

The synthetic compound linker (chemical cross-linking agent) is a cross-linking agent usually used in peptide cross-linking, for example, N-hydroxysuccinimide (NHS), disuccinimidyl suberate (DSS), bis(sulfosuccinimidyl) suberate (BS3), dithiobis(succinimidyl propionate) (DSP), dithiobis(sulfosuccinimidyl propionate) (DTSSP), ethylene glycol bis(succinimidyl succinate) (EGS), ethylene glycol bis(sulfosuccinimidyl succinate) (sulfo-EGS), disuccinimidyl tartrate (DST), disulfosuccinimidyl tartrate (sulfo-DST), bis[2-(succinimidoxycarbonyloxy)ethyl]sulfone (BSOCOES), or bis[2-(sulfosuccinimidoxycarbonyloxy)ethyl]sulfone (sulfo-BSOCOES).

These cross-linking agents are commercially available.

Antibodies

The term "antibody" herein is used in the broadest sense and encompasses various antibody structures, including but not limited to monoclonal antibodies, polyclonal antibodies, multispecific antibodies (e.g., bispecific antibodies), and antibody fragments so long as they exhibit the desired antigen-binding activity.

Antibody fragments

An "antibody fragment" refers to a molecule other than an intact antibody that comprises a portion of an intact antibody that binds the antigen to which the intact antibody **binds. Examples of antibody fragments include but are not limited to Fv, Fab, Fab', Fab**'-SH, F(ab')$_2$; diabodies; linear antibodies; single-chain antibody molecules (e.g. scFv); and multispecific antibodies formed from antibody fragments.

**The terms "full length antibody," "intact antibody," and "whole antibody" are used** herein interchangeably to refer to an antibody having a structure substantially similar to a native antibody structure or having heavy chains that contain an Fc region as defined herein.

The term **"variable region" or "variable domain" refers to the domain** of an antibody heavy or light chain that is involved in binding the antibody to antigen. The variable domains of the heavy chain and light chain (VH and VL, respectively) of an antibody generally have similar structures, with each domain comprising four conserved framework regions (FRs) and three complementarity determining regions (CDRs). (See, e.g., Kindt et al. Kuby Immunology, 6th ed., W.H. Freeman and Co., page 91 (2007).) A single VH or VL domain may be sufficient to confer antigen-binding specificity.

Fc regions

**The term "Fc region" herein is used to define a C**-terminal region of an immunoglobulin heavy chain that contains at least a portion of the constant region. The term includes native sequence Fc regions and mutant Fc regions. In one embodiment, a human IgG heavy chain Fc region extends from Cys226, or from Pro230, to the carboxyl-terminus of the heavy chain. However, the C-terminal lysine (Lys447) or glycine-lysine (Gly446-Lys447) of the Fc region may or may not be present. Unless otherwise specified herein, numbering of amino acid residues in the Fc region or constant region is according to the EU numbering system, also called the EU index, as described in Kabat et al., Sequences of Proteins of Immunological Interest, 5th Ed. Public Health Service, National Institutes of Health, Bethesda, MD, 1991. In the present invention, the Fc region can include various modifications. For example, modifications for increasing the yield of molecules in which Fc is heteroassociated and modifications for

**suppressing binding to Fc$\gamma$R are known.**

Nucleic acids/polynucleotides

An "isolated" nucleic acid/polynucleotide refers to a nucleic acid/polynucleotide molecule that has been separated from a component of its natural environment. An isolated nucleic acid/polynucleotide includes a nucleic acid/polynucleotide molecule contained in cells that ordinarily contain the nucleic acid/polynucleotide molecule, but the nucleic acid/polynucleotide molecule is present extrachromosomally or at a chromosomal location that is different from its natural chromosomal location.

The present disclosure also relates to a nucleic acid/polynucleotide that encodes a CXCR3 ligand, or a nucleic acid/polynucleotide that encodes a fusion protein comprising the CXCR3 ligand.

**"Nucleic acid/polynucleotide encoding a CXCR3 ligand" refers to one or more nucleic** acid molecules encoding a CXCR3 ligand, including such nucleic acid molecule(s) in a single vector or separate vectors, and such nucleic acid molecule(s) present at one or more locations in a host cell. **The "nucleic acid/polynucleotide encoding a CXCR3 ligand" may be functionally** linked to an expression control region. An expression control region includes a promoter, enhancer, terminator, and such. The expression control region to be linked to a nucleic acid/polynucleotide is generally heterogenic. Alternatively, the expression control region of a gene encoding an originally naturally-occurring CXCR3 ligand may be combined as necessary.

Vectors

The present disclosure also relates to vectors comprising a nucleic acid/polynucleotide encoding a CXCR3 ligand, or a nucleic acid/polynucleotide encoding a fusion protein comprising the CXCR3 ligand. The term "vector," as used herein, refers to a nucleic acid molecule capable of propagating another nucleic acid to which it is linked. The term includes the vector as a self-replicating nucleic acid structure as well as the vector incorporated into the genome of a host cell into which it has been introduced. Certain vectors are capable of directing the expression of nucleic acids to which they are operatively linked. Such vectors are referred to herein as "expression vectors." **The "nucleic acid/polynucleotide encoding a CXCR3 ligand"** may be functionally linked to an expression control region in an expression vector. An expression control region includes a promoter, enhancer, terminator, and such. The expression control region to be linked to a nucleic acid/polynucleotide is generally heterogenic. Alternatively, the expression control region of a gene encoding an originally naturally-occurring CXCR3 ligand may be combined as necessary.

Host cells

The terms "host cell," "host cell line," and "host cell culture" are used interchangeably and refer to cells into which exogenous nucleic acid has been introduced, including the progeny of such cells. Host cells include "transformants" and "transformed cells," which include the primary transformed cell and progeny derived therefrom without regard to the number of passages. Progeny may not be completely identical in nucleic acid content to a parent cell, but may contain mutations. Mutant progenies that have the same function or biological activity as screened or selected for in the originally transformed cell are included herein.

The present disclosure also relates to host cells comprising a nucleic acid/polynucleotide encoding a CXCR3 ligand or a nucleic acid/polynucleotide encoding a fusion protein comprising the CXCR3 ligand.

Methods for producing a CXCR3 ligand

The polynucleotide according to the present disclosure is usually carried by (or inserted in) an appropriate vector and transfected into host cells. The vector is not particularly limited as long as the vector can stably retain an inserted nucleic acid. For example, when *E. coli* is used as the host, a pBluescript vector (manufactured by Stratagene Corp.) or the like is preferred as a vector for cloning, although various commercially available vectors can be used.

In the case of using a vector for the purpose of producing a CXCR3 ligand or a fusion protein comprising a CXCR3 ligand of the present disclosure, an expression vector is particularly useful. The expression vector is not particularly limited as long as the vector permits expression of the ligand-binding molecule *in vitro,* in *E. coli,* in cultured cells, or in individual organisms. The expression vector is preferably, for example, a pBEST vector (manufactured by Promega Corp.) for *in vitro* expression, a pET vector (manufactured by Invitrogen Corp.) for expression in *E. coli,* a pME18S-FL3 vector (GenBank Accession No. AB009864) for expression in cultured cells, and a pME18S vector (Mol Cell Biol. 8: 466-472 (1988)) for expression in individual organisms. The insertion of the DNA of the present disclosure into the vector can be performed by a routine method, for example, ligase reaction using restriction sites (Current protocols in Molecular Biology edit. Ausubel et al. (1987) Publish. John Wiley & Sons. Section 11.4-11.11).

The host cells are not particularly limited, and various host cells are used according to the purpose. Examples of the cells for expressing the CXCR3 ligand or the fusion protein may include bacterial cells (e.g., *Streptococcus, Staphylococcus, E. coli, Streptomyces,* and *Bacillus subtilis*), fungal cells (e.g., yeasts and *Aspergillus*), insect cells (e.g., *Drosophila* S2 and *Spodoptera* SF9), animal cells (e.g., CHO, COS, HeLa, C127, 3T3, BHK, HEK293, and Bowes melanoma cells) and plant cells. The transfection of the vector to the host cells may be performed by a method known in the art, for example, a calcium phosphate precipitation method, an electroporation method (Current protocols in Molecular Biology edit. Ausubel et al., (1987) Publish. John Wiley & Sons. Section 9.1-9.9), a Lipofectamine method (manufactured by GIBCO-BRL), or a microinjection method.

An appropriate secretory signal can be incorporated into the ligand-binding molecule or the fusion protein of interest, in order to secrete the CXCR3 ligand or the fusion protein expressed in the host cells to the endoplasmic reticulum lumen, periplasmic space, or an extracellular environment. The signal may be endogenous to the ligand-binding molecule or the fusion protein of interest, or may be a foreign signal. Specifically, for example, the signal sequence MNQTAILICCLIFLTLSGIQG (SEQ ID NO: 48), MKKSGVLFLLGIILLVLIGVQG (SEQ ID NO: 49), MSVKGMAIALAVILCATVVQG (SEQ ID NO: 50), or MGWSCIILFLVATATGVHS (SEQ ID NO: 52) can be used. Among these signal sequences, SEQ ID NOs: 48 to 50 are derived from human CXCL10, human CXCL9, and human CXCL11, respectively.

When the CXCR3 ligand or the fusion protein of the present disclosure is secreted into a medium, the recovery of the CXCR3 ligand or the fusion protein in the above production method is performed by collecting the medium. When the CXCR3 ligand or the fusion protein of the present disclosure is produced in cells, the cells are first lysed and the CXCR3 ligand or the fusion protein is subsequently recovered.

A method known in the art including ammonium sulfate or ethanol precipitation, acid extraction, anion- or cation-exchange chromatography, phosphocellulose chromatography, hydrophobic interaction chromatography, affinity chromatography, hydroxylapatite chromatography, and lectin chromatography can be used for recovering and purifying the CXCR3 ligand or the fusion protein of the present disclosure from the recombinant cell cultures.

The CXCR3 ligands provided herein can also be produced by enhancing the activity of a parent CXCR3 ligand to induce migration of CXCR3-expressing-cells. In a particular embodiment, the method of producing a CXCR3 ligand provided herein comprises modifying the sequence of a parent CXCR3 ligand. In a particular embodiment, the method of producing a CXCR3 ligand provided herein comprises either one or both modifications of at least (i) introducing a disulfide bond and (ii) substituting an amino acid at the N-terminus region.

In a particular embodiment, the 1st amino acid from the N-terminus of a parent CXCR3 ligand used in a method of producing a CXCR3 ligand provided herein is V. In a particular embodiment, the 2nd amino acid from the N-terminus of a parent CXCR3 ligand used in a method of producing a CXCR3 ligand provided herein is P. In a more specific embodiment, the N-terminal sequence of a parent CXCR3 ligand used in a method of producing a CXCR3 ligand provided herein is V-P-L or V-V-L. In a more specific embodiment, the N-terminal sequence of a parent CXCR3 ligand used in a method of producing a CXCR3 ligand provided herein is V-P-L-S-R-T (SEQ ID NO: 19) or Y-V-L-S-R-T (SEQ ID NO: 14).

In a particular embodiment, the parent CXCR3 ligand used in a method of producing a CXCR3 ligand provided herein has a C-X-C motif. The two cysteines contained in the C-X-C motif can each form disulfide bonds with cysteines other than those of the C-X-C motif comprised in the parent CXCR3 ligand. The C-X-C motif in the parent CXCR3 ligand can be selected from C-T-C (Cys-Thr-Cys), C-L-C (Cys-Leu-Cys), and C-S-C (Cys-Ser-Cys). In a specific embodiment, the C-X-C motif in the parent CXCR3 ligand can be located adj acent to the C-terminus of the N-terminal sequence V-P-L-S-R-T-V-R (SEQ ID NO: 21) or V-V-L-S-R-T-V-R (SEQ ID NO: 22).

In a particular embodiment, the parent CXCR3 ligand used in a method of producing a CXCR3 ligand provided herein comprises a portion of the amino acid sequence of a naturally-occurring human CXCR3 ligand sequence. In a particular embodiment, the parent CXCR3 ligand used in a method of producing a CXCR3 ligand provided herein comprises a portion of the amino acid sequence of human CXCL10.

In a particular embodiment, the parent CXCR3 ligand used in a method of producing a CXCR3 ligand provided herein is selected from a naturally-occurring CXCL10, naturally-occurring CXCL11, naturally-occurring CXCL9, CXCL10 variant, CXCL11 variant, CXCL9 variant, and chimeric proteins prepared from them.

In the present invention, when a CXCR3 ligand is obtained by expression of a **polynucleotide encoding the same, an "amino acid substitution" may be introduced via** substitution of a polynucleotide codon. Alternatively, when a CXCR3 ligand is synthesized by binding of amino acid residues, an amino acid residue for substitution may be linked to obtain a CXCR3 ligand comprising an amino acid substitution.

The methods of producing a CXCR3 ligand provided herein may further additionally include, in a specific embodiment, a step of recovering or isolating the CXCR3 ligand containing the above-mentioned modifications.

Treatments

**As used herein, "treatment" (and grammatical variations thereof such as "treat" or "treating") refers to clinical intervention in an attempt to alter the natural course of the individual** being treated, and can be performed either for prophylaxis or during the course of clinical pathology. Desirable effects of treatment include, but are not limited to, preventing occurrence or recurrence of disease, alleviation of symptoms, diminishment of any direct or indirect pathological consequences of the disease, preventing metastasis, decreasing the rate of disease progression, amelioration or palliation of the disease state, and remission or improved prognosis. In some embodiments, the CXCR3 ligands of the present disclosure are used to delay development of a disease or to slow the progression of a disease.

Pharmaceutical compositions

The terms "pharmaceutical formulation" or "pharmaceutical composition" refer to a preparation which is in such form as to permit the biological activity of an active ingredient contained therein to be effective, and which contains no additional components which are unacceptably toxic to a subject to which the formulation would be administered.

Pharmaceutically acceptable carriers

**A "pharmaceutically acceptable carrier" refers to an ingredient in a pharmaceutical** formulation, other than an active ingredient, which is nontoxic to a subject. A pharmaceutically acceptable carrier includes, but is not limited to, a buffer, excipient, stabilizer, or preservative.

Pharmaceutical compositions comprising a CXCR3 ligand or a fusion protein comprising a CXCR3 ligand

The present disclosure also relates to pharmaceutical compositions (agents) comprising a CXCR3 ligand of the present disclosure and a pharmaceutically acceptable carrier, and pharmaceutical compositions (agents) comprising a fusion protein comprising a CXCR3 ligand of the present disclosure and a pharmaceutically acceptable carrier. In a particular embodiment, the pharmaceutical compositions of the present disclosure are pharmaceutical compositions for treating a disease, including, but not limited to, cancers, tumors, and inflammatory diseases.

In the present disclosure, the term "pharmaceutical composition comprising a CXCR3 ligand" may be used interchangeably with a "method for treating a disease, comprising administering a CXCR3 ligand to a subject to be treated" and may be used interchangeably with "use of a CXCR3 ligand for the manufacture of a medicament for treating a disease". Also, the term "pharmaceutical composition comprising a CXCR3 ligand" may be used interchangeably with "use of a CXCR3 ligand for treating a disease" or "use of a CXCR3 ligand in the treatment of a disease". The CXCR3 ligand of the present disclosure, the fusion protein comprising a CXCR3 ligand of the present disclosure, and the pharmaceutical composition of the present disclosure can be used for treating a disease, including, but not limited to, cancers, tumors, and inflammatory diseases.

The term "pharmaceutical composition comprising a fusion protein comprising a CXCR3 ligand" may be used interchangeably with a "method for treating a disease, comprising administering a fusion protein comprising a CXCR3 ligand to a subject to be treated" and may be used interchangeably with "use of a fusion protein comprising a CXCR3 ligand for the manufacture of a medicament for treating a disease". Also, the term "pharmaceutical composition comprising a fusion protein comprising a CXCR3 ligand" may be used interchangeably with "use of a fusion protein comprising a CXCR3 ligand for treating a disease" or "use of a fusion protein comprising a CXCR3 ligand in the treatment of a disease". Further, the present invention relates to "a method of producing a pharmaceutical composition for treating a disease, comprising the step of combining (or mixing) a CXCR3 ligand or a fusion protein comprising the same with a pharmaceutically acceptable carrier". In a particular embodiment, a disease may be a disease that requires administration of a CXCR3 ligand or a fusion protein comprising the same for the treatment thereof.

In some embodiments of the present disclosure, a composition comprising a CXCR3 ligand can be administered to an individual. In some embodiments of the present disclosure, a fusion protein comprising a CXCR3 ligand can be administered to an individual. When administering a CXCR3 ligand or a fusion protein comprising the same to a subject for the treatment of a disease, these active ingredients may be administered at a therapeutically effective amount. More specifically, the present invention may include the step of administering to a subject in need of treatment of a disease a therapeutically effective amount of a CXCR3 ligand or a fusion protein comprising the same.

The pharmaceutical composition of the present disclosure can be formulated by use of a method known to those skilled in the art. For example, the pharmaceutical composition can be parenterally used in a form of an injection of a sterile solution or suspension with water or any other pharmaceutically acceptable liquids. The pharmaceutical composition can be formulated, for example, by appropriately combining with a pharmacologically acceptable carrier or medium, specifically, sterile water or physiological saline, a vegetable oil, an emulsifier, a suspending agent, a surfactant, a stabilizer, a flavoring agent, an excipient, a vehicle, an antiseptic, a binder, etc. and mixing them into a unit dosage form required for generally accepted pharmaceutical practice. The amount of the active ingredient in these formulations is set so as to give an appropriate volume in a prescribed range.

A sterile composition for injection can be formulated according to usual pharmaceutical practice using a vehicle such as injectable distilled water. Examples of the injectable aqueous solution include isotonic solutions containing physiological saline, glucose, or other adjuvants (e.g., D-sorbitol, D-mannose, D-mannitol, and sodium chloride). The aqueous solution can be used in combination with an appropriate solubilizer, for example, an alcohol (ethanol, etc.), a polyalcohol (propylene glycol, polyethylene glycol, etc.), or a nonionic surfactant (Polysorbate 80™, HCO-50, etc.).

Examples of the oily liquid include sesame oil and soybean oil, and benzyl benzoate and/or benzyl alcohol can be used in combination as a solubilizer. The oily liquid can be combined with a buffer (e.g., a phosphate buffer solution and a sodium acetate buffer solution), a soothing agent (e.g., procaine hydrochloride), a stabilizer (e.g., benzyl alcohol and phenol), or an antioxidant. The prepared injection solution is usually filled into an appropriate ampule.

The pharmaceutical composition of the present disclosure is preferably administered through a parenteral route. For example, a composition for injection, transnasal administration, transpulmonary administration, or percutaneous administration is administered. The pharmaceutical composition can be administered systemically or locally by, for example, intravenous injection, intramuscular injection, intraperitoneal injection, or subcutaneous injection.

The administration method can be appropriately selected according to the age and symptoms of a patient. The dose of the pharmaceutical composition containing the CXCR3 ligand can be determined to the range of, for example, 0.0001 mg to 1000 mg per kg body weight per dose. Alternatively, the dose can be determined to, for example, 0.001 mg to 100000 mg per patient. However, the present disclosure is not necessarily limited by these numerical values. The dose and the administration method vary depending on the body weight, age, symptoms, and such of a patient, and those skilled in the art can determine an appropriate dose and administration method in consideration of these conditions.

[Examples]

The following are examples of methods and compositions of the present disclosure. It is understood that various other embodiments may be practiced, given the general description provided above.

Example 1. Construction of human CXCL10 (HCXCL10) variants and HCXCL10 variant Fc fusions

An hCXCL10 variant, hCXCL10R75A (SEQ ID NO: 1), in which human CXCL10 (hCXCL10, Refseq: NP_001556.2, Uniprot ID: P02778) has been mutated to have resistance to furin protease, and human CXCL10 variants in which modifications have been introduced into the N-loop, the a-helix region at the C-terminus, and/or an N-terminal region were constructed. Table 2 shows the names, modifications, and SEQ ID NOs of the constructed hCXCL10 variants. In wild-type CXCL10 with no introduction of the R75A modification, when expressed in mammalian cells, this portion is degraded by protease derived from the cells and separated from a protein fused to a C-terminal region. Therefore, it is desirable to add the R75A modification to express a CXCL10-protein fusion in which wild-type CXCL10 or a variant thereof is fused with a protein at the C-terminus.

[Table 2]

| Sequence name | SEQ ID NO | Modification | N-terminal sequence | Corresponding hCXCL10 variant Fc fusion |
|---|---|---|---|---|
| hCXCL10R75A | 1 | R75A | VPLSRTVR ... | hCXCL10R75A-G1T4k.one//VHn-G1T4h.one.H435R |

(continued)

| Sequence name | SEQ ID NO | Modification | N-terminal sequence | Corresponding hCXCL10 variant Fc fusion |
|---|---|---|---|---|
| hCXCL10R75A.0639 | 4 | V1Y/R75A | YPLSRTVR··· | hCXCL10R75A.0639-G1T4k.one//VHn-G1T4h.one.H435R |
| hCXCL10R75A.0640 | 5 | P18C/R75A | VPLSRTVR··· | hCXCL10R75A.0640-G1T4k.one//VHn-G1T4h.one.H435R |
| hCXCL10R75A.0641 | 6 | A60C/R75A | VPLSRTVR ... | hCXCL10R75A.0641-G1T4k.one//VHn-G1T4h.one.H435R |
| hCXCL10R75A.0642 | 7 | P18C/A60C/R75A | VPLSRTVR··· | hCXCL10R75A.0642-G1T4k.one//VHn-G1T4h.one.H435R |
| hCXCL10R75A.0016 | 8 | P2V/R75A | VVLSRTVR ... | hCXCL10R75A.0016-G1T4k.one//VHn-G1T4h.one.H435R |
| hCXCL10R75A.0643 | 9 | V1Y/P2V/R75A | YVLSRTVR··· | hCXCL10R75A.0643-G1T4k.one//VHn-G1T4k.one.H435R |
| hCXCL10R75A.0644 | 10 | P2V/P18C/A60C/R75A | VVLSRTVR··· | hCXCL10R75A.0644-G1T4k.one//VHn-G1T4h.one.H435R |
| hCXCL10R75A.0669 | 11 | V1Y/P18C/A60C/R75A | YPLSRTVR··· | hCXCL10R75A.0669-G1T4k.one//VHn-G1T4h.one.H435R |
| hCXCL10R75A.0670 | 12 | V1Y/P2V/P18C/A60C/R75A | YVLSRTVR··· | hCXCL10R75A.0670-G1T4k.one//VHn-G1T4h.one.H435R |

In Table 2, either of YPLSRTVR (SEQ ID NO: 15), YVLSRTVR (SEQ ID NO: 16), VPLSRTVR (SEQ ID NO: 21), and VVLSRTVR (SEQ ID NO: 22) is shown as the N-terminal sequence.

As described above, to promote binding to CXCR3 and enhance the ability to activate CXCR3-expressing-cells, modifications to stabilize into a CXCR3-binding state were examined, and the modifications that contribute to the receptor binding step were determined by analysis using general computational science system, Molecular Operating Environment (Chemical Computing Group ULC., MOE 2020.0901 win64). Specifically, the MOE read the structural data of HCXCL10 (PDB ID: 1O7Y) and computed structural stabilization resulting from introduction of modifications, and the modifications that are considered to stabilize the structure around the docking domain were discovered from them (Figure 1A). Any of the amino acid combination selected from the 14th and the 55th, the 18th and the 60th, the 21st and the 67th, the 25th and the 46th, and the 41st and the 56th from the N-terminus was extracted as a candidate for the positions to introduce a disulfide bond which increases the stability of a variant relative to parent CXCR3 ligands. Among them, the variant where the combination of the 18th and the 60th is substituted with cysteines was examined.

Regarding modifications in an N-terminal region that contribute to the activation step, searching was performed for substitutions of amino acid residues in the N-terminal region, with the idea that the interaction can be stabilized by regulating the hydrophobicity and the size of side chains of the N-terminal region because an interaction site on a receptor is the transmembrane regions. Specifically, variants where the V1Y substitution was made to the amino acid at the N-terminus were examined.

In order to facilitate the purification of the various hCXCL10 variants, hCXCL10 variant Fc fusions were constructed by fusing the hCXCL10 variants with the human IgG1 antibody Fc domain. A schematic diagram of an hCXCL10 variant Fc fusion is shown in FIG. 1B.

hCXCLIO variant Fc fusions, in which the above hCXCLIO variants are fused with the human IgG1 antibody (hIgG1) Fc domain variant G1T4k.one//VHn-G1T4h.one.H435R (G1T4k.one (SEQ ID NO: 2) and VHn-G1T4h.one.H435R (SEQ ID NO: 3)), were prepared. Specifically, expression vectors encoding genes of peptide chains in which the C-terminus of each hCXCL10 variant and the N-terminus of G1T4k.one in G1T4k.one//VHn-G1T4h.one.H435R are linked were prepared using a method known to those skilled in the art. These peptide chains were combined with VHn-G1T4h.one.H435R, and HCXCL10 variant Fc fusions, in which one hCXCL10 variant is bound to an hIgG1 Fc domain variant, were expressed by transient expression using Expi 293 (Life Technologies) by a method known to those skilled in the art and purified by a method known to those skilled in the art using protein A.

The Fc domain variant used in this study has a mutation for increasing the yield of Fc heteroassociated molecules **and a mutation that suppresses Fc$\gamma$R binding.**

Example 2. Evaluation of the cell migration-inducing activity of hCXCL10 variant Fc fusions

Whether the hCXCLIO variant Fc fusions prepared in Example 1 induce migration of cells expressing the CXCL10 receptor, i.e., the activity of inducing migration of cells expressing CXCR3 was evaluated.

Cell migration-inducing activity was evaluated using Ba/F3 transfectant cells expressing mouse CXCR3 (mCXCR3) (hereinafter referred to as BaF3/mCXCR3) and HTS Transwell™-96 Permeable Supports **with 5.0 $\mu$m Pore Polycarbonate Membrane (Cat. 3387, Corning).**

As analytes, the following hCXCL10 variant Fc fusions prepared in Example 1 were used:

hCXCL10R75A-G1T4k.one//VHn-G1 T4h.one.H43 5R,
hCXCL10R75A.0639-G1T4k.one//VHn-G1T4h.one.H435R,
hCXCL10R75A.0640-G1T4k.one//VHn-G1T4h.one.H435R,
hCXCL10R75A.0641-G1T4k.one//VHn-G1T4h.one.H435R,
hCXCL10R75A.0642-G1T4k.one//VHn-G1T4h.one.H435R,
hCXCL10R75A.0016-G1T4k.one//VHn-G1T4h.one.H435R,
hCXCL10R75A.0643-G1T4k.one//VHn-G1T4h.one.H435R,
hCXCL10R75A.0644-G1T4k.one//VHn-G1T4h.one.H435R,
hCXCL10R75A.0669-G1T4k.one//VHn-G1T4h.one.H435R, and
hCXCL10R75A.0670-G1T4k.one//VHn-G1T4h.one.H435R.

After adjusting the final concentrations in the solutions of each analyte to be analyzed to 3 nM, 10 nM, 100 **nM, 300 nM, and 1000 nM, 235 $\mu$L of each solution was transferred to the lower chamber. Then, BaF3/mCXCR3 cells were seeded into the upper chamber at 75 $\mu$L/well** so as to be $2.0 \times 10^5$ cells/well, and the reaction was carried out for 5 hours. The reaction was carried out under the conditions of 5% carbon dioxide and 37°C. After 3 or 6 hours of reaction, **100 $\mu$L of the solution in the lower chamber was transferred to OptiPlate-96 (Cat. 6005299, PerkinElmer) and 100 $\mu$L of CellTiter**-Glo™ Luminescent Cell Viability Assay solution (Cat. G7571, Promega) was added. After reacting at room temperature for 10 minutes, the luminescence value was measured with a SpectraMax M3 multimode microplate reader (Molecular Devices) to evaluate the level of migration of cells into the lower chamber. The migration rate of cells to analytes in each well was calculated as a relative value of luminescence from the lower chamber to luminescence obtained from the total amount of cells loaded in the upper chamber, *i.e.,* **"luminescence/luminescence from all cells".**

The amount of cells that migrated to the lower chamber is reflected in fluorescence intensities. WO2020/116498A1 discloses that both unmodified hCXCL10 and hCXCL10R75A-G1T4k.one//VHn-G1T4h.one.H435R showed concentration-dependent cell migration-inducing activity.

(1) P18C, A60C variant

Comparison of the cell migration-inducing activity between hCXCL10R75A-G1 T4k.one//VHn-G1 T4h.one.H435R and other hCXCL10 variant Fc fusions was performed after five hours of reaction, and the results are shown in Figure 2 and Table 3. Compared to hCXCL10R75A-G1T4k.one//VHn-G1T4h.one.H435R, hCXCL10R75A.0642-G1T4k.one//VHn-G1T4h.one.H435R into which the P18C/A60C modification is introduced showed higher cell migration-inducing activity at each of the concentrations of 3 nM, 10 nM, and 300 nM than hCXCL10R75A-G1T4k.one//VHn-G1T4h.one.H435R at the same concentration. Meanwhile, each of the P18C modification and the A60C modification did not show the effect of enhancing the activity individually. Accordingly, it was strongly suggested that the enhancement of the activity by the P18C/A60C modification was due to introduction of a S-S bond between amino acid positions 18 and 60 and resulting structural stabilization of peripheral regions.

[Table 3]

| Activity of each variant when activity of hCXCL10R75A at each concentration is regarded as 100% (P18C, A60C variant) | | | | | | | | |
|---|---|---|---|---|---|---|---|---|
| Variant name | Modification | 0 nM | 3 nM | 10 nM | 30 nM | 100 nM | 300 nM | 1000 nM |
| hCXCL10R75A | R75A | 100% | 100% | 100% | 100% | 100% | 100% | 100% |
| hCXCL10R75A.0640 | P18C/R75A | 100% | 22% | 24% | 46% | 104% | 175% | 368% |
| hCXCL10R75A.0641 | A60C/R75A | 100% | 17% | 33% | 54% | 103% | 177% | 370% |
| hCXCL10R75A.0642 | P18C/A60C/R75A | 100% | 196% | 150% | 106% | 98% | 116% | 80% |

(2) P18C, A60C, P2V variant

P2V is reported as a modification that is introduced into HCXCL10 to confer DPPIV cleavage resistance (WO2020/116498A1). Here, the effect of enhancing the activity by the combination of the P2V modification and the P18C/A60C modification was verified.

Compared to hCXCL10R75A-G1 T4k.one//VHn-G1 T4h.one.H435R, hCXCL10R75A.0644-G1T4k.one//VHn-G1T4h.one.H435R into which the P2V/P18C/A60C modification is introduced showed higher cell migration-inducing activity at each of the concentrations of 3 nM, 10 nM, 300 nM, and 1000 nM than hCXCL10R75A-G1T4k.one//VHn P18C/A60C modified-G1T4h.one.H435R at the same concentration (Figure 3 and Table 4). Accordingly, it was shown that the combination of the P18C/A60C modification and the P2V modification exerted the effect of enhancing the activity. Furthermore, the CXCL10 variant having DPPIV cleavage resistance and the effect of enhancing the activity was created by combining the P18C/A60C modification and the P2V modification.

[Table 4]

| Activity of each variant when activity of hCXCL10R75A at each concentration is regarded as 100% (P18C, A60C, P2V variant) | | | | | | | | |
|---|---|---|---|---|---|---|---|---|
| Variant name | Modification | 0 nM | 3 nM | 10 nM | 30 nM | 100 nM | 300 nM | 1000 nM |
| hCXCL10R75A | R75A | 100% | 100% | 100% | 100% | 100% | 100% | 100% |
| hCXCL10R75A.0642 | P18C/A60C/R75A | 100% | 196% | 150% | 106% | 98% | 116% | 80% |
| hCXCL10R75A.0016 | P2V/R75A | 100% | 79% | 83% | 92% | 105% | 129% | 158% |
| hCXCL10R75A.0644 | P2V/P18C/A60C/R75A | 100% | 174% | 144% | 109% | 107% | 153% | 163% |

(3) V1Y variant

Compared to hCXCL10R75A-G1T4k.one//VHn-G1T4h.one.H435R, hCXCL10R75A.0639-G1T4k.one//VHn-G1T4h.one.H435R having an improved degree of hydrophobicity at the N-terminus due to V1Y showed higher cell migration-inducing activity at each of the concentrations of 3 nM, 30 nM, 100 nM, and 300 nM than hCXCL10R75A-G 1 T4k.one//VHn-G 1 T4h.one.H435R at the same concentration.

As described above, P2V is reported as a modification that is introduced into hCXCL10 to confer DPPIV cleavage resistance. hCXCL10R75A.0643-G1T4k.one//VHn-G1T4h.one.H435R into which the V1Y/P2V modification is introduced showed higher cell migration-inducing activity at each of the concentrations of 3 nM, 30 nM, 100 nM, and 300 nM than hCXCL10R75A-G1T4k.one//VHn-G1T4h.one.H435R at the same concentration (Figure 4 and Table 5). Accordingly, it was shown that the combination of the V1Y modification and the P2V modification exerted the effect of enhancing the activity. Furthermore, the CXCL10 variant having DPPIV cleavage resistance and the effect of enhancing the activity was created by combining the V1Y modification and the P2V modification.

[Table 5]

| Activity of each variant when activity of hCXCL10R75A at each concentration is regarded as 100% (V1Y variant) | | | | | | | | |
|---|---|---|---|---|---|---|---|---|
| Variant name | Modification | 0 nM | 3 nM | 10 nM | 30 nM | 100 nM | 300 nM | 1000 nM |
| hCXCL10R75A | R75A | 100% | 100% | 100% | 100% | 100% | 100% | 100% |

(continued)

| Activity of each variant when activity of hCXCL10R75A at each concentration is regarded as 100% (V1Y variant) | | | | | | | | |
|---|---|---|---|---|---|---|---|---|
| Variant name | Modification | 0 nM | 3 nM | 10 nM | 30 nM | 100 nM | 300 nM | 1000 nM |
| hCXCL10R75A.0639 | V1Y/R75A | 100% | 124% | 100% | 113% | 118% | 110% | 25% |
| hCXCL10R75A.0016 | P2V/R75A | 100% | 79% | 83% | 92% | 105% | 129% | 158% |
| hCXCL10R75A.0643 | V1Y/P2V/R75A | 100% | 126% | 100% | 129% | 141% | 124% | 26% |

(4) V1Y, P18C, A60C variant

hCXCL10R75A.0642-G1T4k.one//VHn-G1T4h.one.H435R into which the V1Y/P18C/A60C modification, which is the combination of the P18C/A60C modification and the V1Y modification discovered herein, is introduced, and hCXCL10R75A.0670-G1T4k.one//VHn-G1T4h.one.H435R into which the V1Y/P2V/P18C/A60C modification, where the P2V modification is further joined to the above combination, is introduced, showed higher cell migration-inducing activity at each of the concentrations of 3 nM, 10 nM, 30 nM, 100 nM, and 300 nM than hCXCL10R75A-G1T4k.one//VHn-G1T4h.one.H435R at the same concentration (Figure 5 and Table 6).

[Table 6]

| Activity of each variant when activity of hCXCL10R75A at each concentration is regarded as 100% (V1Y, P18C, A60C variant) | | | | | | | | |
|---|---|---|---|---|---|---|---|---|
| Variant name | Modification | 0 nM | 3 nM | 10 nM | 30 nM | 100 nM | 300 nM | 1000 nM |
| hCXCL10R75A | R75A | 100% | 100% | 100% | 100% | 100% | 100% | 100% |
| hCXCL10R75A.0639 | V1Y/R75A | 100% | 124% | 100% | 113% | 118% | 110% | 25% |
| hCXCL10R75A.0642 | P18C/A60C/R75A | 100% | 196% | 150% | 106% | 98% | 116% | 80% |
| hCXCL10R75A.0670 | V1Y/P2V/P18C/A60C/R75A | 100% | 195% | 146% | 132% | 132% | 127% | 24% |

While hCXCL10R75A.0639-G1T4k.one//VHn-G1T4h.one.H435R and hCXCL10R75A.0642-G1T4k.one//VHn-G1T4h.one.H435R tended to show higher cell migration-inducing activity than hCXCL10R75A-G1T4k.one//VHn-G1T4h.one.H435R in a low concentration range and a middle concentration range, respectively, hCXCL10R75A.0670-G1T4k.one//VHn-G1T4h.one.H435R exerted the effect of enhancing the activity in a wide concentration range from a low concentration range to a middle concentration range.

Accordingly, it was shown that while the modifications discovered in each of the step where CXCL10 binds to CXCR3 and the activation step exerted the effect individually, the effect of enhancing the activity was exerted in a wider concentration range by combining them.

In view of the above, it was shown that human CXCL10 variants included in these human CXCL10 variant Fc fusions had higher activity relative to parent CXCR3 ligands before modification.

Example 3. Construction of human CXCL10 (hCXCL 1 0) variants for improvement of stability in blood and hCXCL10 variant-antibody fusions

(3-1) Human CXCL10 (hCXCL10) variants for improvement of resistance against protease cleavage

The N-terminus of hCXCLIO is known to be cleaved by various proteases such as DPP4, DPP8, DPP9, Chathepsin K, Chathepsin S, Chathepsin L, MMP-9, and MMP-2, and such cleavage is known to lower the activity as a CXCR3 agonist (Non-Patent Literature: Bronger, H., Magdolen, V., Goettig, P. et al. Proteolytic chemokine cleavage as a regulator of lymphocytic infiltration in solid tumors. Cancer Metastasis Rev 38, 417-430 (2019)). Therefore, to retain the activity of CXCL10, it is preferable to prevent undesirable cleavage from taking place at the hCXCLIO N-terminal region by proteases that are present, for example, in blood, tumor sites, and sites of inflammation. To improve the resistance to protease cleavage of the hCXCL10 N-terminal region, hCXCL10R75A.0303 (SEQ ID NO: 55) which is hCXCL10R75A.0016 to which a V7P modification has been added was constructed.

(3-2) Construction of hCXCL10 variant-antibody fusions

As molecules that enable CXCL10 variants of the present invention to exert effects at specific sites (for example, tumors) *in vivo*, construction of fusions between a CXCL10 variant which is a ligand and a ligand-binding molecule which binding activity to the CXCL10 variant can be adjusted was undertaken. Ligand-binding molecules which ligand-binding activity can be adjusted have been already reported (for example, WO 2019/107380 and WO 2019/107384). Using as template a DNA sequence encoding MabCXCL10_G7 (heavy chain: G7HFR0039H-G1T4h (SEQ ID NO: 56); light chain: G7L-LT0 (SEQ ID NO: 57)) which is a neutralizing antibody against human CXCL10, a DNA sequence encoding the anti-CXCL10 antibody heavy chain G7HFR0039H.12aa0054-G1T4h (SEQ ID NO: 58), which has a sequence that is cleaved by cancer-specifically expressed urokinase (uPA) and matriptase (MT-SP1) inserted near the boundary between the variable region and the constant region of G7HFR0039H-G 1T4h, was prepared by a method known to those skilled in the art. Furthermore, expression vectors encoding the hCXCLIO fusion-type anti-hCXCL10 antibody heavy chains (hCXCL10R75A.G4SGGGG.G7HFR0039H.12aa0054-G1T4h (SEQ ID NO: 59), hCXCL10R75A.0016.G4SGGGG.G7HFR0039H.12aa0054-G1T4h (SEQ ID NO: 60), and hCXCL10R75A.0303.G4SGGGG.G7HFR0039H.12aa0054-G1T4h (SEQ ID NO: 61)) that have a linker sequence (SEQ ID NO: 66) consisting of a glycine-serine polymer inserted to the boundary between the hCXCLIO mutants (hCXCL10R75A (SEQ ID NO: 1), hCXCL10R75A.0016 (SEQ ID NO: 8), and hCXCL10R75A.0303 (SEQ ID NO: 55)) and the variable region of the anti-CXCL10 antibody heavy chain G7HFR0039H.12aa0054-G1T4h were prepared by a method known to those skilled in the art.

Furthermore, when expressing hCXCL10 fusion-type anti-hCXCL10 antibodies, to express molecules that have one HCXCL10 molecule bound per molecule, an expression vector encoding the anti-keyhole-limpet hemocyanin antibody heavy chain (IC17HdK-G1 T4k.H435R (SEQ ID NO: 62)) was prepared by a method known to those skilled in the art. Fc variants used in this study have introduced therein a mutation for increasing the yield of molecules in which Fc is heteroassociated **and a mutation that suppresses FcγR binding**.

These heavy chain variants were combined with a light chain to express the HCXCL10 fusion-type anti-hCXCL10 antibodies:

hCXCL10R75A.G4SGGGG.G7HFR0039H.12aa0054-G1T4h/G7L-LT0//IC17HdK-G1T4k.H435R/G7L-LT0 (SEQ ID NO: 59, SEQ ID NO: 57, SEQ ID NO: 62);

hCXCL10R75A.0016.G4SGGGG.G7HFR0039H.12aa0054-G1T4h/G7L-LT0//IC17HdK-G1T4k.H435R/G7L-LT0 (SEQ ID NO: 60, SEQ ID NO: 57, SEQ ID NO: 62); and

hCXCL10R75A.0303.G4SGGGG.G7HFR0039H.12aa0054-G1T4h/G7L-LT0//IC17HdK-G1T4k.H435R/G7L-LT0 (SEQ ID NO: 61, SEQ ID NO: 57, SEQ ID NO: 62);

by transient expression using Expi293 (Life Technologies) by a method known to those skilled in the art, and then these were purified by a method known to those skilled in the art using protein A.

Example 4. Evaluation of the stability in blood of hCXCL10 variant-antibody fusions

(4,1) (P2V, V7P)

Mouse PK studies were performed to evaluate the stability in blood of CXCL10.
As analytes, the following hCXCL10 fusion-type anti-hCXCL10 antibodies prepared in Example 3 were used:

hCXCL10R75A.G4SGGGG.G7HFR0039H.12aa0054-G1T4h/G7L-LT0//IC17HdK-G1T4k.H43 5R/G7L-LTO;
hCXCL10R75A.0016.G4SGGGG.G7HFR0039H.12aa0054-G1T4h/G7L-LT0//IC17HdK-G1T4k.H435R/G7L-LT0; and
hCXCL10R75A.0303.G4SGGGG.G7HFR0039H.12aa0054-G1T4h/G7L-LT0//IC17HdK-G1T4k.H43 5R/G7L-LT0.

Each antibody molecule was intravenously administered at 10 mg/kg to 6-week-old male C57BL/6J mice (Jackson Laboratory Japan, Inc.) at N=3, and blood was collected over time from the jugular vein 5 minutes, 7 hours, 1 day, 3 days, and 7 days after administration, or 5 minutes, 30 minutes, 1 hour, 4 hours, 1 day, and 5 days after administration, or 5 minutes, 1 hour, 4 hours, 1 day, and 5 days after administration. The collected blood samples were centrifuged at 4000 g for 10 minutes at 4°C, and the supernatants were collected as plasma samples. The plasma antibody concentrations were determined by the LC/ESI-MS/MS method. Calibration curve samples were prepared at 0.5, 1.0, 2.0, 4.0, 8.0, 16, 32 $\mu$g/mL or at 0.25, 0.5, 1.0, 2.0, 4.0, 8.0, 16, 32 $\mu$g/mL using mouse plasma, and then 3 $\mu$L of the calibration curve sample or a plasma sample collected from mice was mixed with 50 $\mu$L of anti-human Fc region antibody-immobilized magnetic beads (prepared in-house) suspended in 50 mmol/L ammonium hydrogen carbonate or in LowCross-buffer (Candor Bioscience GmbH, 100 500). The beads were washed three times with PBS containing 0.05% Tween-20 and once with PBS, then mixed with 25 $\mu$L of a protein denaturing solution (50 mmol/L ammonium hydrogen carbonate containing 8 mmol/L dithiothreitol, 7.5 mol/L urea, and 99 ng/mL lysozyme), and then incubated at 56°C for 45 minutes. This was then subjected to addition of 2 $\mu$L of 500 mmol/L iodoacetamide, incubation at 37°C for 30 minutes in the dark, addition of 160 $\mu$L of 50 mmol/L ammonium hydrogen carbonate containing 0.621 $\mu$g/mL sequencing grade modified trypsin (Promega, V5117), then incubation overnight at 37°C, and then trypsin digestion. Following addition of 5 $\mu$L of 10% TFA, 80 $\mu$L of the quenched sample was collected, and analyzed by LC/ESI-MS/MS.
LC/ESI-MS/MS was performed using a Xevo TQ-S tandem quadrupole mass spectrometer (Waters) equipped with Acquity I-class or I-class plus 2D high-performance liquid chromatography (Waters). Antibody-derived peptides produced by trypsin digestion were quantified by selected reaction monitoring (SRM). The peptide sequences used for quantification and SRM are shown in Table 7. Using the Masslynx Ver.4.2 (Waters) software, sample quantification was performed using a calibration curve constructed by a $1/X^2$ weighted linear regression of the peak areas versus the antibody concentrations. Each of the quantified peptide concentrations was divided by the peptide concentration of the antibody CH region, and the residual ratio of each sequence position was calculated.
The results are shown in Fig. 7 and Table 8. Compared to hCXCL10R75A.G4SGGGG.G7HFR0039H.12aa0054-G1T4h/G7L-LT0//IC17HdK-G1T4k.H435R/G7L-LT0, hCXCL 10R75A.0016.G4SGGGG.G7HFR0039H. 12aa0054-G1T4h/G7L-LT0//IC17HdK-G1T4k.H435R/G7L-LT0 into which the P2V modification is introduced showed higher percentage of hCXCL10 N-terminal retention than hCXCL10R75A.G4SGGGG.G7HFR0039H.12aa0054-G1T4h/G7L-LT0//IC17HdK-G1T4k.H435R/G7L-LT0 at each of the time points, which were Day 1 and Day 5.
Furthermore, compared to hCXCL10R75A.G4SGGGG.G7HFR0039H.12aa0054-G1T4h/G7L-LT0//IC17HdK-G1T4k.H435R/G7L-LT0, hCXCL10R75A.0303.G4SGGGG.G7HFR0039H.12aa0054-G1T4h/G7L-LT0//IC17HdK-G1T4k.H435R/G7L-LT0 into which the P2V/V7P modification is introduced showed higher percentage of hCXCL10 N-terminal retention than hCXCL10R75A.G4SGGGG.G7HFR0039H.12aa0054-G1T4h/G7L-LT0//IC17HdK-G1T4k.H435R/G7L-LT0 and hCXCL10R75A.0016.G4SGGGG.G7HFR0039H.12aa0054-G1T4h/G7L-LT0//IC17HdK-G1T4k.H435R/G7L-LT0 at each of the time points, which were Day 1 and Day 5.
Accordingly, HCXCL10 containing the P2V and/or V7P modifications were shown to have improved stability in blood compared to HCXCL10 not containing these modifications. This shows that hCXCL10 exhibits a particularly superior effect when fused with an antigen-binding molecule or a peptide having a cleavage site that may be cleaved by a tumor- or inflammation-specific protease. Specifically, hCXCL10 containing the P2V and/or V7P modification have improved stability in blood compared to hCXCL10 not containing these modifications; therefore, these modifications can prevent hCXCR10 from being non-specifically cleaved by proteases in the blood. Accordingly, for example, hCXCL10 variant-antibody fusions can reach sites such as target organs, lesions, and tumors without the hCXCL10 variant becoming cleaved in the blood, and the hCXCLIO variant (released from the antibody, for example, by cleavage of the antibody fused to the hCXCL10 variant by a site-specific protease) can exert its effects at those sites.

[Table 7]

| Table showing correspondence between the monitor peptides and SRM | | |
|---|---|---|
| Region | Monitor peptide | SRM |
| CXCL10 (N-terminus) | VPLSR | 286. 182>236. 648 |
| CXCL10 (N-terminus) | VVLSR | 287. 19>375. 215 |
| CXCL10 | CTCISISNQPVNPR | 823. 393>1111. 585 |
| CXCL10 | CTGISISNQCVNPR | 854. 882 > 1174. 563 |
| CH | GPSVFPLAPSSK | 593. 827>699. 404 |

[Table 8]

| Percentage of N-terminal peptide retention | | |
|---|---|---|
| Antibody | Days after administration | N-terminus retention rate |
| hCXCL10R75A.G4SGGGG.G7HFR0039H.12aa0054-G1T4h/G7L-LT0//IC17HdK-G1T4k.H435R/G7L-LT0 | 1 | 23.6% |
| hCXCL10R75A.0016.G4SGGGG.G7HFR0039H.12aa0054-G1T4h/G7L-LT0//IC17HdK-G1T4k.H435R/G7L-LT0 | 1 | 65.0% |
| | 5 | 34.0% |
| hCXCL10R75A.0303.G4SGGGG.G7HFR0039H.12aa0054-G1T4h/G7L-LT0//IC17HdK-G1T4k.H435R/G7L-LT0 | 1 | 73.6% |
| | 5 | 51.5% |

Example 5. Evaluation of the cell migration-inducing activity of hCXCL 10 variant Fc fusions

Examinations up to Example 4 led to the discovery of V7P as the modification to be introduced into hCXCL10 to achieve stability in blood. In this Example, the effects of enhancement of cell migration-inducing activity when the V7P modification is combined with the V1Y/P2V/P18C/A60C modification was confirmed.

The hCXCL10 variant (hCXCL10R75A.0613 (SEQ ID NO: 63)) having the additionally introduced V7P modification as compared to hCXCL10R75A.0670, which was formed by introducing the P2V modification which is a DPPIV resistance modification and V1Y/P18C/A60A which is an activity-enhancing modification to hCXCL10R75A, was constructed. hCXCL10 variant Fc fusions, in which this hCXCL10 variant is fused with the human IgG1 antibody (hIgGI) Fc domain variant G1T4k.one//VHn-G1T4h.one.H435R (G1T4k.one (SEQ ID NO: 2) and VHn-G1T4h.one.H435R (SEQ ID NO: 3)) were prepared. Specifically, expression vectors encoding genes of peptide chains in which the C-terminus of each hCXCL10 variant and the N-terminus of G1T4k.one within G1T4k.one//VHn-G1T4h.one.H435R are linked were prepared using a method known to those skilled in the art. These peptide chains were combined with VHn-G1T4h.one.H435R, and hCXCL10 variant Fc fusions, in which one hCXCL10 variant is bound to an hIgG1 Fc domain variant, were expressed by transient expression using Expi 293 (Life Technologies) by a method known to those skilled in the art and purified by a method known to those skilled in the art using protein A.

The Fc domain variant used in this study has introduced therein a mutation for increasing the yield of molecules in which Fc is heteroassociated and a mutation that suppresses FcγR binding.

hCXCL10R75A.0613-G1T4k.one//VHn-G1T4h.one.H435R showed the same or higher cell migration-inducing activity at each of the concentrations of 3 nM, 10 nM, 30 nM, 100 nM, and 300 nM than hCXCL10R75A.0670-G1 T4k.one//VHn-G1 T4h.one.H435R at the same concentrations (Fig. 8, Table 9). Accordingly, it was shown that the V7P modification does not inhibit the effect of enhancing the activity even when it is combined with the P2V modification which is a DPPIV resistance modification and with V1Y/P18C/A60A which is an activity-enhancing modification.

[Table 9]

| Activity of hCXCL10R75A.0613 when activity of hCXCL10R75A.0670 at each concentration is regarded as 100% | | | | | | | | |
|---|---|---|---|---|---|---|---|---|
| Variant name | Modification | 0nM | 3nM | 10nM | 30nM | 100nM | 300nM | 1000nM |
| hCXCL10R75A.0670 | V1Y/P2V/P18C/A60C/R75A | 100% | 100% | 100% | 100% | 100% | 100% | 100% |
| hCXCL10R75A.0613 | V1Y/P2V/V7P/P18C/A60C/R75A | 81% | 209% | 132% | 110% | 102% | 93% | 49% |

Example 6. Evaluation of stability in blood of HCXCL10 variant-antibody fusions

(6.1)
Using the DNA sequence encoding

hCXCL10R75A.0303.G4SGGGG.G7HFR0039H.12aa0054-G1T4h (SEQ ID NO: 61) as a template, a DNA sequence encoding
hCXCL10R75A.0659.G4SGGGG.G7HFR0039H.0004.N0222-G1T4h (SEQ ID NO: 64) was prepared using a method known to those skilled in the art.
hCXCL10R75A.0659.G4SGGGG.G7HFR0039H.0004.N0222-G1T4h has P18C/A60C, which is an activity-enhancing modification, added to the hCXCL10 portion as compared to hCXCL10R75A.0303.G4SGGGG.G7HFR0039H.12aa0054-G1T4h. Modifications have also been added to the protease recognition sequence inserted between the variable region and the constant region and to the heavy chain variable region portion of MabCXCL10_G7; however, these modifications were intended for improving the efficiency of cleavage of the protease recognition sequence and for improving the efficiency of hCXCL10 release after cleavage, respectively, and do not contribute to stability of the hCXCLIO N-terminus.

Using a DNA sequence encoding light chain: G7L-LT0 (SEQ ID NO: 57) as a template, a DNA sequence encoding G7L.R38E-LT0 (SEQ ID NO: 65) was produced by a method known to those skilled in the art. The modification introduced into the MabCXCL10_G7 light chain is also a modification intended to improve the efficiency of hCXCLIO release after cleavage of the heavy chain, and does not contribute to the stability of the hCXCLIO N-terminus.

These heavy chain variants were combined with the light chain and IC17HdK-G1T4k.H435R (SEQ ID NO: 62) to express the hCXCL10 fusion-type anti-hCXCL10 antibody: hCXCL10R75A.0659.G4SGGGG.G7HFR0039H.0004.N0222-G1T4h/G7L.R38E-LT0//IC17HdK-G1T4k.H435R/G7L.R38E-LT0 (SEQ ID NO: 63)
by transient expression using Expi 293 (Life Technologies) by a method known to those skilled in the art, and then this was purified by a method known to those skilled in the art using protein A.

(6.2)
Mouse PK studies were performed to evaluate the stability in blood of CXCL10.

Experiments were performed by the method described in 4.1. Blood was collected 5 minutes, 1 hour, 7 hours, 1 day, and 5 days after administration.
As an analyte, the following hCXCL10 fusion-type anti-hCXCL10 antibody prepared in Example 6.1 was used: hCXCL10R75A.0659.G4SGGGG.G7HFR0039H.0004.N0222-G1T4h/G7L.R38E-LT0//IC17HdK-G1T4k.H435R/G7L.R38E-LT0

The results are shown in Fig. 9 and Table 10.
hCXCL10R75A.0659.G4SGGGG.G7HFR0039H.0004.N0222-G1T4h/G7L.R38E-LT0//IC17HdK-G1T4k.H435R/G7L.R38E-LT0 showed the same percentage of HCXCL10 N-terminal retention as hCXCL10R75A.0303.G4SGGGG.G7HFR0039H.12aa0054-G1T4h/G7L-LT0//IC17HdK-G1T4k.H435R/G7L-LT0 at each of the time points of Day 1 and Day 5.

This showed that hCXCLIO N-terminal stabilization by the P2V and/or V7P modifications can be combined with the P18C/A60C modification.

[Table 10]

| Percentage of N-terminal peptide retention | | |
|---|---|---|
| Antibody | Days after administration | N-terminus retention rate |
| hCXCL10R75A. 0659. G4SGGGG. G7HFR0039H. 0004. N0222-GIT4h/G7L. R38E-LT0//IC17HdK-G1T4k. H435R/G7L. R38E-LT0 | 1 | 71.0% |
| | 5 | 31.8% |

Although the foregoing invention has been described in some detail by way of illustration and example for purposes of clarity of understanding, the descriptions and examples should not be construed as limiting the scope of the disclosure. The disclosures of all patent and scientific literature cited herein are expressly incorporated in their entirety by reference.

[Industrial Applicability]

The present disclosure discloses CXCR3 ligands having the activity of inducing migration of cells expressing CXCR3. Such CXCR3 ligands are useful for disease treatment/prevention by causing migration of cells expressing CXCR3.

**Claims**

1.   A CXCR3 ligand, which is obtained by adding at least either or both modifications of:

   (i) introduction of a disulfide bond; and
   (ii) modification of an amino acid in an N-terminal region;
   to the amino acid sequence of a parent CXCR3 ligand,
   wherein the CXCR3 ligand has enhanced activity to induce migration of a cell expressing CXCR3 relative to the parent CXCR3 ligand.

2.   The CXCR3 ligand of claim 1, wherein the parent CXCR3 ligand is a naturally-occurring human CXCL10.

3.   The CXCR3 ligand of claim 2, wherein the disulfide bond is introduced by substituting amino acids corresponding to the amino acid positions of the following (i) and (ii) in the amino acid sequence of the naturally-occurring human CXCL10 with Cys:

   (i) at least one position selected from the group consisting of positions 18, 14, 21, 25, and 41; and
   (ii) at least one position selected from the group consisting of positions 60, 55, 67, 46, and 56.

4.   The CXCR3 ligand of any one of claims 1 to 3, wherein the 18th amino acid from the N-terminus, P, is substituted with C and the 60th amino acid from the N-terminus, A, is substituted with C in the amino acid sequence of the parent CXCR3 ligand.

5.   The CXCR3 ligand of any one of claims 1 to 4, wherein the first amino acid from the N-terminus in the amino acid sequence of the parent CXCR3 ligand is V and the amino acid modification in the N-terminal region comprises the amino acid substitution V1Y.

6.   The CXCR3 ligand of any one of claims 1 to 5, further wherein the second amino acid from the N-terminus in the amino acid sequence of the parent CXCR3 ligand is P, and the amino acid modification in the N-terminal region comprises the amino acid substitution P2V.

7.   The CXCR3 ligand of claim 1 to claim 6, further wherein at least one modification selected from amino acid substitution, deletion, and insertion has additionally been made.

8.   The CXCR3 ligand of any one of claims 1 to 7, wherein the parent CXCR3 ligand is a CXCL10 variant obtained by adding an R75A modification to the amino acid sequence of the naturally-occurring human CXCL10.

9.   A CXCR3 ligand, which is obtained by substituting an amino acid corresponding to amino acid position 7, when taking the N-terminal amino acid in the amino acid sequence of the parent CXCR3 ligand as position 1, with Pro, wherein the CXCR3 ligand has improved stability in blood relative to the parent CXCR3 ligand.

10. A polypeptide, which is obtained by substituting amino acids corresponding to amino acid positions 18 and 60 in the amino acid sequence of naturally-occurring human CXCL10 or a CXCL10 variant, when taking the N-terminal amino acid of the naturally-occurring human CXCL10 as position 1, with Cys.

11. A polypeptide, which is obtained by substituting the N-terminal amino acid (position 1) in the amino acid sequence of naturally-occurring human CXCL10 or a CXCL10 variant with Tyr.

12. A polypeptide comprising the following amino acid sequence:
(X1)(X2)LSRTVRCT CISISNQ(X3)VN PRSLEKLEII PASQFCPRVE IIATMKKKG EKRCLNPESK(X4) IKNLLKAVSK ERSK(X5)SP,

wherein

(i) (X1) is V or Y, (X2) is P or V, (X3) and (X4) are C, and (X5) is R or A (SEQ ID NO: 53); or
(ii) (X1) is Y, (X2) is P or V, (X3) is P or C, (X4) is A or C, and (X5) is R or A (SEQ ID NO: 54).

13. A polypeptide, which is obtained by substituting an amino acid corresponding to amino acid position 7 in the amino acid sequence of naturally-occurring human CXCL10 or a CXCL10 variant, when taking the N-terminal amino acid of the naturally-occurring human CXCL10 as position 1, with Pro.

14. A fusion protein comprising the CXCR3 ligand of any one of claims 1 to 9 or the polypeptide of any one of claims 10 to 13.

15. An isolated nucleic acid encoding the CXCR3 ligand of any one of claims 1 to 9, the polypeptide of any one of claims 10 to 13, or the fusion protein of claim 14.

16. A vector comprising the nucleic acid of claim 15.

17. A host cell comprising the nucleic acid of claim 15 or the vector of claim 16.

18. A method of enhancing the activity of a CXCR3 ligand to induce migration of a cell expressing CXCR3, comprising adding at least either or both modifications of:

(i) introduction of a disulfide bond; and
(ii) modification of an amino acid in an N-terminal region;

to the amino acid sequence of a parent CXCR3 ligand.

19. A method of improving the stability in blood of a CXCR3 ligand, comprising substituting an amino acid corresponding to amino acid position 7, when taking the N-terminal amino acid in the amino acid sequence of the parent CXCR3 ligand as position 1, with Pro.

**A**

COOH

NH2

hCXCL10 P18C/A60C
(model structure based on 1o7y)
Cys residues are shown in stick

Conventional S-S bond,
Cys9-Cys36, and Cys11-Cys53

Introduced S-S bond by
P18C/A60C substitution

**B**

hCXCL10R75A

IgG1 Fc

FIG. 1

FIG. 2

FIG. 3

FIG. 4

FIG. 5

FIG. 6

FIG. 7

FIG. 8

FIG. 9

## INTERNATIONAL SEARCH REPORT

| International application No. |
| --- |
| **PCT/JP2022/047366** |

**A. CLASSIFICATION OF SUBJECT MATTER**

*C07K 14/52*(2006.01)i; *C07K 19/00*(2006.01)i; *C12N 1/15*(2006.01)i; *C12N 1/19*(2006.01)i; *C12N 1/21*(2006.01)i; *C12N 5/10*(2006.01)i; *C12N 15/19*(2006.01)i; *C12N 15/62*(2006.01)i; *C12N 15/63*(2006.01)i
FI:   C07K14/52 ZNA; C12N15/19; C12N15/62 Z; C07K19/00; C12N15/63 Z; C12N1/15; C12N1/19; C12N1/21; C12N5/10

According to International Patent Classification (IPC) or to both national classification and IPC

**B. FIELDS SEARCHED**

Minimum documentation searched (classification system followed by classification symbols)

C07K14/52; C07K19/00; C12N1/15; C12N1/19; C12N1/21; C12N5/10; C12N15/19; C12N15/62; C12N15/63

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII); CAplus/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
| --- | --- | --- |
| X | WO 2003/082335 A1 (SUMITOMO PHARMACEUTICALS CO., LTD.) 09 October 2003 (2003-10-09) claims 1-33, p. 7, line 18 to p. 9, line 20 | 1-2, 15-17 |
| Y | | 1-19 |
| X | WO 2020/116498 A1 (CHUGAI PHARMACEUTICAL CO LTD) 11 June 2020 (2020-06-11) claims 1-19, paragraphs [0005]-[0009], fig. 1 | 1-2, 5-9, 13-19 |
| Y | | 1-19 |
| X | GERLZA, T. et al. Designing an improved T-cell mobilising CXCL10 mutant through enhanced GAG binding affinity. PROTEIN ENGINEERING, DESIGN AND SELECTION. 23 January 2020, vol. 32, no. 8, pp. 367-373, DOI: 10.1093/protein/gzz043 abstract, table 1, fig. 4-5 | 1-2, 15-18 |
| Y | | 1-19 |

☑ Further documents are listed in the continuation of Box C.      ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **01 March 2023** | **14 March 2023** |

| Name and mailing address of the ISA/JP | Authorized officer |
| --- | --- |
| **Japan Patent Office (ISA/JP)** 3-4-3 Kasumigaseki, Chiyoda-ku, Tokyo 100-8915 Japan | |
| | Telephone No. |

Form PCT/ISA/210 (second sheet) (January 2015)

## INTERNATIONAL SEARCH REPORT

| | International application No. |
|---|---|
| | **PCT/JP2022/047366** |

**C.    DOCUMENTS CONSIDERED TO BE RELEVANT**

| Category* | Citation of document, with indication, where appropriate, of the relevant passages | Relevant to claim No. |
|---|---|---|
| X | WO 2021/038033 A1 (ANTAGONIS BIOTHERAPEUTICS GMBH) 04 March 2021 (2021-03-04)<br>     claims 1-18 | 1-2, 7, 15-18 |
| Y | | 1-19 |
| X | JP 2008-545396 A (INTERMUNE INC) 18 December 2008 (2008-12-18)<br>     claims 1-35, paragraph [0066], fig. 3, SEQ ID NO: 10, 6 | 1-2, 7, 14-17 |
| Y | | 1-19 |
| A | CAMPANELLA, G.S.V. et al. CXCR3 and heparin binding sites of the chemokine IP-10 (CXCL10). THE JOURNAL OF BIOLOGICAL CHEMISTRY. 09 May 2003, vol. 278, no. 19, pp. 17066-17074, DOI: 10.1074/jbc.M212077200<br>     fig. 5 | 1-19 |

Form PCT/ISA/210 (second sheet) (January 2015)

## INTERNATIONAL SEARCH REPORT

International application No.

**PCT/JP2022/047366**

| Box No. I | Nucleotide and/or amino acid sequence(s) (Continuation of item 1.c of the first sheet) |
|---|---|

1. With regard to any nucleotide and/or amino acid sequence disclosed in the international application, the international search was carried out on the basis of a sequence listing:

    a. ☑ forming part of the international application as filed:

        ☑ in the form of an Annex C/ST.25 text file.

        ☐ on paper or in the form of an image file.

    b. ☐ furnished together with the international application under PCT Rule 13*ter*.1(a) for the purposes of international search only in the form of an Annex C/ST.25 text file.

    c. ☐ furnished subsequent to the international filing date for the purposes of international search only:

        ☐ in the form of an Annex C/ST.25 text file (Rule 13*ter*.1(a)).

        ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

    "The form of Annex C/ST.25 text file" above shall read as "the form of ST.26.".

Form PCT/ISA/210 (continuation of first sheet) (January 2015)

**INTERNATIONAL SEARCH REPORT**
Information on patent family members

International application No.

**PCT/JP2022/047366**

| Patent document cited in search report | | | Publication date (day/month/year) | Patent family member(s) | | | Publication date (day/month/year) |
|---|---|---|---|---|---|---|---|
| WO | 2003/082335 | A1 | 09 October 2003 | US 2005/0119174 A1 claims 1-33, paragraphs [0026]-[0033] | | | |
| WO | 2020/116498 | A1 | 11 June 2020 | US 2022/0017585 A1 claims 1-19, paragraphs [0002]-[0007], fig. 1 EP 3892330 A1 | | | |
| WO | 2021/038033 | A1 | 04 March 2021 | US 2022/0281934 A1 claims 1-18 EP 4021927 A1 | | | |
| JP | 2008-545396 | A | 18 December 2008 | US 2009/0131312 A1 claims 1-35, paragraph [0071], fig. 3, SEQ ID NOs 6, 10 WO 2006/125077 A2 EP 1885386 A2 | | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2021038033 A
- WO 2018097308 A
- WO 2019107384 A
- WO 2020116498 A1
- WO 2019107380 A

**Non-patent literature cited in the description**

- **TOKUNAGA, R et al.** *Cancer Treat Rev,* 2017, vol. 63, 40-47
- **RAINCZUK, A. et al.** *Int J Cancer,* 2014, vol. 134 (3), 530-541
- **AGUILERA-DURAN, G et al.** *Molecules,* 2020, vol. 25 (19
- **GRAHAM, G. J. et al.** *Trends Immunol,* 2019, vol. 40 (6), 472-481
- **GERLZA, T.** *Protein Eng Des Sel.,* 2019, vol. 31;32 (8), 367-373
- **KUNKEL et al.** *Proc. Natl. Acad. Sci. USA,* 1985, vol. 82, 488-492
- *Annu. Rev. Biophys. Biomol. Struct.,* 2006, vol. 35, 225-249
- *Proc. Natl. Acad. Sci. U.S.A.,* 2003, vol. 100 (11), 6353-6357
- **CLARK-LEWIS et al.** *J. Biol. Chem.,* 1994, vol. 269, 16075-16081
- **BELPERIO et al.** *J. Leukoc. Biol.,* 2000, vol. 68 (1-8
- **DUFOUR et al.** *J Immunol.,* 2002, vol. 168, 3195-204
- **KIESEIER et al.** *Brain,* 2002, vol. 125, 823-34
- **NAGASAWA, T.** *Int. J. Hematol.,* 2000, vol. 72, 408-11
- **GAZITT, Y.** *J. Hematother Stem Cell Res,* 2001, vol. 10, 229-36
- **HATTORI et al.** *Blood,* 2001, vol. 97, 3354-59
- **NOMURA et al.** *Int. J. Cancer,* 2001, vol. 91, 597-606
- **MACH ; DRANOFF.** *Curr. Opin. Immunol.,* 2000, vol. 12, 571-75
- **BRUCE, L. et al.** *Methods in Molecular Biology,* 2000, vol. 138, 129-134
- **RAPHAELE, B. et al.** *Methods in Molecular Biology,* 2000, vol. 138, 143-148
- **PAUL D. PONATH et al.** *Methods in Molecular Biology,* 2000, vol. 138, 113-120
- **BOOTH V. et al.** *Biochemistry,* vol. 41 (33), 10418-25
- **RANI MR.** *The Journal of Biological Chemistry,* vol. 271 (37), 22878-84
- **COLE KE.** *The Journal of Experimental Medicine,* vol. 187 (12), 2009-21
- **TENSEN CP.** *The Journal of Investigative Dermatology.,* vol. 112 (5), 716-22
- **ALLEN, S. J. ; S. E. CROWN ; T. M. HANDEL.** Chemokine: receptor structure, interactions, and antagonism. *Annu Rev Immunol,* 2007, vol. 25, 787-820
- **BOEHR, D. D. ; R. NUSSINOV ; P. E. WRIGHT.** The role of dynamic conformational ensembles in biomolecular recognition. *Nat Chem Biol,* 2009, vol. 5 (11), 789-796
- **BOOTH, V. ; I. CLARK-LEWIS ; B. D. SYKES.** NMR structure of CXCR3 binding chemokine CXCL11 (ITAC). *Protein Sci,* 2004, vol. 13 (8), 2022-2028
- **BOOTH, V. ; D. W. KEIZER ; M. B. KAMPHUIS ; I. CLARK-LEWIS ; B. D. SYKES.** The CXCR3 Binding Chemokine IP-10/CXCL10: Structure and Receptor Interactions. *Biochemistry,* 2002, vol. 41 (33), 10418-10425
- **CLARK-LEWIS, I. ; I. MATTIOLI ; J. H. GONG ; P. LOETSCHER.** Structure-function relationship between the human chemokine receptor CXCR3 and its ligands. *J Biol Chem,* 2003, vol. 278 (1), 289-295
- **DISHMAN, A. F. ; R. C. TYLER ; J. C. FOX ; A. B. KLEIST ; K. E. PREHODA ; M. M. BABU ; F. C. PETERSON ; B. F. VOLKMAN.** Evolution of fold switching in a metamorphic protein. *Science,* 2021, vol. 371 (6524), 86-90
- **MATSUO, K. ; K. KITAHATA ; F. KAWABATA ; M. KAMEI ; Y. HARA ; S. TAKAMURA ; N. OISO ; A. KAWADA ; O. YOSHIE ; T. NAKAYAMA.** A Highly Active Form of XCL1/Lymphotactin Functions as an Effective Adjuvant to Recruit Cross-Presenting Dendritic Cells for Induction of Effector and Memory CD8(+) T Cells. *Front Immunol,* 2018, vol. 9, 2775

- **MORTIER, A. ; J. VAN DAMME ; P. PROOST.** Regulation of chemokine activity by posttranslational modification. *Pharmacol Ther,* 2008, vol. 120 (2), 197-217
- **PROOST, P. ; E. SCHUTYSER ; P. MENTEN ; S. STRUYF ; A. WUYTS ; G. OPDENAKKER ; M. DETHEUX ; M. PARMENTIER ; C. DURINX ; A.-M. LAMBEIR.** Amino-terminal truncation of CXCR3 agonists impairs receptor signaling and lymphocyte chemotaxis, while preserving antiangiogenic properties. *Blood, The Journal of the American Society of Hematology,* 2001, vol. 98 (13), 3554-3561
- **REPNIK, U. ; A. E. STARR ; C. M. OVERALL ; B. TURK.** Cysteine Cathepsins Activate ELR Chemokines and Inactivate Non-ELR Chemokines. *J Biol Chem,* 2015, vol. 290 (22), 13800-13811
- **TUINSTRA, R. L. ; F. C. PETERSON ; E. S. ELGIN ; A. J. PELZEK ; B. F. VOLKMAN.** An Engineered Second Disulfide Bond Restricts Lymphotactin/XCL1 to a Chemokine-like Conformation with XCR1 Agonist Activity. *Biochemistry,* 2007, vol. 46 (10), 2564-2573
- *Proc. Natl. Acad. Sci. USA,* 2006, vol. 103 (11), 4005-4010
- *Biochemistry,* 2002, vol. 41, 10418-10425
- *MOLECULAR AND CELLULAR BIOLOGY,* August 2006, 5838-5849
- *Methods Enzymol.,* 2000, vol. 323, 325-40
- *Molecular and Cellular Biology,* August 2006, vol. 26 (15), 5838-5849
- *The Journal of Biological Chemistry,* 09 May 2003, vol. 278 (19), 17066-17074
- *Protein Engineering,* 1996, vol. 9 (3), 299-305
- **KINDT et al.** Kuby Immunology. W.H. Freeman and Co, 2007, 91
- **KABAT et al.** Sequences of Proteins of Immunological Interest. Public Health Service, National Institutes of Health, 1991
- *Mol Cell Biol.,* 1988, vol. 8, 466-472
- Current protocols in Molecular Biology. John Wiley & Sons, 1987
- **BRONGER, H. ; MAGDOLEN, V. ; GOETTIG, P. et al.** Proteolytic chemokine cleavage as a regulator of lymphocytic infiltration in solid tumors. *Cancer Metastasis Rev,* 2019, vol. 38, 417-430